# EUROPEAN PATENT APPLICATION

(11) **EP 3 453 706 A1**
(43) Date of publication of application: **13.03.2019**
(21) Application number: 17190145.7
(22) Date of filing: 08.09.2017
(51) Int. Cl.: C07D 401/04, A01N 43/50

(54) **PESTICIDAL IMIDAZOLE COMPOUNDS**

(71) Applicant: BASF SE, 67056 Ludwigshafen am Rhein (DE)
(72) Inventor: McLaughlin, Martin John, 67056 Ludwigshafen (DE); Narine, Arun, 67056 Ludwigshafen (DE); von Deyn, Wolfgang, 67056 Ludwigshafen (DE); Gockel, Birgit, 67056 Ludwigshafen (DE)
(74) Representative: BASF IP Association

(57) **Abstract**

The present invention relates to compounds of formula I, 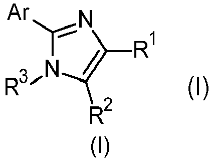 wherein the variables are defined as given in the description and claims. The invention further relates to uses, processes and composition for compounds I.

## Description

The present invention relates to substituted imidazolyl compounds of formula I as agrochemical pesticides. Furthermore, the present invention relates to processes and intermediates for preparing compounds of formula I and to active compound combinations comprising them. Moreover, the present invention relates to agricultural or veterinary compositions comprising the compounds of formula I, and to the use of the compounds of formula I or compositions comprising them for combating or controlling invertebrate pests and/or for protecting crops, plants, plant propagation material and/or growing plants from attack and/or infestation by invertebrate pests. The present invention also relates to methods of applying the compounds of formula I. The present invention also relates to composition comprising seed or plant propagation material and compounds according to the invention. Furthermore, the present invention relates to seed comprising compounds according to the invention.

Invertebrate pests and in particular insects, arachnids and nematodes destroy growing and harvested crops and attack wooden dwelling and commercial structures, thereby causing large economic loss to the food supply and to property. Accordingly, there is an ongoing need for new agents for combating invertebrate pests.

5,6-fused heterocycles are known for pesticidal use, for example, in patent publications WO 2012/086848, WO 2013/180193 and WO 2014/119670 and represent an important class of insecticide. Further, reference is made to publications WO 2017/043386, WO 2017/043386, WO 2017/025419, WO 2017/001314, WO 2016/023954 and WO2017/094790.

Due to the ability of target pests to develop resistance to pesticidally-active agents, there is an ongoing need to identify further compounds, which are suitable for combating invertebrate pests such as insects, arachnids and nematodes. Furthermore, there is a need for new compounds having a high pesticidal activity and showing a broad activity spectrum against a large number of different invertebrate pests, especially against difficult to control insects, arachnids and nematodes.

It is therefore an object of the present invention to identify and provide compounds, which exhibit a high pesticidal activity and have a broad activity spectrum against invertebrate pests.

It has been found that these objects can be achieved by substituted bicyclic compounds of formula I, as depicted and defined below, including their stereoisomers, their salts, in particular their agriculturally or veterinarily acceptable salts, their tautomers and their N-oxides.

In a first aspect, the present invention relates to the compound of formula I, wherein 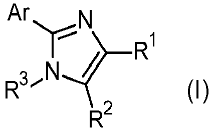
- R¹: is CN, NO₂, C(O)-OR^{b}, C(O)NR^{b}R^{c}, C₁-C₆-alkyl, C₂-C₆-alkenyl-C₁-C₆-alkyl, C₂-C₆-alkenyl-NR^{b}R^{c}, C₂-C₆-alkenyl-CN, C₂-C₆-alkenyl-C(O)-OR^{b}, C₂-C₆-alkenyl-C(O)-R^{b}, C₂-C₆-alkenyl-S(=_{O})ₘR^{e}, C(O)-R^{d}, SO₂NR^{b}R^{c}, or S(=O)ₘR^{e}, which are unsubstituted or substituted with R^{a};
- R²: H, halogen, N₃, CN, NO₂, -SCN, -SF₅, C₁-C₆-alkyl, C₁-C₆-alkoxy, C₂-C₆-alkenyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b}, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloal-kyl, C₃-C₆-cycloalkoxy, C₃-C₆-cycloalkyl-C₁-C₄-alkyl, C₃-C₆-cycloalkoxyx-C₁-C₄-alkyl, C(O)-OR^{b}, NR^{b}R^{c}, C(O)-NR^{b}R^{c}, C(O)-R^{d}, SO₂NR^{b}R^{c}, or S(=O)ₘR^{e}, which are unsubstituted or substituted with R^{a};
- R³: H, C₁-C₆-alkyl, C₁-C₆-alkoxy, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, which are unsubstituted or substituted with R^{a}, C(O)-OR^{a}, NR^{b}R^{c}, C(O)-NR^{b}R^{c}, C(O)-R^{d}, SO₂NR^{b}R^{c}, or S(=O)mR^{e};
- Ar: is phenyl or 5- or 6-membered heteroaryl, which is unsubstituted or substituted with R^{Ar};
R^{Ar} independently of each other, are selected from the group consisting of halogen, N₃, OH, CN, NO₂, -SCN, -SF₅, C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, which are unsubstituted or substituted with halogen,
C(O)-OR^{a}, NR^{b}R^{c}, C₂-C₆-alkenyl-NR^{b}R^{c}, O-C₂-C₆-alkenyl-NR^{b}R^{c}, C₂-C₆-alkenyl-CN, NH-C₂-C₆-alkenyl-NR^{b}R^{c}, C(O)-NR^{b}R^{c}, C(O)-R^{d}, SO₂NR^{b}R^{c}, and S(=O)ₘR^{e}, one radical may also be phenyl, phenoxy, phenylcarbonyl, phenylthio or benzyl, wherein the phenyl ring is unsubstituted or substituted with radicals R^{f};
- each R^{a}: is selected from halogen, OH, CN, NO₂, NR^{b}R^{c}, C₁-C₆-alkyl, C₁-C₆-alkoxy, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, C(O)-OR^{b}, and S(=O)ₘR^{e}, which are unsubstituted or substituted with halogen, phenyl and benzyl, wherein the phenyl ring is unsubstituted or substituted with radicals R^{f};
- each R^{b}: is selected from H, C₁-C₆-alkyl, C₂-C₆-alkenyl, C₂-C₆-alkynyl, C₁-C₆-alkoxy-C₁-C₄-alkyl, C₁-C₆-alkylthio-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which are unsubstituted or substituted with halogen, -(C=O)R^{d},-C(=O)OR^{d}, and -C(=O)NR^{d}, phenyl, benzyl or 5- to 6-membered heterocycle wherein the cyclic groups are unsubstituted or substituted with radicals R^{f};
- each R^{c}: is selected from H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, and C₃-C₆-cycloalkoxy-C₁-C₄-alkyl, which are unsubstituted or substituted with halogen;
- each moiety NR^{b}R^{c}: may also form an N-bound, saturated 3- to 8-membered heterocycle, which in addition to the nitrogen atom may have 1 or 2 further heteroatoms or heteroatom moieties selected from O, S(=O)ₘ and N-R', wherein R' is H, -C(=O)OR, or C₁-C₆-alkyl and wherein the N-bound heterocycle is unsubstituted or substituted with radicals selected from halogen, C₁-C₄-alkyl, C₁-C₄-haloalkyl, C₁-C₄-alkoxy, and C₁-C₄-haloalkoxy;
- each R^{d}: is selected from H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, which are unsubstituted or substituted with halogen,
phenyl and benzyl, wherein the phenyl ring is unsubstituted or substituted with radicals R^{f};
- each R^{e}: is selected from C₁-C₆-alkyl, C₃-C₆-cycloalkyl, C₃-C₆-cycloalkyl-C₁-C₄-alkyl, which are unsubstituted or substituted with halogen,
phenyl, and benzyl, wherein the phenyl ring is unsubstituted or substituted with R^{f};
- each R^{f}: is selected from halogen, N₃, OH, CN, NO₂, SCN, SF₅, C₁-C₆-alkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, and C₃-C₆-cycloalkoxy-C₁-C₄-alkyl, which are unsubstituted or substituted with halogen;
- m: is 0, 1 or 2;
including the N-oxides, stereoisomers, tautomers and agriculturally or veterinarily acceptable salts thereof.

### General Procedure:

With due modification of the starting compounds, the compounds of formula I can principally be prepared by procedures as given in below schemes.

Imidazoles 3 can be prepared by procedures known by someone skilled in the art. Reaction of 1 with organometallic reagent 2 lead then to desired products 3. (Scheme 1). Conditions for such a reaction can be found in WO 2009/35162

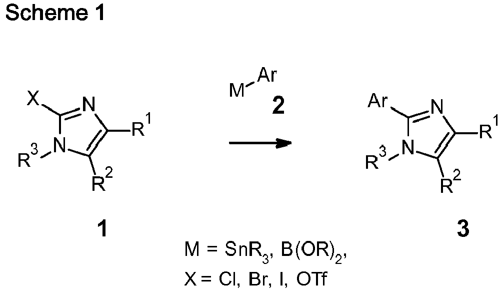

Alternatively, it may be necessary to reverse the coupling partners in such a reaction. (Scheme 2). A description for such a reaction can be found in WO 2012/136776.

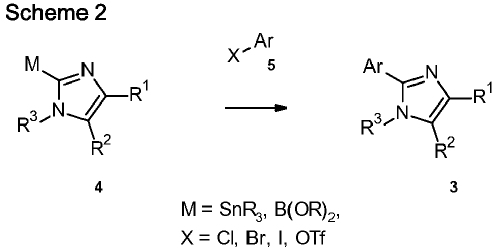

Alternatively compounds 3 can be prepared by acylation of the phosphorous ylid 7 with carboxylic acid, followed by hydrolysis of the ylid, and condensation 9 with aldehyde 10 to give imidazole 11. Alkylation of 11 then affords the desired compound 3. Conditions for this reaction sequence are described by Buck et al. J. Med. Chem. 2005, 48, 6803-6812. (Scheme 3).

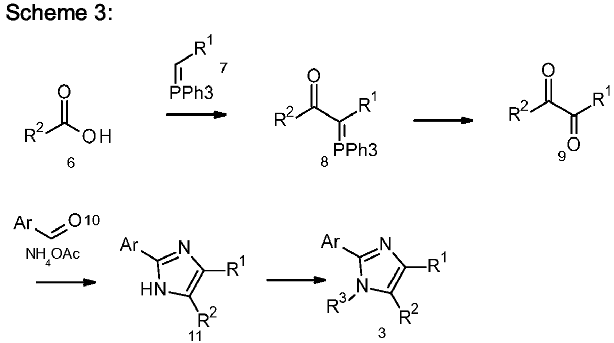

Alternatively compounds 3 can be prepared by condensation of amidine 12 with ketone 13 to give imidazole 11, whose alkylation under standard conditions leads affords compound 3. Conditions for this reaction sequence are described in WO 2009/139076 (Scheme 4).

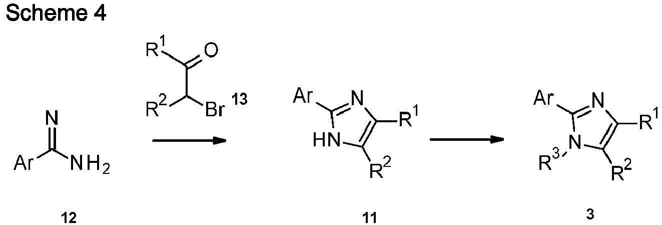

The procedures described above can be used individually or in combination of one another to obtain compounds of formula I.

The starting materials required for preparing the compounds of formula I are commercially available or can be prepared in accordance with the procedures known in literature.

The reaction mixtures are worked up in a customary manner, for example by mixing with water, separating the phases and, if appropriate, chromatographic purification of the crude products.
Some of the intermediates and end products are obtained in the form of colorless or slightly brownish viscous oils which are purified or freed from volatile components under reduced pressure and at moderately elevated temperature. If the intermediates and end products are obtained as solids, purification can also be carried out by recrystallization or digestion.

If individual compounds of formula I cannot be obtained by the routes described above, they can be prepared by derivatization of other compounds of formula I or intermediates thereof.

If the synthesis yields mixtures of isomers, a separation is generally not necessarily required since in some cases the individual isomers can be interconverted during work-up for use or during application (for example under the action of light, acids or bases). Such conversions may also take place after use, for example in the treatment of plants in the treated plant, or in the harmful fungus to be controlled.

A skilled person will readily understand that the preferences for the substituents, also in particular the ones given in the tables below for the respective substituents, given herein in connection with compounds I apply for the intermediates accordingly. Thereby, the substituents in each case have independently of each other or more preferably in combination the meanings as defined herein. The procedures described above can be used individually or in combination of one another to obtain compounds of formula I.

The N-oxides may be prepared from the inventive compounds according to conventional oxidation methods, e. g. by treating compounds I with an organic peracid such as metachloroperbenzoic acid (cf. WO 03/64572 or J. Med. Chem. 38(11), 1892-903, 1995); or with inorganic oxidizing agents such as hydrogen peroxide (cf. J. Heterocyc. Chem. 18(7), 1305-8, 1981) or oxone (cf. J. Am. Chem. Soc. 123(25), 5962-5973, 2001). The oxidation may lead to pure mono-N-oxides or to a mixture of different N-oxides, which can be separated by conventional methods such as chromatography.

Unless otherwise indicated, the term "compound(s) according to the invention" or "compound(s) of the invention" or "compound(s) of formula (I)", refers to the compounds of formula I.

The term "compound(s) according to the invention", or "compounds of formula I" comprises the compound(s) as defined herein as well as a stereoisomer, salt, tautomer or N-oxide thereof. The term "compound(s) of the present invention" is to be understood as equivalent to the term "compound(s) according to the invention", therefore also comprising a stereoisomer, salt, tautomer or N-oxide thereof.

The term "composition(s) according to the invention" or "composition(s) of the present invention" encompasses composition(s) comprising at least one compound of formula I according to the invention as defined above. The compositions of the invention are preferably agricultural or veterinary compositions.

Depending on the substitution pattern, the compounds according to the invention may have one or more centers of chirality, in which case they are present as mixtures of enantiomers or diastereomers. The invention provides both the single pure enantiomers or pure diastereomers of the compounds according to the invention, and their mixtures and the use according to the invention of the pure enantiomers or pure diastereomers of the compounds according to the invention or their mixtures. Suitable compounds according to the invention also include all possible geometrical stereoisomers (cis/trans isomers) and mixtures thereof. Cis/trans isomers may be present with respect to an alkene, carbon-nitrogen double-bond or amide group. The term "stereoisomer(s)" encompasses both optical isomers, such as enantiomers or diastereomers, the latter existing due to more than one center of chirality in the molecule, as well as geometrical isomers (cis/trans isomers). The present invention relates to every possible stereoisomer of the compounds of formula I, i.e. to single enantiomers or diastereomers, as well as to mixtures thereof.

The compounds according to the invention may be amorphous or may exist in one or more different crystalline states (polymorphs) which may have different macroscopic properties such as stability or show different biological properties such as activities. The present invention relates to amorphous and crystalline compounds according to the invention, mixtures of different crystalline states of the respective compounds according to the invention, as well as amorphous or crystalline salts thereof.

Salts of the compounds according to the invention are preferably agriculturally and/or veterinary acceptable salts, preferably agriculturally acceptable salts. They can be formed in a customary manner, e.g. by reacting the compound with an acid of the anion in question if the compounds according to the invention have a basic functionality or by reacting acidic compounds according to the invention with a suitable base.

Veterinary and/or agriculturally useful salts of the compounds according to the invention encompass especially the acid addition salts of those acids whose cations and anions, respectively, have no adverse effect on the pesticidal action of the compounds according to the invention.

Suitable cations are in particular the ions of the alkali metals, preferably Li, Na and K, of the alkaline earth metals, preferably Ca, Mg and Ba, and of the transition metals, preferably Mn, Cu, Zn and Fe, and also ammonium (NH₄⁺) and substituted ammonium in which one to four of the H atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethyl-ammonium, bis(2-hydroxyethyl)ammonium, benzyltrimethylammonium and benzyltriethylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting compounds according to the invention with an acid of the corresponding anion, preferably of hydrochloric acid, hydrobromic acid, sulfuric acid, phosphoric acid or nitric acid.

The term "N-oxide" includes any compound of the present invention which has at least one tertiary nitrogen atom that is oxidized to an N-oxide moiety.

The organic moieties mentioned in the above definitions of the variables are - like the term halogen - collective terms for individual listings of the individual group members. The prefix Cₙ-Cₘ indicates in each case the possible number of carbon atoms in the group.

The term "halogen" denotes in each case F, Br, Cl or I, in particular F, Br or Cl.

The term "alkyl" as used herein and in the alkyl moieties of alkylamino, alkylcarbonyl, alkylthio, alkylsulfinyl, alkylsulfonyl and alkoxyalkyl denotes in each case a straight-chain or branched alkyl group having usually from 1 to 10 carbon atoms, frequently from 1 to 6 carbon atoms, preferably 1 to 4 carbon atoms, more preferably from 1 to 3 carbon atoms. Examples of an alkyl group are CH₃, C₂H₅, n-propyl, iso-propyl, n-butyl, 2-butyl, iso-butyl, tert-butyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl.

The term "haloalkyl" as used herein and in the haloalkyl moieties of haloalkylcarbonyl, haloalkoxycarbonyl, haloalkylthio, haloalkylsulfonyl, haloalkylsulfinyl, haloalkoxy and haloalkoxyalkyl, denotes in each case a straight-chain or branched alkyl group having usually from 1 to 6 carbon atoms, preferably from 1 to 4 carbon atoms, wherein the H atoms of this group are partially or fully replaced with halogen atoms. Preferred haloalkyl moieties are selected from C₁-C₄-haloalkyl, more preferably from C₁-C₃-haloalkyl or C₁-C₂-haloalkyl, in particular from C₁-C₂-fluoroalkyl such as fluoromethyl, difluoromethyl, trifluoromethyl, 1-fluoroethyl, 2-fluoroethyl, 2,2-difluoroethyl, 2,2,2-trifluoroethyl, pentafluoroethyl, and the like.

The term "alkoxy" as used herein denotes in each case a straight-chain or branched alkyl group which is bonded via an oxygen atom and has usually from 1 to 6 carbon atoms, preferably 1 to 4 carbon atoms. Examples of an alkoxy group are methoxy, ethoxy, n-propoxy, iso-propoxy, n-butyloxy, 2-butyloxy, iso-butyloxy, tert.-butyloxy, and the like.

The term "alkoxyalkyl" as used herein refers to alkyl usually comprising 1 to 4, preferably 1 to 2 carbon atoms, wherein 1 carbon atom carries an alkoxy radical usually comprising 1 to 4, preferably 1 or 2 carbon atoms as defined above. Examples are CH₂OCH₃, CH₂-OC₂H₅, 2-(methoxy)ethyl, and 2-(ethoxy)ethyl.

The term "haloalkoxy" as used herein denotes in each case a straight-chain or branched alkoxy group having from 1 to 6 carbon atoms, preferably 1 to 4 carbon atoms, wherein the H atoms of this group are partially or fully replaced with halogen atoms, in particular fluorine atoms. Preferred haloalkoxy moieties include C₁-C₄-haloalkoxy, in particular C₁-C₂-fluoroalkoxy, such as fluoromethoxy, difluoromethoxy, trifluoromethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoro-ethoxy, 2,2-dichloro-2-fluorethoxy, 2,2,2-trichloroethoxy, pentafluoroethoxy and the like.

The term "alkylthio "(alkylsulfanyl: alkyl-S-)" as used herein refers to a straight-chain or branched saturated alkyl group having 1 to 6 carbon atoms, preferably 1 to 4 carbon atoms (= C₁-C₄-alkylthio), more preferably 1 to 3 carbon atoms, which is attached via a sulfur atom. Examples include methylthio, ethylthio, propylthio, isopropylthio, and n-butylthio.

The term "haloalkylthio" as used herein refers to an alkylthio group as mentioned above wherein the H atoms are partially or fully substituted by fluorine, chlorine, bromine and/or iodine. Examples include chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio and pentafluoroethylthio and the like.

The term "alkylsulfinyl" (alkylsulfoxyl: C₁-C₆-alkyl-S(=O)-), as used herein refers to a straight-chain or branched saturated alkyl group (as mentioned above) having 1 to 6 carbon atoms, preferably 1 to 4 carbon atoms (= C₁-C₄-alkylsulfinyl), more preferably 1 to 3 carbon atoms bonded through the sulfur atom of the sulfinyl group at any position in the alkyl group.

The term "alkylsulfonyl" (alkyl-S(=O)₂-) as used herein refers to a straight-chain or branched saturated alkyl group having 1 to 6 carbon atoms, preferably 1 to 4 carbon atoms (= C₁-C₄-alkylsulfonyl), preferably 1 to 3 carbon atoms, which is bonded via the sulfur atom of the sulfonyl group at any position in the alkyl group.

The term "alkoxycarbonyl" refers to an alkylcarbonyl group as defined above, which is bonded via an oxygen atom to the remainder of the molecule.

The term "alkenyl" as used herein denotes in each case a singly unsaturated hydrocarbon radical having usually 2 to 6, preferably 2 to 4 carbon atoms, wherein the double bond may be present in any position, e.g. vinyl, allyl (2-propen-1-yl), 1-propen-1-yl, 2-propen-2-yl, methallyl (2-methylprop-2-en-1-yl), 2-buten-1-yl, 3-buten-1-yl, 2-penten-1-yl, 3-penten-1-yl, 4-penten-1-yl, 1-methylbut-2-en-1-yl, 2-ethylprop-2-en-1-yl and the like.

The term "haloalkenyl" as used herein refers to an alkenyl group as defined above, wherein the H atoms are partially or fully replaced with halogen atoms.

The term "alkynyl" as used herein denotes in each case a singly unsaturated hydrocarbon radical having usually 2 to 6, preferably 2 to 4 carbon atoms, wherein the triple bond may be present in any position, e.g. ethynyl, propargyl (2-propyn-1-yl), 1-propyn-1-yl, 1-methylprop-2-yn-1-yl), 2-butyn-1-yl, 3-butyn-1-yl, 1-pentyn-1-yl, 3-pentyn-1-yl, 4-pentyn-1-yl, 1-methylbut-2-yn-1-yl, 1-ethylprop-2-yn-1-yl and the like.

The term "haloalkynyl" as used herein refers to an alkynyl group as defined above, wherein the H atoms are partially or fully replaced with halogen atoms.

The term "cycloalkyl" as used herein and in the cycloalkyl moieties of cycloalkoxy and cycloalkylthio denotes in each case a monocyclic cycloaliphatic radical having usually from 3 to 8 or from 3 to 6 carbon atoms, such as cyclopropyl, cyclobutyl, cyclopentyl, cyclohexyl, cycloheptyl, cyclooctyl, cyclononyl and cyclodecyl or cyclopropyl, cyclobutyl, cyclopentyl and cyclohexyl.

The term "halocycloalkyl" as used herein and in the halocycloalkyl moieties of halocycloalkoxy and halocycloalkylthio denotes in each case a monocyclic cycloaliphatic radical having usually from 3 to 8 C atoms or 3 to 6 C atoms, wherein at least one, e.g. 1, 2, 3, 4 or 5 of the H atoms, are replaced by halogen, in particular by fluorine or chlorine. Examples are 1- and 2- fluorocyclopropyl, 1,2-, 2,2- and 2,3-difluorocyclopropyl, 1,2,2-trifluorocyclopropyl, 2,2,3,3-tetrafluorocyclpropyl, 1- and 2-chlorocyclopropyl, 1,2-, 2,2- and 2,3-dichlorocyclopropyl, 1,2,2-trichlorocyclopropyl, 2,2,3,3-tetrachlorocyclpropyl, 1-,2- and 3-fluorocyclopentyl, 1,2-, 2,2-, 2,3-, 3,3-, 3,4-, 2,5-difluorocyclopentyl, 1-,2- and 3-chlorocyclopentyl, 1,2-, 2,2-, 2,3-, 3,3-, 3,4-, 2,5-dichlorocyclopentyl and the like.

The term "cycloalkenyl" as used herein and in the cycloalkenyl moieties of cycloalkenyloxy and cycloalkenylthio denotes in each case a monocyclic singly unsaturated non-aromatic radical having usually from 3 to 8, e.g. 3 or 4 or from 5 to 10 carbon atoms, preferably from 3- to 8 carbon atoms. Exemplary cycloalkenyl groups include cyclopropenyl, cycloheptenyl or cyclooctenyl.

The term "substituted" if not specified otherwise refers to substituted by 1, 2 or maximum possible number of substituents. If substituents as defined in compounds of formula I are more than one then they are independently from each other are same or different if not mentioned otherwise.

The term "carbocycle" or "carbocyclyl" includes, unless otherwise indicated, in general a 3- to 12-membered, preferably a 3- to 8-membered or a 5- to 8-membered, more preferably a 5- or 6-membered mono-cyclic, non-aromatic ring comprising 3 to 12, preferably 3 to 8 or 5 to 8, more preferably 5 or 6 carbon atoms. Preferably, the term "carbocycle" covers cycloalkyl and cycloalkenyl groups as defined above, for example cyclopropane, cyclobutane, cyclopentane and cyclohexane rings.

The term "heterocycle" or "heterocyclyl" includes, unless otherwise indicated, in general 3- to 10-membered, preferably 3- to 8-membered or 5- to 8-membered, more preferably 5- or 6-membered, in particular 6-membered monocyclic heterocyclic non-aromatic radicals. The heterocyclic non-aromatic radicals usually comprise 1, 2, 3, 4 or 5, preferably 1, 2 or 3 heteroatoms selected from N, O and S as ring members, where S-atoms as ring members may be present as S, SO or SO₂. If not mentioned contrary, the N and S atoms of the heterocycle can be oxidized. Examples of 5- or 6-membered heterocyclic radicals comprise saturated or unsaturated, non-aromatic heterocyclic rings, such as oxiranyl, oxetanyl, thietanyl, thietanyl-S-oxid (S-oxothietanyl), thietanyl-S-dioxid (S-dioxothiethanyl), pyrrolidinyl, pyrrolinyl, pyrazolinyl, tetrahydrofuranyl, dihydrofuranyl, 1,3-dioxolanyl, thiolanyl, S-oxothiolanyl, S-dioxothiolanyl, dihydrothienyl, S-oxodihydrothienyl, S-dioxodihydrothienyl, oxazolidinyl, oxazolinyl, thiazolinyl, oxathiolanyl, piperidinyl, piperazinyl, pyranyl, dihydropyranyl, tetrahydropyranyl, 1,3- and 1,4-dioxanyl, thiopyranyl, S.oxothiopyranyl, S-dioxothiopyranyl, dihydrothiopyranyl, S-oxodihydrothiopyranyl, S-dioxodihydrothiopyranyl, tetrahydrothiopyranyl, S-oxotetrahydrothiopyranyl, S-dioxotetrahydrothiopyranyl, morpholinyl, thiomorpholinyl, S-oxothiomorpholinyl, S-dioxothiomorpholinyl, thiazinyl and the like. Examples for heterocyclic ring also comprising 1 or 2 carbonyl groups as ring members comprise pyrrolidin-2-onyl, pyrrolidin-2,5-dionyl, imidazolidin-2-onyl, oxazolidin-2-onyl, thiazolidin-2-onyl and the like.

The term "partially or fully unsaturated heterocycle" or "partially or fully unsaturated heterocyclic ring" refers to heterocycle which is partially unsaturated or heterocycle which is fully unsaturated. Partially unsaturated heterocycle includes monocyclic 3- or 6-membered partially unsaturated heterocyclic radicals comprising as ring members 1, 2, 3 or 4 heteroatoms selected from N, O and S. Examples of 3- to 6-membered partially unsaturated heterocycles include azirine, oxeteen, dihydropyrol, dihydrofuran, dihydrothiophene, dihydrooxazole, dihydroimidazole, dihydrothiazole, tetrahydropyrazine, dihydrooxazine. Fully unsaturated heterocycle includes monocyclic 5- or 6-membered fully unsaturated heterocyclic radicals comprising as ring members 1, 2, 3 or 4 heteroatoms selected from N, O and S. Examples of 5- or 6-membered fully unsaturated heterocycles include pyridyl, i.e. 2-, 3-, or 4-pyridyl, pyrimidinyl, i.e. 2-, 4- or 5-pyrimidinyl, pyrazinyl, pyridazinyl, i.e. 3- or 4-pyridazinyl, thienyl, i.e. 2- or 3-thienyl, furyl, i.e. 2-or 3-furyl, pyrrolyl, i.e. 2- or 3-pyrrolyl, oxazolyl, i.e. 2-, 3- or 5-oxazolyl, isoxazolyl, i.e. 3-, 4- or 5-isoxazolyl, thiazolyl, i.e. 2-, 3- or 5-thiazolyl, isothiazolyl, i.e. 3-, 4- or 5-isothiazolyl, pyrazolyl, i.e. 1-, 3-, 4- or 5-pyrazolyl, i.e. 1-, 2-, 4- or 5-imidazolyl, oxadiazolyl, e.g. 2- or 5-[1,3,4]oxadiazolyl, 4- or 5-(1,2,3-oxadiazol)yl, 3- or 5-(1,2,4-oxadiazol)yl, 2-or 5-(1,3,4-thiadiazol)yl, thiadiazolyl, e.g. 2- or 5-(1,3,4-thiadiazol)yl, 4- or 5-(1,2,3-thiadiazol)yl, 3-or 5-(1,2,4-thiadiazol)yl, triazolyl, e.g. 1H-, 2H- or 3H-1,2,3-triazol-4-yl, 2H-triazol-3-yl, 1H-, 2H-, or 4H-1,2,4-triazolyl and tetrazolyl, i.e. 1 H- or 2H-tetrazolyl. The term "partially or fully unsaturated heterocycle" or "partially or fully unsaturated heterocyclic ring" also includes bicyclic 8 to 10-membered partially or fully unsaturated heterocyclic radicals comprising as ring members 1, 2 or 3 heteroatoms selected from N, O and S, wherein a 5- or 6-membered hetercyclic ring is fused to a phenyl ring or to a 5- or 6-membered heteroaromatic radical. Examples of a 5- or 6-membered heteroaromatic ring fused to a phenyl ring or to a 5- or 6-membered heteroaromatic radical include benzofuranyl, benzothienyl, indolyl, indazolyl, benzimidazolyl, benzoxathiazolyl, benzoxadiazolyl, benzothiadiazolyl, benzoxazinyl, chinolinyl, isochinolinyl, purinyl, 1,8-naphthyridyl, pteridyl, pyrido[3,2-d]pyrimidyl or pyridoimidazolyl and the like. These fused hetaryl radicals may be bonded to the remainder of the molecule via any ring atom of 5- or 6-membered heteroaromatic ring or via a carbon atom of the fused phenyl moiety.

The term oxo (=O) as a substitution referes to replacement of two H by oxo (=O) radical.

The term "heteroaryl" refers to "fully unsaturated heterocycle".

The terms "alkylene", "alkenylene", and "alkynylene" refer to alkyl, alkenyl, and alkynyl as defined above, respectively, which are bonded to the remainder of the molecule, via two atoms, preferably via two carbon atoms, of the respective group, so that they represent a linker between two moieties of the molecule. In particular, the term "alkylene" may refer to alkyl chains such as -CH₂CH₂-, -CH(CH₃)-, -CH₂CH₂CH₂-, -CH(CH₃)CH₂-, -CH₂CH(CH₃)-, -CH₂CH₂CH₂CH₂-, -CH₂CH₂CH₂CH₂CH₂-, -CH₂CH₂CH₂CH₂CH₂CH₂-, and -CH₂CH₂CH₂CH₂CH₂CH₂CH₂-. Similarly, "alkenylene" and "alkynylene" may refer to alkenyl and alkynyl chains, respectively.

The term "CN" refers to cyano group.

With respect to the variables, the particularly preferred embodiments of the compounds of the formula I are given below.

In one preferred embodiment of compounds of formula I, R¹ is C₂-C₆-alkenyl-CN, C₂-C₆-alkenylS(=O)ₘR^{e}, C(O)-R^{d}, SO₂NR^{b}R^{c}, or S(=O)ₘR^{e}, which are unsubstituted or substituted with R^{a};
In another preferred embodiment of compounds of formula I, R¹ is CN, C(O)-OR^{b}, C(O)NR^{b}R^{c}, C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b} which are unsubstituted or substituted with R^{a};
In another preferred embodiment of compounds of formula I, R¹ is CN, C(O)-OR^{b}, C(O)NR^{b}R^{c}, which are unsubstituted or substituted with R^{a};
In another preferred embodiment of compounds of formula I, R¹ is C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b} which are unsubstituted or substituted with R^{a};
In another preferred embodiment of compounds of formula I, R¹ is CN, C(O)-OR^{b}, C(O)NR^{b}R^{c}, C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b} which are substituted with R^{a};
In another preferred embodiment of compounds of formula I, R¹ is CN, C(O)-OR^{b}, C(O)NR^{b}R^{c}, which are substituted with R^{a};
In another preferred embodiment of compounds of formula I, R¹ is C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b} which are substituted with R^{a}.

In another preferred embodiment of compounds of formula I, R¹ is CN, C(O)-OR^{b}, C(O)NR^{b}R^{c}, C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b} which are unsubstituted.

In another preferred embodiment of compounds of formula I, R¹ is CN, C(O)-OR^{b}, C(O)NR^{b}R^{c}, which are unsubstituted.

In another preferred embodiment of compounds of formula I, R¹ is C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b} which are unsubstituted.

In another preferred embodiment of compounds of formula I, R¹ is C(O)-OR^{b} or C(O)NR^{b}R^{c};
In another preferred embodiment of compounds of formula I, R¹ is C₂-C₆-alkenyl-C₁-C₆-alkyl, C₂-C₆-alkenyl-C₁-C₆-haloalkyl, C₂-C₆-alkenyl-NR^{b}R^{c}, C₂-C₆-alkenyl-C(O)-OR^{b}, C₂-C₆-alkenyl-C(O)-R^{b};
In more preferred embodiment of compounds of formula I, R¹ is C(O)-OR^{b}, C(O)NR^{b}R^{c}, or C₂-C₆-alkenyl-C₁-C₆-haloalkyl;
In most preferred embodiment of compounds of formula I, R¹ is C(O)-OR^{b};
In another most preferred embodiment of compounds of formula I, R¹ is C(O)NR^{b}R^{c};
In another most preferred embodiment of compounds of formula I, R¹ is C₂-C₆-alkenyl-C₁-C₆-haloalkyl;
In one preferred embodiment of compounds of formula I, R² is H, halogen, N₃, NO₂, -SCN, -SF₅, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cyclo-alkoxyx-C₁-C₄-alkyl, NR^{b}R^{c}, C(O)-R^{d}, SO₂NR^{b}R^{c} and S(=O)ₘR^{e}, which are unsubstituted or substituted with R^{a};
In another preferred embodiment of compounds of formula I, R² is CN, C(O)-OR^{b}, C(O)NR^{b}R^{c}, C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b} which are unsubstituted or substituted with R^{a};
In another preferred embodiment of compounds of formula I, R² is CN, C(O)-OR^{b}, C(O)NR^{b}R^{c}, which are unsubstituted or substituted with R^{a};
In another preferred embodiment of compounds of formula I, R² is C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b} which are unsubstituted or substituted with R^{a};
In another preferred embodiment of compounds of formula I, R² is CN, C(O)-OR^{b}, C(O)NR^{b}R^{c}, C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b} which are substituted with R^{a};
In another preferred embodiment of compounds of formula I, R² is CN, C(O)-OR^{b}, C(0)NR^{b}R^{c}, which are substituted with R^{a};
In another preferred embodiment of compounds of formula I, R² is C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b} which are substituted with R^{a}.

In another preferred embodiment of compounds of formula I, R² is CN, C(O)-OR^{b}, C(O)NR^{b}R^{c}, C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b} which are unsubstituted.

In another preferred embodiment of compounds of formula I, R² is CN, C(O)-OR^{b}, C(O)NR^{b}R^{c}, which are unsubstituted.

In another preferred embodiment of compounds of formula I, R² is C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b} which are unsubstituted.

In another preferred embodiment of compounds of formula I, R² is C(O)-OR^{b} or C(O)NR^{b}R^{c};

In another preferred embodiment of compounds of formula I, R² is C₂-C₆-alkenyl-C₁-C₆-alkyl, C₂-C₆-alkenyl-C₁-C₆-haloalkyl, C₂-C₆-alkenyl-NR^{b}R^{c}, C₂-C₆-alkenyl-C(O)-OR^{b}, C₂-C₆-alkenyl-C(O)-R^{b};

In more preferred embodiment of compounds of formula I, R² is C(O)-OR^{b}, C(O)NR^{b}R^{c}, or C₂-C₆-alkenyl-C₁-C₆-haloalkyl;

In most preferred embodiment of compounds of formula I, R² is C(O)-OR^{b};

In another most preferred embodiment of compounds of formula I, R² is C(O)NR^{b}R^{c};

In another most preferred embodiment of compounds of formula I, R² is C₂-C₆-alkenyl-C₁-C₆-haloalkyl.

In another preferred embodiment of compounds of formula I, R¹ and R² independently of each other are selected from CN, C(O)-OR^{b}, C(O)NR^{b}R^{c}, C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b}, which are unsubstituted or substituted with R^{a}, wherein
- R^{a}: is selected from halogen, OH, CN, NO₂, NR^{b}R^{c}, C(O)-OR^{b}, and S(=O)ₘR^{e};
- R^{b}: is selected from H, C₁-C₆-alkyl, C₁-C₆-haloalkyl, C₃-C₆-cycloalkyl, C₂-C₆-alkenyl, and C₂-C₆-alkynyl;
- R^{c}: is selected from H, C₁-C₆-alkyl, C₁-C₆-haloalkyl, and C₃-C₆-cycloalkyl.

In one preferred embodiment of compounds of formula I, R³ is H, C₁-C₆-alkyl, C₁-C₆-alkoxy-C₁-C₄-alkyl, C₃-C₆-cycloalkyl, or C₃-C₆-cycloalkyl-C₁-C₄-alkyl, which are unsubstituted or substituted with R^{a}.

In another preferred embodiment of compounds of formula I, R³ is C₁-C₆-alkoxy-C₁-C₄-alkyl, or C₃-C₆-cycloalkyl-C₁-C₄-alkyl, which are unsubstituted or substituted with R^{a};
In more preferred embodiment of compounds of formula I, R³ is H, C₁-C₆-alkyl, or C₃-C₆-cycloalkyl;
In another more preferred embodiment of compounds of formula I, R³ is C₁-C₆-alkyl, or C₃-C₆-cycloalkyl;
In another more preferred embodiment of compounds of formula I, R³ is C₁-C₆-alkyl;
In another more preferred embodiment of compounds of formula I, R³ is CH₃, C₂H₅, n-propyl, iso-propyl, or cyclopropyl;
In most preferred embodiment of compounds of formula I, R³ is H;
In another most preferred embodiment of compounds of formula I, R³ is CH₃;
In another most preferred embodiment of compounds of formula I, R³ is C₂H₅;
In another most preferred embodiment of compounds of formula I, R³ is n-propyl, isopropyl, or cyclopropyl.

In one preferred embodiment of compounds of formula I, R^{a} is selected from halogen, OH, CN, NO₂, NR^{b}R^{c}, C(O)-OR^{b}, and S(=O)ₘR^{e};
In another preferred embodiment of compounds of formula I, R^{a} is selected from C₁-C₆-alkyl, C₁-C₆-alkoxy, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, and C₃-C₆-cycloalkoxy-C₁-C₄-alkyl;
In more preferred embodiment of compounds of formula I, R^{a} is selected from halogen, OH, CN, NR^{b}R^{c}, C(O)-OR^{b}, and S(=O)ₘR^{e};
In most preferred embodiment of compounds of formula I, R^{a} is halogen;
In another most preferred embodiment of compounds of formula I, R^{a} is OH or CN;
In another most preferred embodiment of compounds of formula I, R^{a} is NR^{b}R^{c};
In another most preferred embodiment of compounds of formula I, R^{a} is C(O)-OR^{b};
In another most preferred embodiment of compounds of formula I, R^{a} is S(=O)ₘR^{e}.

In one preferred embodiment of compounds of formula I, R^{b} is selected from H, C₁-C₆-alkyl, C₂-C₆-alkenyl, C₂-C₆-alkynyl, C₁-C₆-alkoxy-C₁-C₄-alkyl, C₁-C₆-alkylthio-C₁-C₄-alkyl, C₃-C₆-cycloalkyl, C₃-C₆-cycloalkyl-C₁-C₄-alkyl, and C₃-C₆-cycloalkoxy-C₁-C₄-alkyl;
In another preferred embodiment of compounds of formula I, R^{b} is selected from H, C₁-C₆-alkyl, C₁-C₆-haloalkyl, C₃-C₆-cycloalkyl, C₂-C₆-alkenyl, and C₂-C₆-alkynyl;
In more preferred embodiment of compounds of formula I, R^{b} is selected from H, CH₃, C₂H₅, n-propyl, isopropyl, cyclopropyl, allyl and propargyl.

In most preferred embodiment of compounds of formula I, R^{b} is selected from H, CH₃, C₂H₅, n-propyl, isopropyl, and cyclopropyl.

In a preferred embodiment of compounds of formula I, R^{c} is selected from H, C₁-C₆-alkyl, C₂-C₆-alkenyl, C₂-C₆-alkynyl, C₃-C₆-cycloalkyl, and C₁-C₆-haloalkyl,.
In more preferred embodiment of compounds of formula I, R^{c} is selected from H, C₁-C₆-alkyl, and C₃-C₆-cycloalkyl;
In most preferred embodiment of compounds of formula I, R^{c} is selected from H, CH₃, C₂H₅, n-propyl, isopropyl, cyclopropyl, allyl and propargyl, which are unsubstituted or substituted with halogen.

In another preferred embodiment of compounds of formula I, each moiety NR^{b}R^{c} may also form an N-bound, saturated 3- to 8-membered heterocycle, which in addition to the nitrogen atom may have 1 or 2 further heteroatoms or heteroatom moieties selected from O, S(=O)ₘ and N-R', wherein R' is H, -C(=O)OR, or C₁-C₆-alkyl and wherein the N-bound heterocycle is unsubstituted or substituted with radicals selected from halogen, C₁-C₄-alkyl, C₁-C₄-haloalkyl, C₁-C₄-alkoxy and C₁-C₄-haloalkoxy
In a preferred embodiment of compounds of formula I, R^{d} is selected from H, C₁-C₆-alkyl, C₁-C₆-haloalkyl, C₃-C₆-cycloalkyl, C₂-C₆-alkenyl, and C₂-C₆-alkynyl;
In more preferred embodiment of compounds of formula I, R^{d} is selected from H, CH₃, C₂H₅, n-propyl, isopropyl, cyclopropyl, allyl and propargyl.

In most preferred embodiment of compounds of formula I, R^{d} is selected from H, CH₃, C₂H₅, n-propyl, isopropyl, and cyclopropyl.

In a preferred embodiment of compounds of formula I, Re is selected from C₁-C₆-alkyl, C₃-C₆-cycloalkyl, C₁-C₆-haloalkyl, and C₃-C₆ halocycloalkyl.

In another preferred embodiment of compounds of formula I, R^{e} is selected from CH₃, C₂H₅, n-propyl, isopropyl, cyclopropyl, allyl, and propargyl.

In one preferred embodiment of compounds of formula I, R^{f} is selected from halogen, CN, C₁-C₆-alkyl, C₁-C₆-alkoxy, C₂-C₆-alkenyl, C₂-C₆-alkynyl, C₃-C₆-cycloalkyl, C₁-C₆-haloalkyl, C₁-C₆-haloalkoxy, C₂-C₆-haloalkenyl, C₂-C₆-haloalkynyl, and C₃-C₆ halocycloalkyl.

In another preferred embodiment of compounds of formula I, R^{f} is selected from halogen, C₁-C₆-alkyl, C₁-C₆-alkoxy, C₃-C₆-cycloalkyl, C₂-C₆-alkenyl, and C₂-C₆-alkynyl, which are unsubstituted or substituted with halogen;
In more preferred embodiment of compounds of formula I, R^{f} is selected from Cl, F, Br, OCH₃, OC₂H₅, SCH₃, SC₂H₅, CN, CH₃, C₂H₅, n-propyl, isopropyl, cyclopropyl, allyl, propargyl, CF₃, CHF₂, and CF₂CF₃.

In one preferred embodiment of compounds of formula I, m is 0;
In another preferred embodiment of compounds of formula I, m is 1 or 2;
In more preferred embodiment of compounds of formula I, m is 1;

In another more preferred embodiment of compounds of formula I, m is 2.

In one preferred embodiment of compounds of formula I, Ar is phenyl;
In another preferred embodiment of compound of formula I, Ar is heteroaryl;
In another preferred embodiment of compound of formula I, Ar is 5- or 6-membered heteroaryl containing one heteroatom selected from O, N, and S;
In more preferred embodiment of compound of formula I, Ar is phenyl or 5-6 membered heteroaryl substituted with S(=O)ₘR^{e} at the ortho position to the bond connecting to 5-membered heteroaryl of compound of formula I, and optionally further substituted with 1 R^{Ar}, preferably selected from halogen, C₁-C₆-alkyl, C₁-C₆-haloalkyl, C₁-C₆-alkoxy, C₁-C₆-haloalkoxy, phenyl, and benzyl, wherein the phenyl ring is unsubstituted or substituted by radicals R^{f};
In another preferred embodiment of compounds of formula I, Ar is selected from formula Ar-1 to Ar-9, wherein one R^{Ar} substituent is preferably at the ortho position to 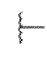 bond;

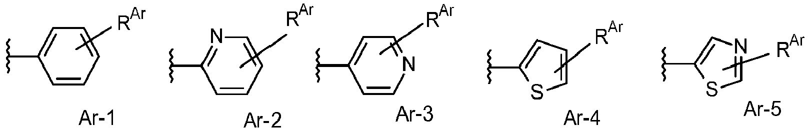

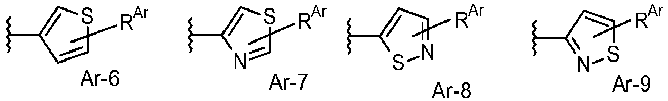

In another preferred embodiment of compounds of formula I, Ar is pyridinyl or thiophenyl substituted with R^{Ar} as S(=O)ₘR^{e} at the ortho position to bond connecting to 5-membered heteroaryl of compound of formula I, and optionally further substituted with 1 R^{Ar} selected from halogen, C₁-C₆-alkyl, C₁-C₆-haloalkyl, C₁-C₆-alkoxy, C₁-C₆-haloalkoxy, phenyl, and benzyl, wherein the phenyl ring of R^{Ar} is unsubstituted or substituted with radicals R^{f};
In more preferred embodiment of compound of formula I, Ar is is pyridinyl substituted with R^{Ar} as S(=O)ₘR^{e} at the ortho position to bond connecting to imidazolyl ring of compound of formula I, and optionally further substituted with 1 R^{Ar} selected from halogen, C₁-C₆-alkyl, C₁-C₆-haloalkyl, C₁-C₆-alkoxy, C₁-C₆-haloalkoxy, phenyl, and benzyl, wherein the phenyl ring of R^{Ar} is unsubstituted or substituted with radicals R^{f};
In one preferred embodiment of compounds of formula I, R^{Ar} is selected from halogen, C₁-C₆-alkyl, C₁-C₆-haloalkyl, C₁-C₆-alkoxy, C₁-C₆-haloalkoxy, S(=O)ₘR^{e}, phenyl, phenoxy, phenylcarbonyl, phenylthio and benzyl, wherein the phenyl ring is unsubstituted or substituted by radicals R^{f}.

In more preferred embodiment of compounds of formula I, R^{Ar} is selected from halogen, C₁-C₆-alkyl, C₁-C₆-haloalkyl, C₁-C₆-alkoxy, C₁-C₆-haloalkoxy, S(=O)ₘR^{e}, phenyl, and benzyl, wherein the phenyl ring is unsubstituted or substituted by radicals R^{f}.

In one preferred embodiment of compounds of formula I, R^{Ar} is selected from halogen, C₁-C₆-alkyl, C₁-C₆-haloalkyl, C₁-C₆-alkoxy, and C₁-C₆-haloalkoxy.

In another preferred embodiment of compounds of formula I, R^{Ar} is selected from C₂-C₆-alkenyl, C₂-C₆-haloalkenyl, C₂-C₆-alkynyl, C₂-C₆-haloalkynyl, C₃-C₆-cycloalkyl, C₃-C₆ halocycloalkyl, C₃-C₆-cycloalkoxy and C₃-C₆halocycloalkoxy.

In another preferred embodiment of compounds of formula I, R^{Ar} is selected from C₁-C₆-sulfenyl, C₁-C₆-sulfinyl and C₁-C₆-sulfonyl.

In more preferred embodiment of compounds of formula I, R^{Ar} is selected from Cl, F, Br, CH₃, SCH₃, SC₂H₅, S(=O)CH₃, S(=O)C₂H₅, S(=O)₂CH₃, S(=O)₂C₂H₅.

In most preferred embodiment of compounds of formula I, R^{Ar} is S(=O)₂C₂H₅.

In a preferred embodiment of the compound of formula I, n is 1 or 2.

In more preferred embodiment of the compound of formula I, n is 1.

In a particularly preferred embodiment of compound of formula I, Ar is selected from the group of formula Ar-1a to Ar-1f, Ar-2a to Ar-2f, Ar-3a to Ar-3c, Ar-4a to Ar-4f, Ar-7a to Ar-7c, Ar-8a to Ar-8c, and Ar-9a to Ar-9c:

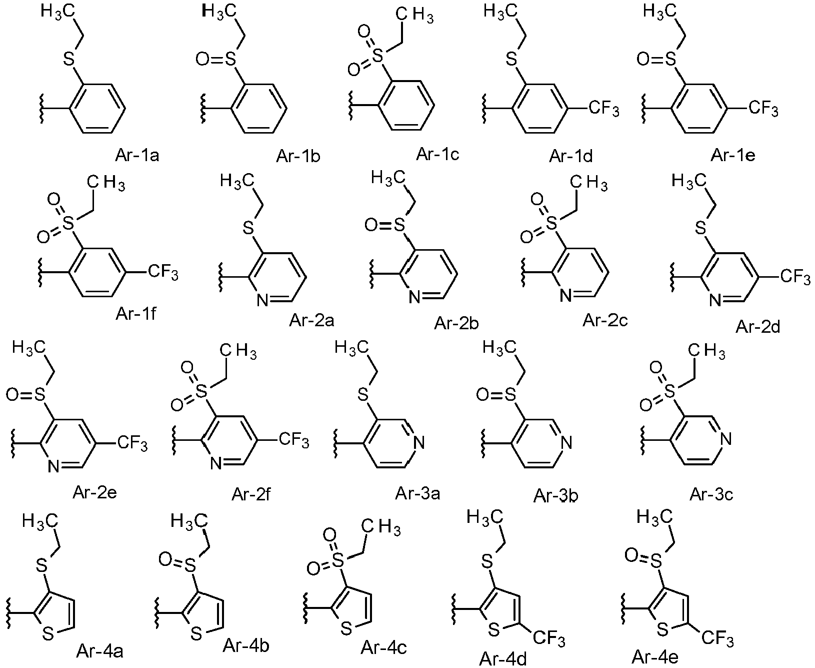

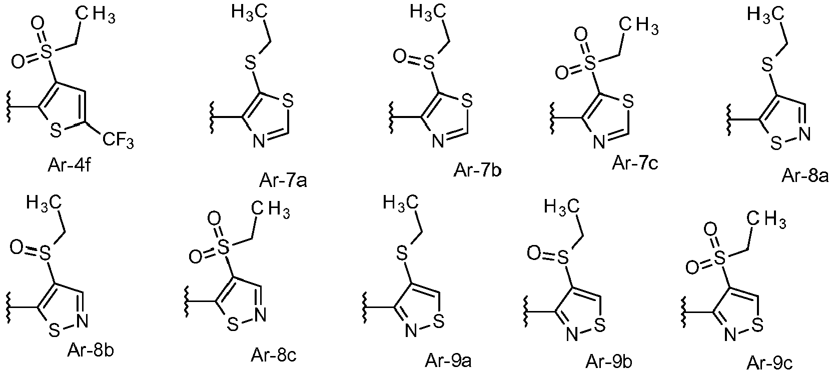

In more preferred embodiment of the compound of formula I,
- Ar: is a phenyl or 5- or 6-membered heteroaryl which is substituted with R^{Ar};
- R^{Ar}: is selected from halogen, C₁-C₆-alkyl, C₁-C₆-haloalkyl, C₁-C₆-alkoxy, C₁-C₆-haloalkoxy, S(=O)ₘR^{e}, phenyl, phenoxy, phenylcarbonyl, phenylthio and benzyl, wherein the phenyl ring is unsubstituted or substituted with radicals R^{f};
- R^{e}: is selected from C₁-C₆-alkyl, C₃-C₆- cycloalkyl, C₁-C₆-haloalkyl, and C₃-C₆- halocycloalkyl;
- R^{f}: is selected from halogen, C₁-C₆-alkyl, C₁-C₆-alkoxy, C₃-C₆-cycloalkyl, C₂-C₆-alkenyl, and C₂-C₆-alkynyl, which are unsubstituted or substituted with halogen.

In another more preferred embodiment of the compound of formula I,
- R¹ and R²: independently of each other are selected from CN, C(O)-OR^{b}, C(O)NR^{b}R^{c}, C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b}, which are unsubstituted or substituted with R^{a};
- R³: is C₁-C₆-alkyl;
- Ar: is pyridinyl substituted with R^{Ar} as S(=O)ₘR^{e} at the ortho position to bond connecting to imidazolyl ring of compound of formula I, and optionally further substituted with 1 R^{Ar} selected from halogen, C₁-C₆-alkyl, C₁-C₆-haloalkyl, C₁-C₆-alkoxy, C₁-C₆-haloalkoxy, phenyl, and benzyl, wherein the phenyl ring of R^{Ar} is unsubstituted or substituted with radicals R^{f};
- R^{a}: is selected from halogen, OH, CN, NO₂, NR^{b}R^{c}, C(O)-OR^{b}, and S(=O)ₘR^{e};
- R^{b}: is selected from H, C₁-C₆-alkyl, C₁-C₆-haloalkyl, C₃-C₆-cycloalkyl, C₂-C₆-alkenyl, and C₂-C₆-alkynyl;
- R^{c}: is selected from H, C₁-C₆-alkyl, and C₃-C₆-cycloalkyl;
- R^{e}: is selected from C₁-C₆-alkyl, C₃-C₆-cycloalkyl, C₁-C₆-haloalkyl, and C₃-C₆ halocycloalkyl;
- R^{f}: is selected from halogen, C₁-C₆-alkyl, C₁-C₆-alkoxy, C₃-C₆-cycloalkyl, C₂-C₆-alkenyl, and C₂-C₆-alkynyl, which are unsubstituted or substituted with halogen;
- m: is 0, 1, or 2.

Particularly preferred are the compounds of formula I, wherein
- R¹ and R²: independently of each other are selected from C(O)-OC₂H₅, C(O)NH₂, C(O)NHCH₃, C(O)NHC₂H₅, CF₃, CH₂-CH₂-CF₃, and CH=CH-CF₃;
- R³: is CH₃;

- Ar: is selected from Ar-1a to Ar-1f, Ar-2a to Ar-2f, Ar-3a to Ar-3c, Ar-4a to Ar-4f, Ar-7a to Ar-7c, Ar-8a to Ar-8c, and Ar-9a to Ar-9c;

More particularly preferred are the compounds of formula I, wherein the combinations of variables R¹, R², R³ and Ar corresponds to each row of Table T; each of the groups mentioned for a substituent in the tables is furthermore per se, independently of the combination in which it is mentioned, a particularly preferred aspect of the substituent in question.

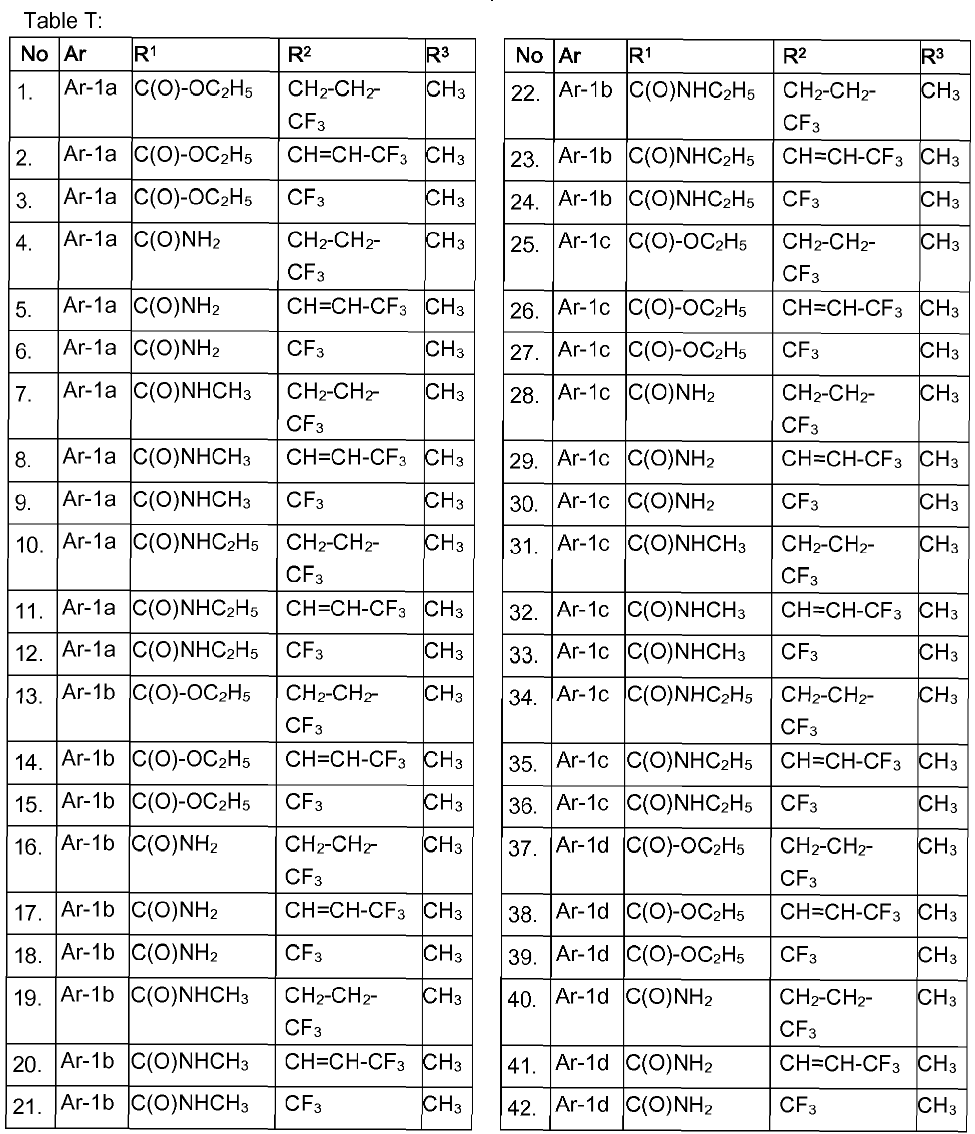

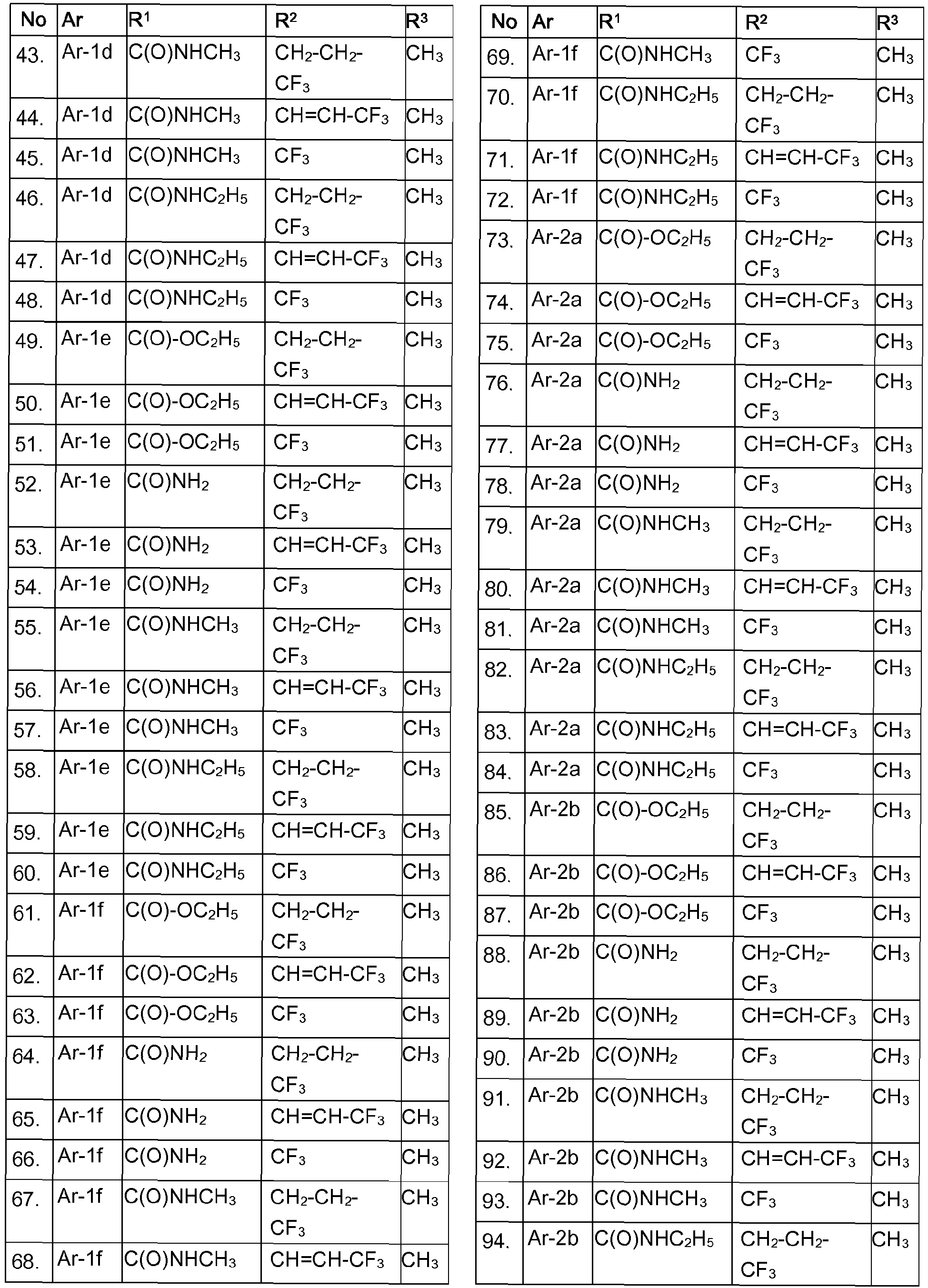

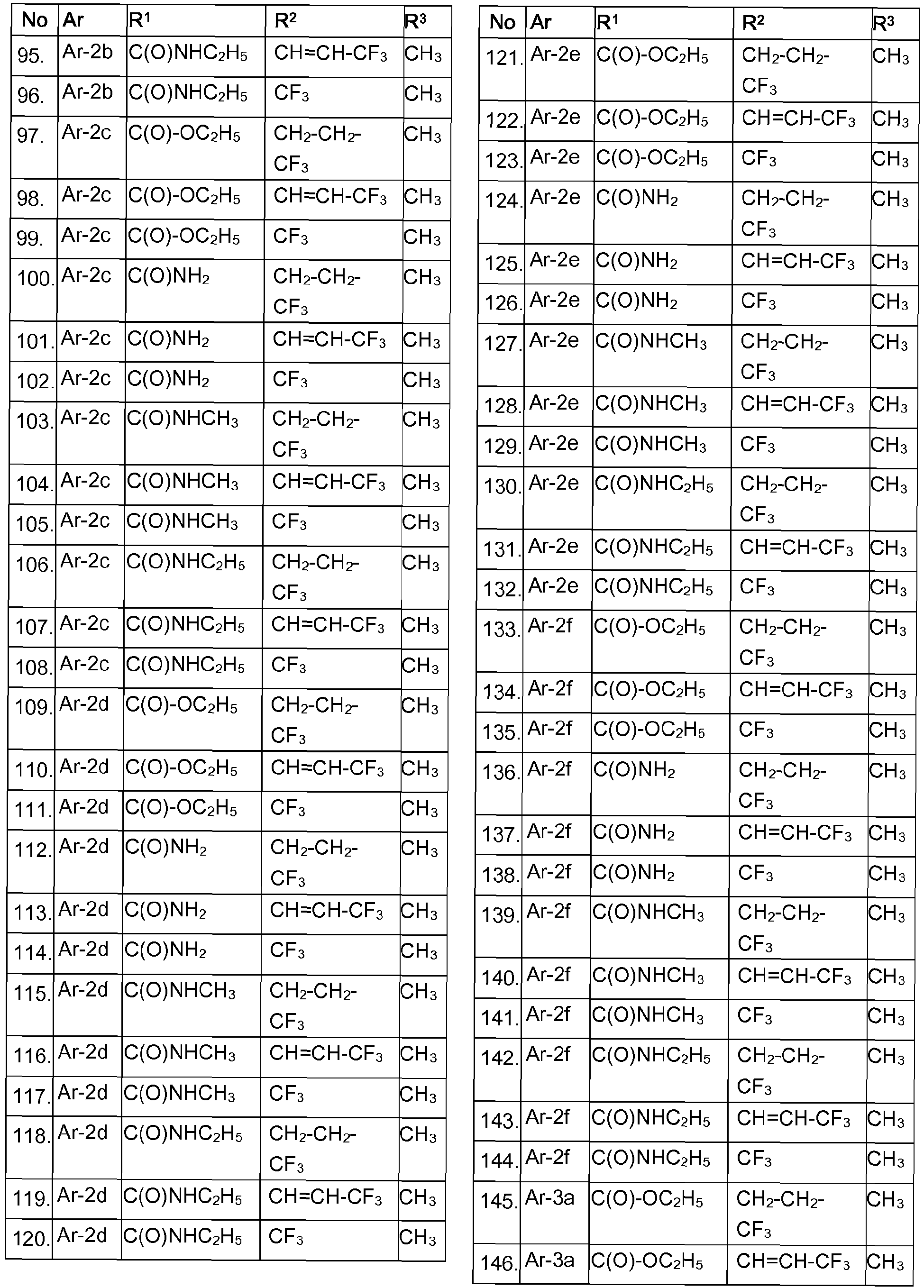

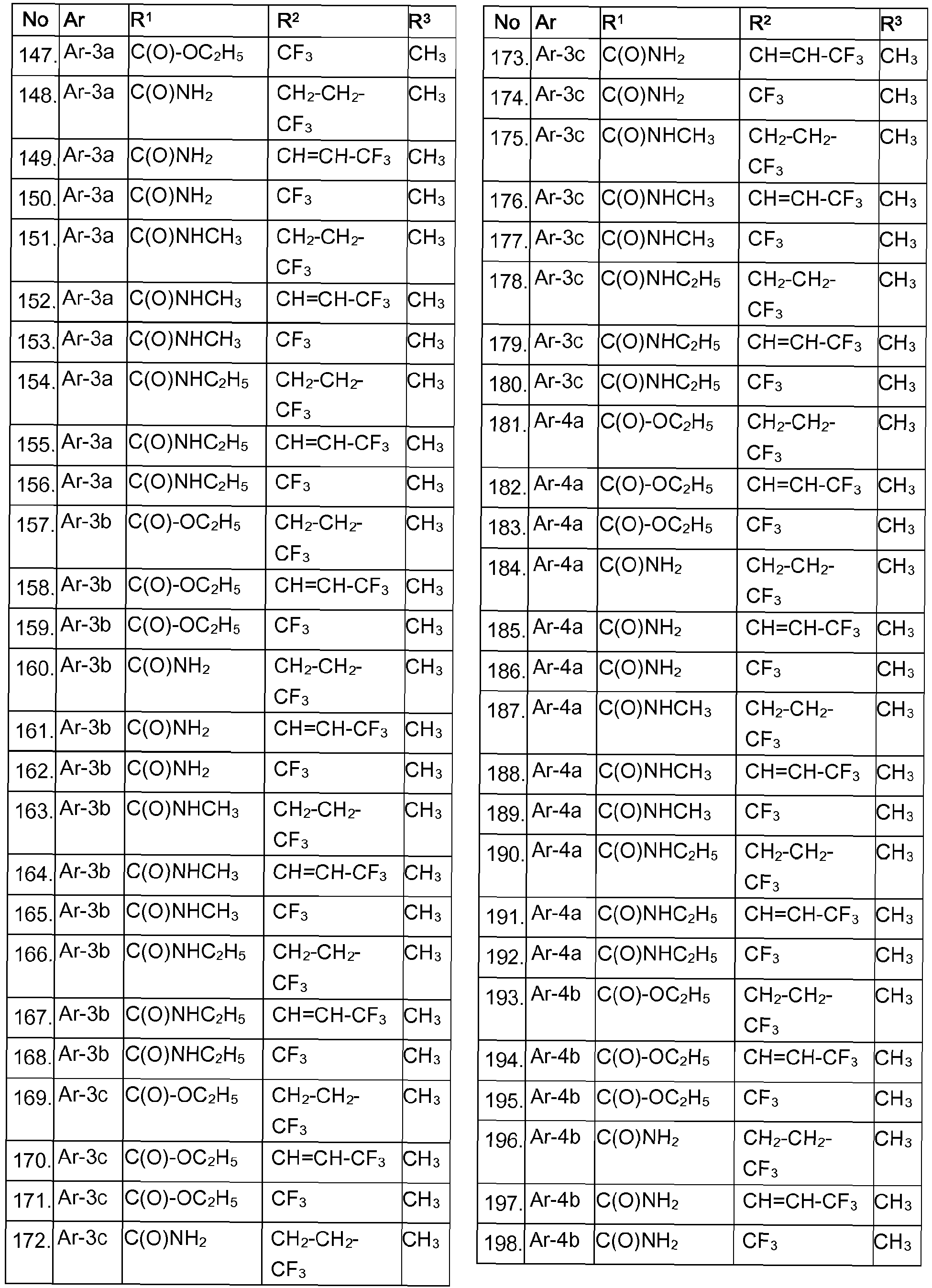

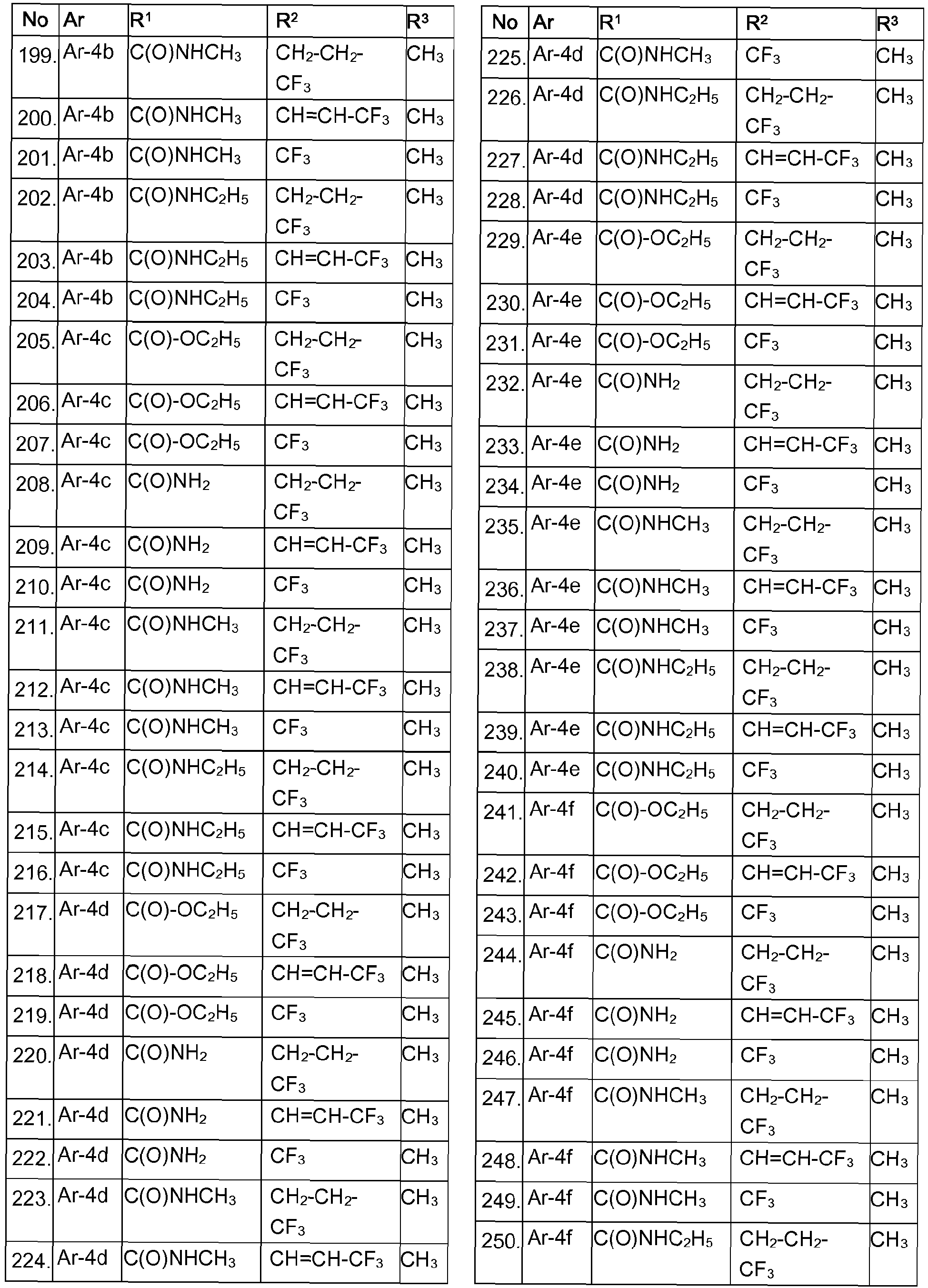

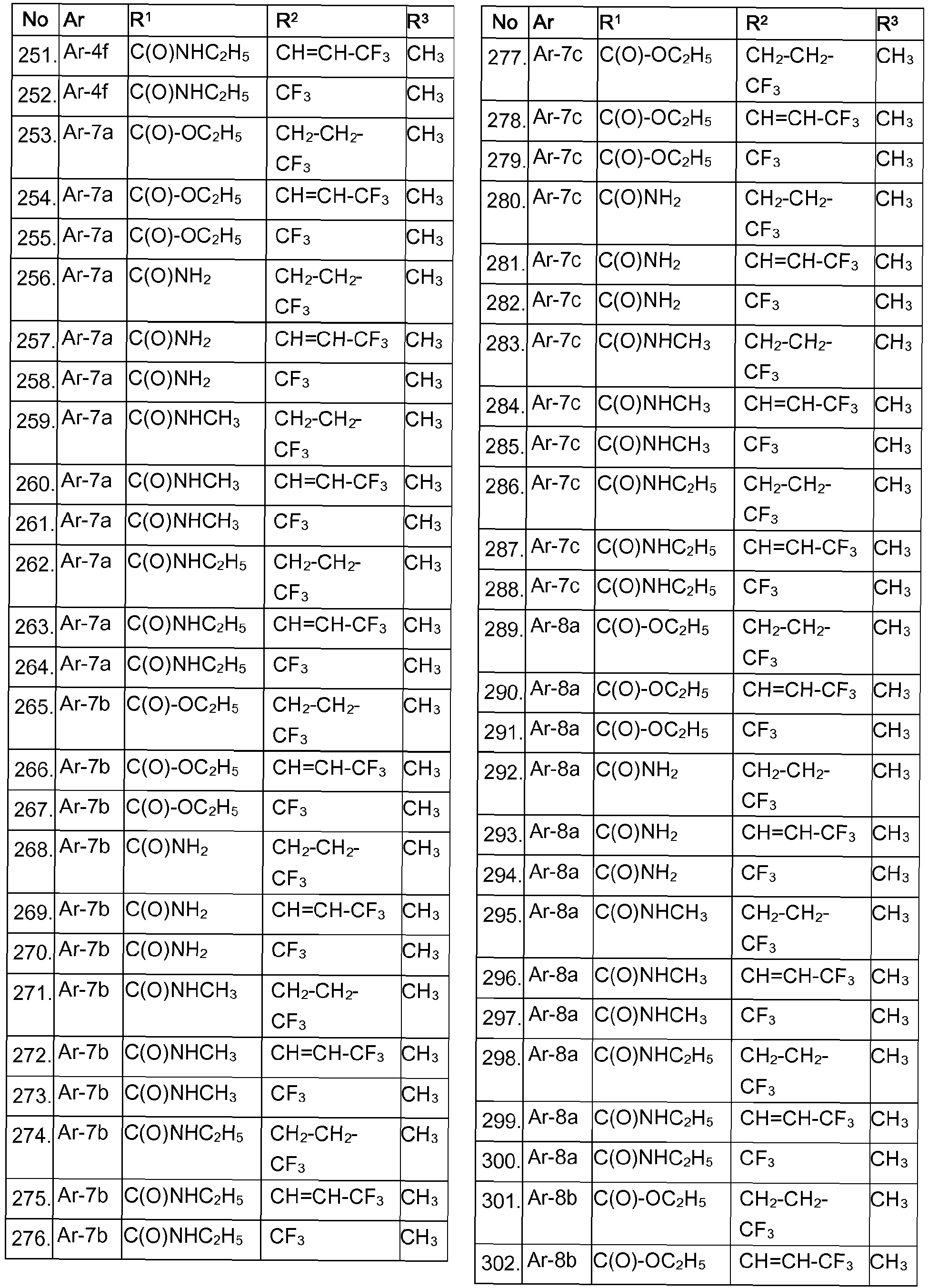

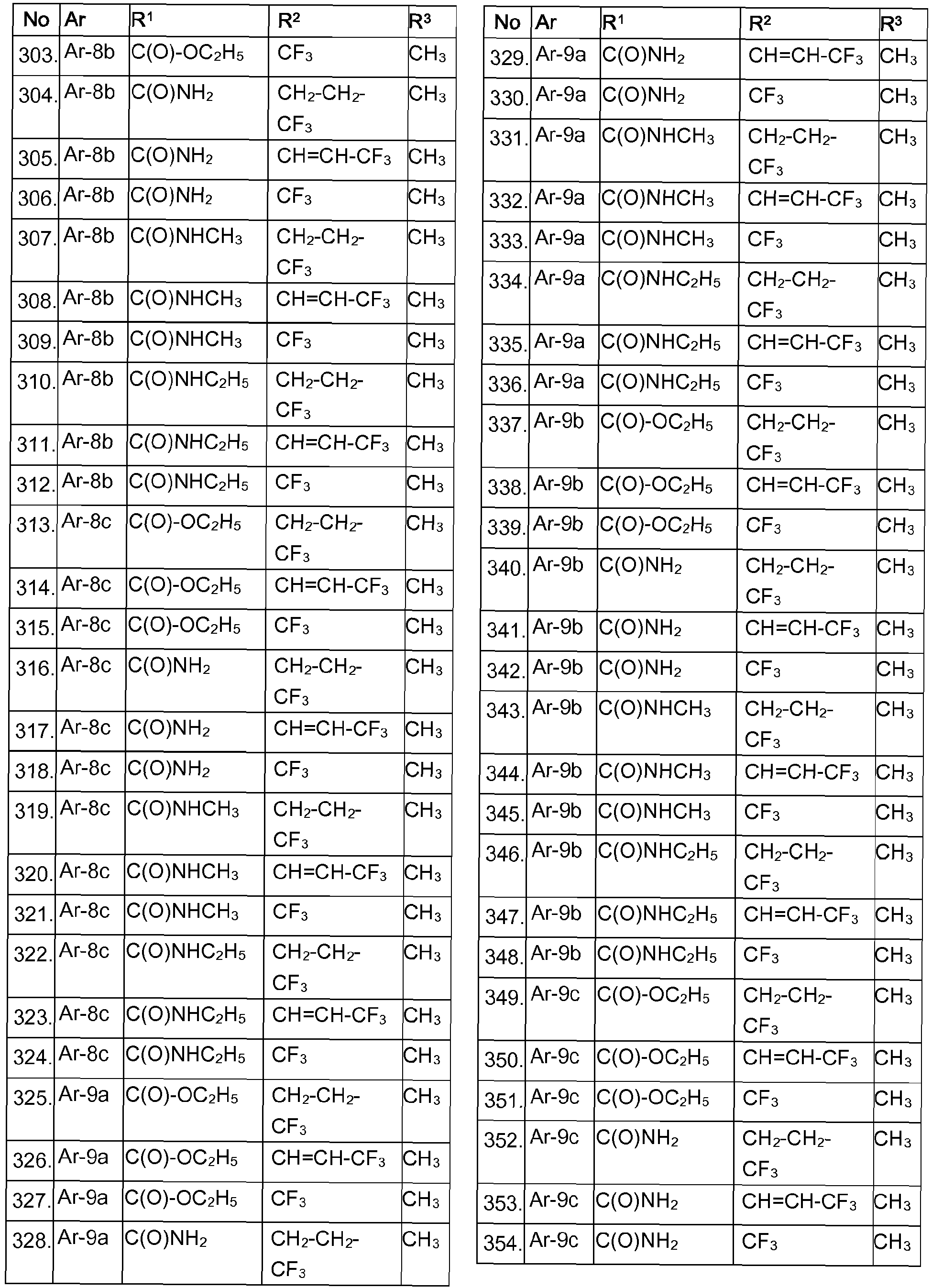

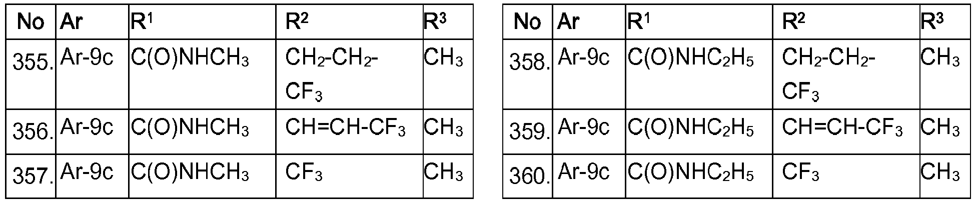

As used herein, the term "compound(s) of the present invention" or "compound(s) according to the invention" refers to the compound(s) of formula (I) as defined above, which are also referred to as "compound(s) of formula I" or "compound(s) I" or "formula I compound(s)", and includes their salts, tautomers, stereoisomers, and N-oxides.

The present invention also relates to a mixture of at least one compound of the invention with at least one mixing partner as defined herein. Preferred are binary mixtures of one compound of the invention as component I with one mixing partner as defined herein as component II. Preferred weight ratios for such binary mixtures are from 5000:1 to 1:5000, preferably from 1000:1 to 1:1000, more preferably from 100:1 to 1:100, particularly from 10:1 to 1:10. In such binary mixtures, components I and II may be used in equal amounts, or an excess of component I, or an excess of component II may be used.

Mixing partners can be selected from pesticides, in particular insecticides, nematicides, and acaricides, fungicides, herbicides, plant growth regulators, fertilizers. Preferred mixing partners are insecticides, nematicides and fungicides.

The following list M of pesticides, grouped and numbered according the Mode of Action Classification of the Insecticide Resistance Action Committee (IRAC), together with which the compounds of the invention can be used and with which potential synergistic effects might be produced, is intended to illustrate the possible combinations, but not to impose any limitation:
M.1 Acetylcholine esterase (AChE) inhibitors: M.1A carbamates, e.g. aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb and triazamate; or M.1 B organophosphates, e.g. acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos- methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, and vamidothion;
M.2. GABA-gated chloride channel antagonists: M.2A cyclodiene organochlorine compounds, e.g. endosulfan or chlordane; or M.2B fiproles (phenylpyrazoles), e.g. ethiprole, fipronil, flufiprole, pyrafluprole, and pyriprole;
M.3 Sodium channel modulators from the class of M.3A pyrethroids, e.g. acrinathrin, allethrin, d-cis-trans allethrin, d-trans allethrin, bifenthrin, bioallethrin, bioallethrin S-cylclopentenyl, bioresmethrin, cycloprothrin, cyfluthrin, beta-cyfluthrin, cyhalothrin, lambda-cyhalothrin, gammacyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin, metofluthrin, momfluorothrin, permethrin, phenothrin, prallethrin, profluthrin, pyrethrin (pyrethrum), resmethrin, silafluofen, tefluthrin, tetramethylfluthrin, tetramethrin, tralomethrin, and transfluthrin; or M.3B sodium channel modulators such as DDT or methoxychlor;
M.4 Nicotinic acetylcholine receptor agonists (nAChR): M.4A neonicotinoids, e.g. acetamiprid, clothianidin, cycloxaprid, dinotefuran, imidacloprid, nitenpyram, thiacloprid and thiamethoxam; or the compounds M.4A.1 4,5-Dihydro-N-nitro-1-(2-oxiranylmethyl)-1H-imidazol-2-amine, M.4A.2: (2E-)-1-[(6-Chloropyridin-3-yl)methyl]-N'-nitro-2-pentylidenehydrazinecarboximidamide; or M4.A.3: 1-[(6-Chloropyridin-3-yl)methyl]-7-methyl-8-nitro-5-propoxy-1,2,3,5,6,7-hexahydroimidazo[1,2-a]pyridine; or M.4B nicotine; M.4C sulfoxaflor; M.4D flupyradifurone; M.4E triflumezopyrim;
M.5 Nicotinic acetylcholine receptor allosteric activators:spinosyns, e.g. spinosad or spinetoram;
M.6 Chloride channel activators from the class of avermectins and milbemycins, e.g. abamectin, emamectin benzoate, ivermectin, lepimectin, or milbemectin;
M.7 Juvenile hormone mimics, such as M.7A juvenile hormone analogues hydroprene, kinoprene, and methoprene; or M.7B fenoxycarb, or M.7C pyriproxyfen;
M.8 miscellaneous non-specific (multi-site) inhibitors, e.g. M.8A alkyl halides as methyl bromide and other alkyl halides, M.8B chloropicrin, M.8C sulfuryl fluoride, M.8D borax, or M.8E tartar emetic;
M.9 Chordotonal organ TRPV channel modulators, e.g. M.9B pymetrozine; pyrifluquinazon;
M.10 Mite growth inhibitors, e.g. M.10A clofentezine, hexythiazox, and diflovidazin, or M.10B etoxazole;
M.11 Microbial disruptors of insect midgut membranes, e.g. *bacillus thuringiensis* or *bacillus sphaericus* and the insecticdal proteins they produce such as *bacillus thuringiensis subsp. israelensis, bacillus sphaericus, bacillus thuringiensis subsp. aizawai, bacillus thuringiensis subsp. kurstaki* and *bacillus thuringiensis subsp. tenebrionis,* or the Bt crop proteins: Cry1Ab, Cry1Ac, Cry1Fa, Cry2Ab, mCry3A, Cry3Ab, Cry3Bb, and Cry34/35Ab1;
M.12 Inhibitors of mitochondrial ATP synthase, e.g. M.12A diafenthiuron, or M.12B organotin miticides such as azocyclotin, cyhexatin, or fenbutatin oxide, M.12C propargite, or M.12D tetradifon;
M.13 Uncouplers of oxidative phosphorylation via disruption of the proton gradient, e.g. chlorfenapyr, DNOC, or sulfluramid;
M.14 Nicotinic acetylcholine receptor (nAChR) channel blockers, e.g. nereistoxin analogues bensultap, cartap hydrochloride, thiocyclam, or thiosultap sodium;
M.15 Inhibitors of the chitin biosynthesis type 0, such as benzoylureas e.g. bistrifluron, chlorfluazuron, diflubenzuron, flucycloxuron, flufenoxuron, hexaflumuron, lufenuron, novaluron, noviflumuron, teflubenzuron, or triflumuron;
M.16 Inhibitors of the chitin biosynthesis type 1, e.g. buprofezin;
M.17 Moulting disruptors, Dipteran, e.g. cyromazine;
M.18 Ecdyson receptor agonists such as diacylhydrazines, e.g. methoxyfenozide, tebufenozide, halofenozide, fufenozide, or chromafenozide;
M.19 Octopamin receptor agonists, e.g. amitraz;
M.20 Mitochondrial complex III electron transport inhibitors, e.g. M.20A hydramethylnon, M.20B acequinocyl, M.20C fluacrypyrim; or M.20D bifenazate;
M.21 Mitochondrial complex I electron transport inhibitors, e.g. M.21A METI acaricides and insecticides such as fenazaquin, fenpyroximate, pyrimidifen, pyridaben, tebufenpyrad or tolfenpyrad, or M.21B rotenone;
M.22 Voltage-dependent sodium channel blockers, e.g. M.22A indoxacarb, M.22B metaflumizone, or M.22B.1: 2-[2-(4-Cyanophenyl)-1-[3-(trifluoromethyl)phenyl]ethylidene]-N-[4-(difluoromethoxy)phenyl]-hydrazinecarboxamide or M.22B.2: N-(3-Chloro-2-methylphenyl)-2-[(4-chlorophenyl)[4-[methyl(methylsulfonyl)amino]phenyl]methylene]-hydrazinecarboxamide;
M.23 Inhibitors of the of acetyl CoA carboxylase, such as tetronic and tetramic acid derivatives, e.g. spirodiclofen, spiromesifen, or spirotetramat; M.23.1 spiropidion
M.24 Mitochondrial complex IV electron transport inhibitors, e.g. M.24A phosphine such as aluminium phosphide, calcium phosphide, phosphine or zinc phosphide, or M.24B cyanide;
M.25 Mitochondrial complex II electron transport inhibitors, such as beta-ketonitrile derivatives, e.g. cyenopyrafen or cyflumetofen;
M.28 Ryanodine receptor-modulators from the class of diamides, e.g. flubendiamide, chlorantraniliprole, cyantraniliprole, tetraniliprole, M.28.1: (R)-3-Chlor-N1-{2-methyl-4-[1,2,2,2-tetrafluoro-1-(trifluoromethyl)ethyl]phenyl}-N2-(1-methyl-2-methylsulfonylethyl)phthalamid, M.28.2: (S)-3-Chloro-N1-{2-methyl-4-[1,2,2,2-tetrafluoro-1-(trifluoromethyl)ethyl]phenyl}-N2-(1-methyl-2-methyl-sulfonylethyl)phthalamid, M.28.3: cyclaniliprole, or M.28.4: methyl-2-[3,5-dibromo-2-({[3-bromo-1-(3-chlorpyridin-2-yl)-1H-pyrazol-5-yl]carbonyl}amino)benzoyl]-1,2-dimethylhydrazinecarboxylate; or M.28.5a) N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide; M.28.5b) N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide; M.28.5c) N-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide; M.28.5d) N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide; M.28.5h) N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide; M.28.5i) N-[2-(5-Amino-1,3,4-thiadiazol-2-yl)-4-chloro-6-methylphenyl]-3-bromo-1-(3-chloro-2-pyridinyl)-1H-pyrazole-5-carboxamide; M.28.5j) 3-Chloro-1-(3-chloro-2-pyridinyl)-N-[2,4-dichloro-6-[[(1-cyano-1-methylethyl)amino]carbonyl]phenyl]-1 H-pyrazole-5-carboxamide; M.28.5k) 3-Bromo-N-[2,4-dichloro-6-(methylcarbamoyl)phenyl]-1-(3,5-dichloro-2-pyridyl)-1H-pyrazole-5-carboxamide; M.28.5l) N-[4-Chloro-2-[[(1,1-dimethylethyl)amino]carbonyl]-6-methylphenyl]-1-(3-chloro-2-pyridinyl)-3-(fluoro-methoxy)-1 H-pyrazole-5-carboxamide; or
M.28.6: cyhalodiamide; or
M.29: Chordotonal organ Modulators - undefined target site, e.g. flonicamid;
M.UN. insecticidal active compounds of unknown or uncertain mode of action, e.g. afidopyropen, afoxolaner, azadirachtin, amidoflumet, benzoximate, broflanilide, bromopropylate, chinomethionat, cryolite, dicloromezotiaz, dicofol, flufenerim, flometoquin, fluensulfone, fluhexafon, fluopyram, fluralaner, metoxadiazone, piperonyl butoxide, pyflubumide, pyridalyl, tioxazafen, M.UN.3: 11-(4-chloro-2,6-dimethylphenyl)-12-hydroxy-1,4-dioxa-9-azadispiro[4.2.4.2]-tetradec-11-en-10-one,
M.UN.4: 3-(4'-fluoro-2,4-dimethylbiphenyl-3-yl)-4-hydroxy-8-oxa-1-azaspiro[4.5]dec-3-en-2-one,
M.UN.5: 1-[2-fluoro-4-methyl-5-[(2,2,2-trifluoroethyl)sulfinyl]phenyl]-3-(trifluoromethyl)-1H-1,2,4-triazole-5-amine, or actives on basis of *bacillus firmus* (Votivo, I-1582);
M.UN.6: flupyrimin;
M.UN.8: fluazaindolizine; M.UN.9.a): 4-[5-(3,5-dichlorophenyl)-5-(trifluoromethyl)-4H-isoxazol-3-yl]-2-methyl-N-(1-oxothietan-3-yl)benzamide; M.UN.9.b): fluxametamide; M.UN.10: 5-[3-[2,6-dichloro-4-(3,3-dichloroallyloxy)phenoxy]propoxy]-1 H-pyrazole;
M.UN.11.b) 3-(benzoylmethylamino)-N-[2-bromo-4-[1,2,2,3,3,3-hexafluoro-1-(trifluoromethyl)propyl]-6-(trifluoromethyl)phenyl]-2-fluoro-benzamide; M.UN.11.c) 3-(benzoylmethylamino)-2-fluoro-N-[2-iodo-4-[1,2,2,2-tetrafluoro-1-(trifluoromethyl)ethyl]-6-(trifluoromethyl)phenyl]-benzamide; M.UN.11.d) N-[3-[[[2-iodo-4-[1,2,2,2-tetrafluoro-1-(trifluoromethyl)ethyl]-6-(trifluoromethyl)phenyl]amino]carbonyl]phenyl]-N-methyl-benzamide; M.UN.11.e) N-[3-[[[2-bromo-4-[1,2,2,2-tetrafluoro-1-(trifluoromethyl)ethyl]-6-(trifluoromethyl)phenyl]amino]carbonyl]-2-fluorophenyl]-4-fluoro-N-methyl-benzamide; M.UN.11.f) 4-fluoro-N-[2-fluoro-3-[[[2-iodo-4-[1,2,2,2-tetrafluoro-1-(trifluoromethyl)-ethyl]-6-(trifluoromethyl)phenyl]amino]carbonyl]phenyl]-N-methyl-benzamide; M.UN.11.g) 3-fluoro-N-[2-fluoro-3-[[[2-iodo-4-[1,2,2,2-tetrafluoro-1-(trifluoromethyl)ethyl]-6-(trifluoromethyl)phenyl]amino]carbonyl]phenyl]-N-methyl-benzamide; M.UN.11.h) 2-chloro-N-[3-[[[2-iodo-4-[1,2,2,2-tetrafluoro-1-(trifluoromethyl)ethyl]-6-(trifluoromethyl)phenyl]amino]carbonyl]phenyl]- 3-pyridinecarboxamide; M.UN.11.i) 4-cyano-N-[2-cyano-5-[[2,6-dibromo-4-[1,2,2,3,3,3-hexafluoro-1-(trifluoromethyl)-propyl]phenyl]carbamoyl]phenyl]-2-methyl-benzamide; M.UN.11.j) 4-cyano-3-[(4-cyano-2-methylbenzoyl)amino]-N-[2,6-dichloro-4-[1,2,2,3,3,3-hexafluoro-1-(trifluoromethyl)propyl]phenyl]-2-fluoro-benzamide; M.UN.11.k) N-[5-[[2-chloro-6-cyano-4-[1,2,2,3,3,3-hexafluoro-1-(trifluoromethyl)propyl]phenyl]carbamoyl]-2-cyano-phenyl]-4-cyano-2-methyl-benzamide; M.UN.11.1) N-[5-[[2-bromo-6-chloro-4-[2,2,2-trifluoro-1-hydroxy-1-(trifluoromethyl)ethyl]phenyl]carbamoyl]-2-cyano-phenyl]-4-cyano-2-methyl-benzamide; M.UN.11.m) N-[5-[[2-bromo-6-chloro-4-[1,2,2,3,3,3-hexafluoro-1-(trifluoromethyl)propyl]phenyl]carbamoyl]-2-cyano-phenyl]-4-cyano-2-methyl-benzamide; M.UN.11.n) 4-cyano-N-[2-cyano-5-[[2,6-dichloro-4-[1,2,2,3,3,3-hexafluoro-1-(trifluoromethyl)propyl]phenyl]carbamoyl]phenyl]-2-methyl-benzamide; M.UN.11.o) 4-cyano-N-[2-cyano-5-[[2,6-dichloro-4-[1,2,2,2-tetrafluoro-1-(trifluoromethyl)ethyl]phenyl]carbamoyl]phenyl]-2-methyl-benzamide; M.UN.11.p) N-[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide; or
M.UN.12.a) 2-(1,3-Dioxan-2-yl)-6-[2-(3-pyridinyl)-5-thiazolyl]-pyridine; M.UN.12.b) 2-[6-[2-(5-Fluoro-3-pyridinyl)-5-thiazolyl]-2-pyridinyl]-pyrimidine; M.UN.12.c) 2-[6-[2-(3-Pyridinyl)-5-thiazolyl]-2-pyridinyl]-pyrimidine; M.UN.12.d) N-Methylsulfonyl-6-[2-(3-pyridyl)thiazol-5-yl]pyridine-2-carboxamide; M.UN.12.e) N-Methylsulfonyl-6-[2-(3-pyridyl)thiazol-5-yl]pyridine-2-carboxamide; M.UN.12.f) N-Ethyl-N-[4-methyl-2-(3-pyridyl)thiazol-5-yl]-3-methylthio-propanamide; M.UN.12.g) N-Methyl-N-[4-methyl-2-(3-pyridyl)thiazol-5-yl]-3-methylthio-propanamide; M.UN.12.h) N,2-Dimethyl-N-[4-methyl-2-(3-pyridyl)thiazol-5-yl]-3-methylthio-propanamide; M.UN.12.i) N-Ethyl-2-methyl-N-[4-methyl-2-(3-pyridyl)thiazol-5-yl]-3-methylthio-propanamide; M.UN.12.j) N-[4-Chloro-2-(3-pyridyl)thiazol-5-yl]-N-ethyl-2-methyl-3-methylthio-propanamide; M.UN.12.k) N-[4-Chloro-2-(3-pyridyl)thiazol-5-yl]-N,2-dimethyl-3-methylthio-propanamide; M.UN.12.1) N-[4-Chloro-2-(3-pyridyl)thiazol-5-yl]-N-methyl-3-methylthio-propanamide; M.UN.12.m) N-[4-Chloro-2-(3-pyridyl)thiazol-5-yl]-N-ethyl-3-methylthio-propanamide;
M.UN.14a) 1-[(6-Chloro-3-pyridinyl)methyl]-1,2,3,5,6,7-hexahydro-5-methoxy-7-methyl-8-nitro-imidazo[1,2-a]pyridine; or M.UN.14b) 1-[(6-Chloropyridin-3-yl)methyl]-7-methyl-8-nitro-1,2,3,5,6,7-hexahydroimidazo[1,2-a]pyridin-5-ol;
M.UN.16a) 1-isopropyl-N,5-dimethyl-N-pyridazin-4-yl-pyrazole-4-carboxamide; or M.UN.16b) 1-(1,2-dimethylpropyl)-N-ethyl-5-methyl-N-pyridazin-4-yl-pyrazole-4-carboxamide; M.UN.16c) N,5-dimethyl-N-pyridazin-4-yl-1-(2,2,2-trifluoro-1-methyl-ethyl)pyrazole-4-carboxamide; M.UN.16d) 1-[1-(1-cyanocyclopropyl)ethyl]-N-ethyl-5-methyl-N-pyridazin-4-yl-pyrazole-4-carboxamide; M.UN.16e) N-ethyl-1-(2-fluoro-1-methyl-propyl)-5-methyl-N-pyridazin-4-yl-pyrazole-4-carboxamide; M.UN.16f) 1-(1,2-dimethylpropyl)-N,5-dimethyl-N-pyridazin-4-yl-pyrazole-4-carboxamide; M.UN.16g) 1-[1-(1-cyanocyclopropyl)ethyl]-N,5-dimethyl-N-pyridazin-4-yl-pyrazole-4-carboxamide; M.UN.16h) N-methyl-1-(2-fluoro-1-methyl-propyl]-5-methyl-N-pyridazin-4-yl-pyrazole-4-carboxamide; M.UN.16i) 1-(4,4-difluorocyclohexyl)-N-ethyl-5-methyl-N-pyridazin-4-yl-pyrazole-4-carboxamide; or M.UN.16j) 1-(4,4-difluorocyclohexyl)-N,5-dimethyl-N-pyridazin-4-yl-pyrazole-4-carboxamide,
M.UN.17a) N-(1-methylethyl)-2-(3-pyridinyl)-2H-indazole-4-carboxamide; M.UN.17b) N-cyclopropyl-2-(3-pyridinyl)-2H-indazole-4-carboxamide; M.UN.17c) N-cyclohexyl-2-(3-pyridinyl)-2H-indazole-4-carboxamide; M.UN.17d) 2-(3-pyridinyl)-N-(2,2,2-trifluoroethyl)-2H-indazole-4-carboxamide; M.UN.17e) 2-(3-pyridinyl)-N-[(tetrahydro-2-furanyl)methyl]-2H-indazole-5-carboxamide; M.UN.17f) methyl 2-[[2-(3-pyridinyl)-2H-indazol-5-yl]carbonyl]hydrazinecarboxylate; M.UN.17g) N-[(2,2-difluorocyclopropyl)methyl]-2-(3-pyridinyl)-2H-indazole-5-carboxamide; M.UN.17h) N-(2,2-difluoropropyl)-2-(3-pyridinyl)-2H-indazole-5-carboxamide; M.UN.17i) 2-(3-pyridinyl )-N-(2-pyrimidinylmethyl )-2H-indazole-5-carboxamide; M.UN.17j) N-[(5-methyl-2-pyrazinyl)methyl]-2-(3-pyridinyl)-2H-indazole-5-carboxamide,
M.UN.18a) N-[3-chloro-1-(3-pyridyl)pyrazol-4-yl]-N-ethyl-3-(3,3,3-trifluoropropylsulfanyl)propanamide; M.UN.18b) N-[3-chloro-1-(3-pyridyl)pyrazol-4-yl]-N-ethyl-3-(3,3,3-trifluoropropylsulfinyl)propanamide; M.UN.18c) N-[3-chloro-1-(3-pyridyl)pyrazol-4-yl]-3-[(2,2-difluorocyclopropyl)methylsulfanyl]-N-ethyl-propanamide; M.UN.18d) N-[3-chloro-1-(3-pyridyl)pyrazol-4-yl]-3-[(2,2-difluorocyclopropyl)methylsulfinyl]-N-ethyl-propanamide;
M.UN.19 sarolaner, M.UN.20 lotilaner;
M.UN.21 N-[4-Chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; M.UN.22a 2-(3-ethylsulfonyl-2-pyridyl)-3-methyl-6-(trifluoromethyl)imidazo[4,5-b]pyridine, or M.UN.22b 2-[3-ethylsulfonyl-5-(trifluoromethyl)-2-pyridyl]-3-methyl-6-(trifluoromethyl)imidazo[4,5-b]pyridine;
M.UN.23a 4-[5-(3,5-dichlorophenyl)-5-(trifluoromethyl)-4H-isoxazol-3-yl]-N-[(4R)-2-ethyl-3-oxo-isoxazolidin-4-yl]-2-methyl-benzamide, or M.UN.23b 4-[5-(3,5-dichloro-4-fluoro-phenyl)-5-(trifluoromethyl)-4H-isoxazol-3-yl]-N-[(4R)-2-ethyl-3-oxo-isoxazolidin-4-yl]-2-methyl-benzamide;
M.UN.24a) N-[4-chloro-3-(cyclopropylcarbamoyl)phenyl]-2-methyl-5-(1,1,2,2,2-pentafluoroethyl)-4-(trifluoromethyl)pyrazole-3-carboxamide or M.UN.24b) N-[4-chloro-3-[(1-cyanocyclopropyl)carbamoyl]phenyl]-2-methyl-5-(1,1,2,2,2-pentafluoroethyl)-4-(trifluoromethyl)pyrazole-3-carboxamide; M.UN.25 acynonapyr; M.UN.26 benzpyrimoxan; M.UN.27 2-chloro-N-(1-cyanocyclopropyl)-5-[1-[2-methyl-5-(1,1,2,2,2-pentafluoroethyl)-4-(trifluoromethyl)pyrazol-3-yl]pyrazol-4-yl]benzamide.

The commercially available compounds of the group M listed above may be found in The Pesticide Manual, 17th Edition, C. MacBean, British Crop Protection Council (2015) among other publications. The online Pesticide Manual is updated regularly and is accessible through http://bcpcdata.com/pesticide-manual.html.

Another online data base for pesticides providing the ISO common names is http://www.alan-wood.net/pesticides.

The M.4 cycloxaprid is known from WO2010/069266 and WO2011/069456. M.4A.1 is known from CN 103814937; CN105367557, CN 105481839. M.4A.2, guadipyr, is known from WO 2013/003977, and M.4A.3 (approved as paichongding in China) is known from WO 2007/101369. M.22B.1 is described in CN10171577 and M.22B.2 in CN102126994. Spiropidion M.23.1 is known from WO 2014/191271. M.28.1 and M.28.2 are known from WO2007/101540. M.28.3 is described in WO2005/077934. M.28.4 is described in WO2007/043677. M.28.5a) to M.28.5d) and M.28.5h) are described in WO 2007/006670, WO2013/024009 and WO 2013/024010, M.28.5i) is described in WO2011/085575, M.28.5j) in WO2008/134969, M.28.5k) in US2011/046186 and M.28.5l) in WO2012/034403. M.28.6 can be found in WO2012/034472. M.UN.3 is known from WO2006/089633 and M.UN.4 from WO2008/067911. M.UN.5 is described in WO2006/043635, and biological control agents on the basis of *bacillus firmus* are described in WO2009/124707. Flupyrimin is described in WO2012/029672. M.UN.8 is known from WO2013/055584. M.UN.9.a) is described in WO2013/050317. M.UN.9.b) is described in WO2014/126208. M.UN.10 is known from WO2010/060379. Broflanilide and M.UN.11.b) to M.UN.11.h) are described in WO2010/018714, and M.UN.11i) to M.UN.11.p) in WO 2010/127926. M.UN.12.a) to M.UN.12.c) are known from WO2010/006713, M.UN.12.d) and M.UN.12.e) are known from WO2012/000896, and M.UN.12.f) to M.UN.12.m) from WO 2010/129497. M.UN.14a) and M.UN.14b) are known from WO2007/101369. M.UN.16.a) to M.UN.16h) are described in WO2010/034737, WO2012/084670, and WO2012/143317, resp., and M.UN.16i) and M.UN.16j) are described in WO2015/055497. M.UN.17a) to M.UN.17.j) are described in WO2015/038503. M.UN.18a) to M.UN.18d) are described in US2014/0213448. M.UN.19 is described in WO2014/036056. M.UN.20 is known from WO2014/090918. M.UN.21 is known from EP2910126. M.UN.22a and M.UN.22b are known from WO2015/059039 and WO2015/190316. M.UN.23a and M.UN.23b are known from WO2013/050302. M.UN.24a and M.UN.24b are known from WO2012/126766. Acynonapyr M.UN.25 is known from WO 2011/105506. Benzpyrimoxan M.UN.26 is known from WO2016/104516. M.UN.27 is known from WO2016174049.

The following list of fungicides, in conjunction with which the compounds of the present invention can be used, is intended to illustrate the possible combinations but does not limit them:
A) Respiration inhibitors
   - Inhibitors of complex III at Qo site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxystrobin (A.1.17), 2 (2-(3-(2,6-dichlorophenyl)-1-methyl-allylideneaminooxymethyl)-phenyl)-2 methoxyimino-N methyl-acetamide (A.1.18), pyribencarb (A.1.19), triclopyricarb/chlorodincarb (A.1.20), famoxadone (A.1.21), fen-amidone (A.1.21), methyl-N-[2-[(1,4-dimethyl-5 phenyl-pyrazol-3-yl)oxylmethyl]phenyl]-N-methoxy-carbamate (A.1.22), 1-[3-chloro-2 [[1 (4-chlorophenyl)-1H-pyrazol-3-yl]oxymethyl]phenyl]-4-methyltetrazol-5-one (A.1.23), 1-[3-bromo-2-[[1-(4-chlorophenyl)pyrazol-3-yl]oxymethyl]phenyl]-4-methyltetrazol-5-one (A.1.24), 1-[2-[[1-(4-chlorophenyl)pyrazol-3-yl]oxymethyl]-3-methyl-phenyl]-4-methyltetrazol-5-one (A.1.25), 1-[2-[[1-(4-chlorophenyl)pyrazol-3-yl]oxymethyl]-3-fluoro-phenyl]-4-methyltetrazol-5-one (A.1.26), 1-[2-[[1-(2,4-dichlorophenyl)pyrazol-3-yl]oxymethyl]-3-fluoro-phenyl]-4-methyl-tetrazol-5-one (A.1.27), 1-[3-cyclopropyl-2-[[2-methyl-4 (1 methylpyrazol-3-yl)phenoxy]methyl]phenyl]-4 methyl-tetrazol-5-one (A.1.30), 1 [3 (difluoromethoxy)-2-[[2-methyl-4-(1 methylpyrazol-3 yl)phenoxy]methyl]phenyl]-4 methyl-tetrazol-5-one (A.1.31), 1-methyl-4-[3-methyl-2 [[2 methyl-4-(1-methylpyrazol-3 yl)phenoxy]methyl]phenyl]tetrazol-5-one (A.1.32), (Z,2E)-5-[1-(2,4-dichlorophenyl)pyrazol-3-yl]-oxy-2-methoxyimino-N,3-dimethyl-pent-3-enamide (A.1.34), (Z,2E) 5 [1 (4-chlorophenyl)pyrazol-3-yl]oxy-2-methoxyimino-N,3-dimethyl-pent-3-enamide (A.1.35), pyriminostrobin (A.1.36), bifujunzhi (A.1.37), 2-(ortho-((2,5-dimethylphenyl-oxy-methylen)phenyl)-3-methoxy-acrylic acid methylester (A.1.38);
   - inhibitors of complex III at Qi site: cyazofamid (A.2.1), amisulbrom (A.2.2), [(6S,7R,8R) 8 benzyl-3-[(3-hydroxy-4-methoxy-pyridine-2-carbonyl)amino]-6-methyl-4,9-dioxo-1,5-dioxonan-7-yl] 2-methylpropanoate (A.2.3), fenpicoxamid (A.2.4);
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), 3 (difluoromethyl)-1-methyl-N-(1,1,3-trimethylindan-4-yl)pyrazole-4 carboxamide (A.3.22), 3 (trifluoromethyl)-1-methyl-N-(1,1,3-trimethylindan-4-yl)pyrazole-4 carboxamide (A.3.23), 1,3-dimethyl-N-(1,1,3-trimethylindan-4-yl)pyrazole-4-carboxamide (A.3.24), 3-(trifluoromethyl)-1,5 dimethyl-N-(1,1,3-trimethylindan-4-yl)pyrazole-4-carboxamide (A.3.25), 1,3,5-trimethyl-N-(1,1,3-trimethylindan-4-yl)pyrazole-4-carboxamide (A.3.26), 3-(difluoromethyl)-1,5 dimethyl-N-(1,1,3-trimethylindan-4-yl)pyrazole-4-carboxamide (A.3.27), 3-(difluoromethyl)-N (7 fluoro-1,1,3-trimethyl-indan-4-yl)-1-methyl-pyrazole-4-carboxamide (A.3.28), N-[(5-chloro-2-isopropyl-phenyl)methyl]-N-cyclopropyl-5-fluoro-1,3-dimethyl-pyrazole-4-carboxamide (A.3.29), methyl (E)-2-[2-[(5-cyano-2-methyl-phenoxy)methyl]phenyl]-3-methoxy-prop-2 enoate (A.3.30), N-[(5-chloro-2-isopropyl-phenyl)methyl]-N-cyclopropyl-3-(difluoromethyl)-5 fluoro-1-methyl-pyrazole-4-carboxamide (A.3.31), 2-(difluoromethyl)-N-(1,1,3-trimethyl-indan-4-yl)pyridine-3-carboxamide (A.3.32), 2-(difluoromethyl)-N-[(3R)-1,1,3-trimethylindan-4-yl]pyridine-3-carboxamide (A.3.33), 2-(difluoromethyl)-N-(3-ethyl-1,1-dimethyl-indan-4-yl)pyridine-3-carboxamide (A.3.34), 2-(difluoromethyl)-N-[(3R)-3-ethyl-1,1-dimethyl-indan-4-yl]pyridine-3-carboxamide (A.3.35), 2-(difluoromethyl)-N-(1,1-dimethyl-3-propyl-indan-4-yl)pyridine-3-carboxamide (A.3.36), 2-(difluoromethyl)-N-[(3R)-1,1-dimethyl-3-propyl-indan-4-yl]pyridine-3-carboxamide (A.3.37), 2-(difluoromethyl)-N-(3-isobutyl-1,1-dimethyl-indan-4-yl)pyridine-3-carboxamide (A.3.38), 2-(difluoromethyl)-N-[(3R)-3-isobutyl-1,1-dimethyl-indan-4 yl]pyridine-3-carboxamide (A.3.39);
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromu-conazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), ipfentrifluconazole, (B.1.37), mefentrifluconazole (B.1.38), 2-(chloromethyl)-2-methyl-5-(p-tolylmethyl)-1 (1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines and piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4 amine (C.2.6), 5-fluoro-2-(4-fluorophenylmethoxy)pyrimidin-4 amine (C.2.7), 5-fluoro-2 (4 chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), 3-chloro-4-(2,6-difluorophenyl)-6-methyl-5-phenyl-pyridazine (D.1.6), 3-chloro-6-methyl-5-phenyl-4-(2,4,6-trifluorophenyl)pyridazine (D.1.7), N ethyl-2-[(3-ethynyl-8-methyl-6-quinolyl)oxy]butanamide (D.1.8), N-ethyl-2-[(3-ethynyl-8 methyl-6 quinolyl)oxy]-2-methylsulfanyl-acetamide (D.1.9), 2-[(3-ethynyl-8-methyl-6-quinol-yl)oxy]-N (2-fluoroethyl)butanamide (D.1.10), 2-[(3-ethynyl-8-methyl-6-quinolyl)oxy]-N-(2-flu-oroethyl)-2-methoxy-acetamide (D.1.11), 2-[(3-ethynyl-8-methyl-6-quinolyl)oxy]-N-propyl-butanamide (D.1.12), 2-[(3-ethynyl-8-methyl-6-quinolyl)oxy]-2-methoxy-N-propyl-acetamide (D.1.13), 2-[(3-ethynyl-8-methyl-6-quinolyl)oxy]-2-methylsulfanyl-N-propyl-acetamide (D.1.14), 2 [(3 ethynyl-8-methyl-6-quinolyl)oxy]-N-(2-fluoroethyl)-2-methylsulfanyl-acetamide (D.1.15), 4-(2-bromo-4-fluoro-phenyl)-N-(2-chloro-6-fluoro-phenyl)-2,5-dimethyl-pyrazol-3 amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7);
E) Inhibitors of amino acid and protein synthesis
   - methionine synthesis inhibitors: cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), 2-{3-[2-(1-{[3,5-bis(difluoromethyl-1H-pyrazol-1-yl]acetyl}piperidin-4-yl)-1,3-thiazol-4-yl]-4,5-dihydro-1,2 oxazol-5-yl}phenyl methanesulfonate (G.5.2), 2-{3-[2-(1-{[3,5-bis(difluoromethyl)-1H-pyrazol-1-yl]acetyl}piperidin-4-yl) 1,3-thiazol-4-yl]-4,5-dihydro-1,2-oxazol-5 yl}-3-chlorophenyl methanesulfonate (G.5.3), 4-[1-[2-[3-(difluoromethyl)-5-methyl-pyrazol-1-yl]acetyl]-4-piperidyl]-N-tetralin-1-yl-pyridine-2-carboxamide (G.5.4), 4-[1-[2-[3,5-bis(difluoromethyl)pyrazol-1-yl]acetyl]-4-piperidyl]-N-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-N-tetralin-1-yl-pyridine-2-carboxamide (G.5.6), 4-[1-[2-[5-cyclopropyl-3-(difluoromethyl)pyrazol-1-yl]acetyl]-4-piperidyl]-N-tetralin-1-yl-pyridine-2-carboxamide (G.5.7), 4-[1-[2-[5-methyl-3-(trifluoromethyl)pyrazol-1-yl]acetyl]-4-piperidyl]-N-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-N-tetralin-1-yl-pyridine-2-carboxamide (G.5.9), 4 [1 [2-[3,5-bis(trifluoromethyl)pyrazol-1-yl]acetyl]-4-piperidyl]-N-tetralin-1-yl-pyridine-2-carboxamide (G.5.10), (4-[1-[2-[5-cyclopropyl-3-(trifluoromethyl)pyrazol-1-yl]acetyl]-4-piperidyl]-N-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1 H,SH-[1,4]dithiino[2,3-c:5,6-c']dipyrrole-1,3,5,7(2H,6H)-tetraone (H.4.10);
I) Cell wall synthesis inhibitors
   - inhibitors of glucan synthesis: validamycin (1.1.1), polyoxin B (1.1.2);
   - melanin synthesis inhibitors: pyroquilon (1.2.1), tricyclazole (1.2.2), carpropamid (1.2.3), dicyclomet (1.2.4), fenoxanil (1.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), calcium phosphonate (J.1.11), potassium phosphonate (J.1.12), potassium or sodium bicarbonate (J.1.9), 4 cyclopropyl-N-(2,4-dimethoxyphenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flusulfamide (K.1.15), flutianil (K.1.16), harpin (K.1.17), metha-sulfocarb (K.1.18), nitrapyrin (K.1.19), nitrothal-isopropyl (K.1.20), tolprocarb (K.1.21), oxin-copper (K.1.22), proquinazid (K.1.23), tebufloquin (K.1.24), tecloftalam (K.1.25), triazoxide (K.1.26), N'-(4-(4-chloro-3-trifluoromethyl-phenoxy)-2,5-dimethyl-phenyl)-N-ethyl-N methyl formamidine (K.1.27), N' (4-(4-fluoro-3-trifluoromethyl-phenoxy)-2,5-dimethyl-phenyl)-N-ethyl-N-methyl formamidine (K.1.28), N'-[4-[[3-[(4-chlorophenyl)methyl]-1,2,4-thiadiazol-5-yl]oxy]-2,5-dimethyl-phenyl]-N-ethyl-N-methyl-formamidine (K.1.29), N'-(5-bromo-6-indan-2-yloxy-2-methyl-3-pyridyl)-N-ethyl-N-methyl-formamidine (K.1.30), N'-[5-bromo-6-[1-(3,5-difluorophenyl)ethoxy]-2-methyl-3-pyridyl]-N-ethyl-N-methyl-formamidine (K.1.31), N'-[5-bromo-6-(4-isopropylcyclohexoxy)-2-methyl-3-pyridyl]-N-ethyl-N-methyl-formamidine (K.1.32), N' [5 bromo-2-methyl-6-(1-phenylethoxy)-3-pyridyl]-N-ethyl-N-methyl-formamidine (K.1.33), N'-(2-methyl-5-trifluoromethyl-4-(3-trimethylsilanyl-propoxy)-phenyl)-N-ethyl-N-methyl formamidine (K.1.34), N'-(5-difluoromethyl-2 methyl-4-(3-trimethylsilanyl-propoxy)-phenyl)-N-ethyl-N-methyl formamidine (K.1.35), 2-(4-chloro-phenyl)-N-[4-(3,4-dimethoxy-phenyl)-isoxazol-5 yl]-2-prop-2-ynyloxy-acetamide (K.1.36), 3 [5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3 yl]-pyridine (K.1.38), 5-chloro-1 (4,6-dimethoxy-pyrimidin-2-yl)-2-methyl-1 H-benzoimidazole (K.1.39), ethyl (Z) 3 amino-2-cyano-3-phenyl-prop-2-enoate (K.1.40), picarbutrazox (K.1.41), pentyl N-[6-[[(Z)-[(1-methyltetrazol-5-yl)-phenyl-methylene]amino]oxymethyl]-2-pyridyl]carbamate (K.1.42), but-3-ynyl N-[6-[[(Z)-[(1-methyltetrazol-5-yl)-phenyl-methylene]amino]oxymethyl]-2-pyridyl]carbamate (K.1.43), 2-[2-[(7,8-difluoro-2-methyl-3-quinolyl)oxy]-6-fluoro-phenyl]propan-2-ol (K.1.44), 2-[2-fluoro-6-[(8-fluoro-2-methyl-3-quinolyl)oxy]phen-yl]propan-2-ol (K.1.45), quinofumelin (K.1.47), 9-fluoro-2,2-dimethyl-5-(3-quinolyl)-3H 1,4 benzoxazepine (K.1.49), 2-(6-benzyl-2-pyridyl)quinazoline (K.1.50), 2-[6-(3-fluoro-4 methoxy-phenyl)-5-methyl-2-pyridyl]quinazoline (K.1.51), dichlobentiazox (K.1.52), N'-(2,5-dimethyl-4-phenoxy-phenyl)-N-ethyl-N-methyl-formamidine (K.1.53).

The fungicides described by common names, their preparation and their activity e.g. against harmful fungi is known (cf.: http://www.alanwood.net/pesticides/); these substances are commercially available.

The fungicides described by IUPAC nomenclature, their preparation and their pesticidal activity is also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO2012/168188, WO 2007/006670, WO 2011/77514; WO13/047749, WO 10/069882, WO 13/047441, WO 03/16303, WO 09/90181, WO 13/007767, WO 13/010862, WO 13/127704, WO 13/024009, WO 13/024010 and WO 13/047441, WO 13/162072, WO 13/092224, WO 11/135833), CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441).

### Biopesticides

Suitable mixing partners for the compounds of the present invention also include biopesticides.

Biopesticides have been defined as a form of pesticides based on micro-organisms (bacteria, fungi, viruses, nematodes, etc.) or natural products (compounds, such as metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular.
(2) Biochemical pesticides are naturally occurring substances or structurally-similar and functionally identical to a naturally-occurring substance and extracts from biological sources that control pests or provide other crop protection uses as defined below, but have non-toxic mode of actions (such as growth or developmental regulation, attractants, repellents or defense activators (e.g. induced resistance) and are relatively non-toxic to mammals.

Biopesticides for use against crop diseases have already established themselves on a variety of crops. For example, biopesticides already play an important role in controlling downy mildew diseases. Their benefits include: a 0-Day Pre-Harvest Interval, the ability to use under moderate to severe disease pressure, and the ability to use in mixture or in a rotational program with other registered pesticides.

A major growth area for biopesticides is in the area of seed treatments and soil amendments. Biopesticidal seed treatments are e.g. used to control soil borne fungal pathogens that cause seed rots, damping-off, root rot and seedling blights. They can also be used to control internal seed borne fungal pathogens as well as fungal pathogens that are on the surface of the seed. Many biopesticidal products also show capacities to stimulate plant host defenses and other physiological processes that can make treated crops more resistant to a variety of biotic and abiotic stresses or can regulate plant growth. Many biopesticidal products also show capacities to stimulate plant health, plant growth and/or yield enhancing activity.

The following list of biopesticides, in conjunction with which the compounds of the present invention can be used, is intended to illustrate the possible combinations but does not limit them:
L) Biopesticides
   L1) Microbial pesticides with fungicidal, bactericidal, viricidal and/or plant defense activator activity: *Ampelomyces quisqualis, Aspergillus flavus, Aureobasidium pullulans, Bacillus altitudinis, B. amyloliguefaciens, B. megaterium, B. mojavensis, B. mycoides, B. pumllus, B. simplex, B. solisalsi, B. subtilis, B. subtilis* var. *amyloliquefaciens, Candida oleophila, C. saitoana, Clavibacter michiganensis* (bacteriophages), *Coniothyrium minitans, Cryphonectria parasitica, Cryptococcus albidus, Dilophosphora alopecuri, Fusarium oxysporum, Clonostachys rosea* f. *catenulate* (also named *Gliocladium catenulatum), Gliocladium roseum, Lysobacterantibioticus, L. enzymogenes, Metschnikowia fructicola, Microdochium dimerum, Microsphaeropsis ochracea, Muscodor albus, Paenibacil lus alvei, Paenibacillus polymyxa, Pantoea vagans, Penicillium bilaiae, Phlebiopsis gigantea, Pseudomonas* sp., *Pseudomons chloraphis*, *Pseudozyma flocculosa*, *Pischia anomala*, *Pythium oligandrum, Sphaerodes mycoparasitica, Streptomyces griseoviridis, S. lydicus, S. violaceusniger, Talaromyces flavus, Trichoderma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia,* zucchini yellow mosaic virus (avirulent strain);
   L2) Biochemical pesticides with fungicidal, bactericidal, viricidal and/or plant defense activator activity: harpin protein, *Reynoutria sachalinensis* extract;
   L3) Microbial pesticides with insecticidal, acaricidal, molluscidal and/or nematicidal activity: *Agrobacterium radiobacter, Bacillus cereus, B. firmus, B. thuringiensis, B. thuringiensis* ssp. *aizawai, B. t.* ssp. *israelensis, B. t.* ssp. *galleriae, B. t.* ssp. *kurstaki, B. t.* ssp. *tenebrionis, Beauveria bassiana, B. brongniartii, Burkholderia* spp., *Chromobacterium subtsugae, Cydia pomonella* granulovirus (CpGV), *Cryptophlebia leucotreta* granulovirus (CrleGV), *Flavobacterium* spp., *Helicoverpa armigera* nucleopolyhedrovirus (HearNPV), *Helicoverpa zea nucleopolyhedrovirus* (HzNPV), *Helicoverpa zea* single capsid nucleopolyhedrovirus (HzSNPV), *Heterorhabditis bacteriophora, Isaria fumosorosea, Lecanicillium longisporum, L. muscarium, Metarhizium anisopliae, Metarhizium anisopliae* var. *anisopliae, M. anisopliae* var. *acridum, Nomuraea rileyi, Paecilomyces fumosoroseus, P. lilacinus, Paenibacillus popilliae, Pasteuria* spp., *P. nishizawae, P. penetrans, P. ramosa, P. thornea, P. usgae, Pseudomonas fluorescens, Spodoptera littoralis* nucleopolyhedrovirus (SpliNPV), *Steinernema carpocapsae, S. feltiae, S. kraussei, Streptomyces galbus, S. microflavus*;
   L4) Biochemical pesticides with insecticidal, acaricidal, molluscidal, pheromone and/or nematicidal activity: L-carvone, citral, (E,Z)-7,9-dodecadien-1-yl acetate, ethyl formate, (E,Z)-2,4-ethyl decadienoate (pear ester), (Z,Z,E)-7,11,13-hexadecatrienal, heptyl butyrate, isopropyl myristate, lavanulyl senecioate, cis-jasmone, 2-methyl 1-butanol, methyl eugenol, methyl jasmonate, (E,Z)-2,13-octadecadien-1-ol, (E,Z)-2,13-octadecadien-1-ol acetate, (E,Z)-3,13-octadecadien-1-ol, R-1-octen-3-ol, pentatermanone, (E,Z,Z)-3,8,11-tetradecatrienyl acetate, (Z,E)-9,12-tetradecadien-1-yl acetate, Z-7-tetradecen-2-one, Z-9-tetradecen-1-yl acetate, Z-11-tetradecenal, Z-11-tetradecen-1-ol, extract of *Chenopodium ambrosiodes,* Neem oil, Quillay extract;
   L5) Microbial pesticides with plant stress reducing, plant growth regulator, plant growth promoting and/or yield enhancing activity: *Azospirillum amazonense, A. brasilense, A. lipoferum, A. irakense, A. halopraeferens, Bradyrhizobium* spp., *B. elkanii, B. japonicum, B. liaoningense, B. lupini, Delftia acidovorans, Glomus intraradices, Mesorhizobium* spp., *Rhizobium leguminosarum* bv. *phaseoli, R.* /. bv. *trifolii, R.* /. bv. *viciae, R. tropici, Sinorhizobium meliloti.*

The biopesticides from group L1) and/or L2) may also have insecticidal, acaricidal, molluscidal, pheromone, nematocidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematocidal activity.

Many of these biopesticides have been deposited under deposition numbers mentioned herein (the prefixes such as ATCC or DSM refer to the acronym of the respective culture collection, for details see e. g. here: http://www. wfcc.info/ccinfo/collection/by_acronym/), are referred to in literature, registered and/or are commercially available: mixtures of *Aureobasidium pullulans* DSM 14940 and DSM 14941 isolated in 1989 in Konstanz, Germany (e. g. blastospores in BlossomProtect® from bio-ferm GmbH, Austria), *Azospirillum brasilense* Sp245 originally isolated in wheat reagion of South Brazil (Passo Fundo) at least prior to 1980 (BR 11005; e. g. GELFIX® Gramíneas from BASF Agricultural Specialties Ltd., Brazil), *A. brasilense* strains Ab-V5 and Ab-V6 (e. g. in AzoMax from Novozymes BioAg Produtos papra Agricultura Ltda., Quattro Barras, Brazil or Simbiose-Maiz® from Simbiose-Agro, Brazil; Plant Soil 331, 413-425, 2010), *Bacillus amyloliquefaciens* strain AP-188 (NRRL B-50615 and B-50331; US 8,445,255); *B. amyloliguefaciens* spp. *plantarum* D747 isolated from air in Kikugawa-shi, Japan (US 20130236522 A1; FERM BP-8234; e. g. Double Nickel™ 55 WDG from Certis LLC, USA), *B. amyloliguefaciens* spp*. plantarum* FZB24 isolated from soil in Brandenburg, Germany (also called SB3615; DSM 96-2; J. Plant Dis. Prot. 105, 181-197, 1998; e. g. Taegro® from Novozyme Biologicals, Inc., USA), *B. amyloliquefaciens* ssp. *plantarum* FZB42 isolated from soil in Brandenburg, Germany (DSM 23117; J. Plant Dis. Prot. 105, 181-197, 1998; e. g. RhizoVital® 42 from AbiTEP GmbH, Germany), *B. amyloliquefaciens* ssp. *plantarum* MBl600 isolated from faba bean in Sutton Bonington, Nottinghamshire, U.K. at least before 1988 (also called 1430; NRRL B-50595; US 2012/0149571 A1; e. g. Integral®from BASF Corp., USA), *B. amyloliguefaciens* spp*. plantarum* QST-713 isolated from peach orchard in 1995 in California, U.S.A. (NRRL B-21661; e. g. Serenade® MAX from Bayer Crop Science LP, USA), *B. amyloliquefaciens* spp. *plantarum* TJ 1000 isolated in 1992 in South Dakoda, U.S.A. (also called 1BE; ATCC BAA-390; CA 2471555 A1; e. g. QuickRoots™ from TJ Technologies, Watertown, SD, USA), *B. firmus* CNCM 1-1582, a variant of parental strain EIP-N1 (CNCM 1-1556) isolated from soil of central plain area of Israel (WO 2009/126473, US 6,406,690; e. g. Votivo® from Bayer CropScience LP, USA), *B. pumilus* GHA 180 isolated from apple tree rhizosphere in Mexico (IDAC 260707-01; e. g. PRO-MIX® BX from Premier Horticulture, Quebec, Canada), *B. pumilus* INR-7 otherwise referred to as BU-F22 and BU-F33 isolated at least before 1993 from cucumber infested by *Erwinia tracheiphila* (NRRL B-50185, NRRL B-50153; US 8,445,255), *B. pumilus* KFP9F isolated from the rhizosphere of grasses in South Africa at least before 2008 (NRRL B-50754; WO 2014/029697; e. g. BAC-UP or FUSION-P from BASF Agricultural Specialities (Pty) Ltd., South Africa), *B. pumilus* QST 2808 was isolated from soil collected in Pohnpei, Federated States of Micronesia, in 1998 (NRRL B-30087; e. g. Sonata® or Ballad® Plus from Bayer Crop Science LP, USA), *B. simplex* ABU 288 (NRRL B-50304; US 8,445,255), *B. subtilis* FB17 also called UD 1022 or UD10-22 isolated from red beet roots in North America (ATCC PTA-11857; System. Appl. Microbiol. 27, 372-379, 2004; US 2010/0260735; WO 2011/109395); *B. thuringiensisssp. aizawai* ABTS-1857 isolated from soil taken from a lawn in Ephraim, Wisconsin, U.S.A., in 1987 (also called ABG-6346; ATCC SD-1372; e. g. XenTari® from BioFa AG, Münsingen, Germany), *B. t.* ssp. *kurstaki* ABTS-351 identical to HD-1 isolated in 1967 from diseased Pink Bollworm black larvae in Brownsville, Texas, U.S.A. (ATCC SD-1275; e. g. Dipel® DF from Valent BioSciences, IL, USA), *B. t.* ssp. *kurstaki* SB4 isolated from *E. saccharina* Iarval cadavers (NRRL B-50753; e. g. Beta Pro® from BASF Agricultural Specialities (Pty) Ltd., South Africa), *B. t.* ssp. *tenebrionis* NB-176-1, a mutant of strain NB-125, a wild type strain isolated in 1982 from a dead pupa of the beetle Tenebrio molitor (DSM 5480; EP 585 215 B1; e. g. Novodor® from Valent BioSciences, Switzerland), *Beauveria bassiana* GHA (ATCC 74250; e. g. BotaniGard® 22WGP from Laverlam Int. Corp., USA), *B. bassiana* JW-1 (ATCC 74040; e. g. Naturalis® from CBC (Europe) S.r.l., Italy), *B. bassiana* PPRI 5339 isolated from the larva of the tortoise beetle *Conchyloctenia punctata* (NRRL 50757; e. g. BroadBand® from BASF Agricultural Specialities (Pty) Ltd., South Africa), *Bradyrhizobium elkaniistrains* SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e. g. GELFIX 5 from BASF Agricultural Specialties Ltd., Brazil), *B. japonicum* 532c isolated from Wisconsin field in U.S.A. (Nitragin 61A152; Can. J. Plant. Sci. 70, 661-666, 1990; e. g. in Rhizoflo®, Histick®, Hicoat® Super from BASF Agricultural Specialties Ltd., Canada), *B. japonicum* E-109 variant of strain USDA 138 (INTA E109, SEMIA 5085; Eur. J. Soil Biol. 45, 28-35, 2009; Biol. Fertil. Soils 47, 81-89, 2011); B. japonicum strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e. g. GELFIX 5 or ADHERE 60 from BASF Agricultural Specialties Ltd., Brazil), *B. japonicum* SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e. g. GELFIX 5 or ADHERE 60 from BASF Agricultural Specialties Ltd., Brazil); *Burkholderia sp.* A396 isolated from soil in Nikko, Japan, in 2008 (NRRL B-50319; WO 2013/032693; Marrone Bio Innovations, Inc., USA), *Coniothyrium minitans* CON/M/91-08 isolated from oilseed rape (WO 1996/021358; DSM 9660; e. g. Contans® WG, Intercept® WG from Bayer CropScience AG, Germany), harpin (alpha-beta) protein (Science 257, 85-88, 1992; e. g. Messenger™ or HARP-N-Tek from Plant Health Care plc, U.K.), *Helicoverpa armigera* nucleopolyhedrovirus (HearNPV) (J. Invertebrate Pathol. 107, 112-126, 2011; e. g. Helicovex®from Ader-matt Biocontrol, Switzerland; Diplomata® from Koppert, Brazil; Vivus® Max from AgBiTech Pty Ltd., Queensland, Australia), *Helicoverpa zea* single capsid nucleopolyhedrovirus (HzSNPV) (e. g. Gemstar® from Certis LLC, USA), *Helicoverpa zea* nucleopolyhedrovirus ABA-NPV-U (e. g. Heligen® from AgBiTech Pty Ltd., Queensland, Australia), *Heterorhabditis bacteriophora* (e. g. Nemasys® G from BASF Agricultural Specialities Limited, UK), *Isaria fumosorosea* Apopka-97 isolated from mealy bug on gynura in Apopka, Florida, U.S.A. (ATCC 20874; Biocontrol Science Technol. 22(7), 747-761, 2012; e. g. PFR-97™ or PreFeRal® from Certis LLC, USA), *Metarhizium anisopliae var. anisopliae* F52 also called 275 or V275 isolated from codling moth in Austria (DSM 3884, ATCC 90448; e. g. Met52® Novozymes Biologicals BioAg Group, Canada), *Metschnikowia fructicola* 277 isolated from grapes in the central part of Israel (US 6,994,849; NRRL Y-30752; e. g. formerly Shemer® from Agrogreen, Israel), *Paecilomyces ilacinus* 251 isolated from infected nematode eggs in the Philippines (AGAL 89/030550; WO1991/02051; Crop Protection 27, 352-361, 2008; e. g. BioAct®from Bayer CropScience AG, Germany and MeloCon® from Certis, USA), *Paenibacillus alvei* NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), *Pasteuria nishizawae* Pn1 isolated from a soybean field in the mid-2000s in Illinois, U.S.A. (ATCC SD-5833; Federal Register 76(22), 5808, February 2, 2011; e.g. Clariva™ PN from Syngenta Crop Protection, LLC, USA), *Penicillium bilaiae* (also called *P. bilaiₗ)* strains ATCC 18309 (= ATCC 74319), ATCC 20851 and/or ATCC 22348 (= ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e. g. Jump Start®, Provide® from Novozymes Biologicals BioAg Group, Canada), *Reynoutria sachalinensis* extract (EP 0307510 B1; e. g. Regalia® SC from Marrone Biolnnovations, Davis, CA, USA or Milsana® from BioFa AG, Germany), *Steinernema carpocapsae* (e. g. Millenium® from BASF Agricultural Specialities Limited, UK), *S. feltiae* (e. g. Nemashield® from BioWorks, Inc., USA; Nemasys® from BASF Agricultural Specialities Limited, UK), *Streptomyces microflavus* NRRL B-50550 (WO 2014/124369; Bayer CropScience, Germany), *Trichoderma asperelloides* JM41R isolated in South Africa (NRRL 50759; also referred to as *T. fertile;* e. g. Trichoplus® from BASF Agricultural Specialities (Pty) Ltd., South Africa), *T. harzianum* T-22 also called KRL-AG2 (ATCC 20847; BioControl 57, 687-696, 2012; e. g. Plantshield® from BioWorks Inc., USA or SabrEx™ from Advanced Biological Marketing Inc., Van Wert, OH, USA).

According to the invention, the solid material (dry matter) of the biopesticides (with the exception of oils such as Neem oil) are considered as active components (e.g. to be obtained after drying or evaporation of the extraction or suspension medium in case of liquid formulations of the microbial pesticides).

In accordance with the present invention, the weight ratios and percentages used herein for a biological extract such as Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calculate the total weight of the respective active component with the following equation that 1 x 10¹⁰ CFU equals one gram of total weight of the respective active component. Colony forming unit is measure of viable microbial cells, in particular fungal and bacterial cells. In addition, here "CFU" may also be understood as the number of (juvenile) individual nematodes in case of (entomopathogenic) nematode biopesticides, such as *Steinernema feltiae.*

When mixtures comprising microbial pesticides are employed in crop protection, the application rates preferably range from about 1 x 10⁶ to 5 x 10¹⁵ (or more) CFU/ha, preferably from about 1 x 10⁸ to about 1 x 10¹³ CFU/ha, and even more preferably from about 1 x 10⁹ to about 1 x 10¹² CFU/ha. In the case of (entomopathogenic) nematodes as microbial pesticides (e. g. Steinernema feltiae), the application rates preferably range inform about 1 x 10⁵ to 1 x 10¹² (or more), more preferably from 1 x 10⁸ to 1 x 10¹¹, even more preferably from 5 x 10⁸ to 1 x 10¹⁰ individuals (e. g. in the form of eggs, juvenile or any other live stages, preferably in an infective juvenile stage) per ha.

When mixtures comprising microbial pesticides are employed in seed treatment, the application rates with respect to plant propagation material preferably range from about 1 x 10⁶ to 1 x 10¹² (or more) CFU/seed. Preferably, the concentration is about 1 x 10⁶ to about 1 x 10⁹ CFU/seed. In the case of the microbial pesticides II, the application rates with respect to plant propagation material also preferably range from about 1 x 10⁷ to 1 x 10¹⁴ (or more) CFU per 100 kg of seed, preferably from 1 x 10⁹ to about 1 x 10¹² CFU per 100 kg of seed.

The invention also relates to agrochemical compositions comprising an auxiliary and at least one compound of the present invention or a mixture thereof.

An agrochemical composition comprises a pesticidally effective amount of a compound of the present invention or a mixture thereof. The term "pesticidally effective amount" is defined below.

The compounds of the present invention or the mixtures thereof can be converted into customary types of agro-chemical compositions, e. g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Mono-graph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Examples for suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, al-kylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharide powders, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sul-fates, phosphates, carboxylates, and mixtures thereof. Examples of sulfonates are alkylaryl-sulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Exam-pies of carboxylates are alkyl carboxylates, and carboxylated alcohol or alkylphenol eth-oxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Exam-ples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are homo- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or poly-acid comb polymers. Examples of polybases are polyvinylamines or polyethyleneamines.

Suitable adjuvants are compounds, which have a neglectable or even no pesticidal activity themselves, and which improve the biological performance of the compounds of the present invention on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate-rate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I according to the invention and 5-15 wt% wetting agent (e.g. alcohol alkoxylates) are dissolved in water and/or in a water-soluble solvent (e.g. alcohols) up to 100 wt%. The active substance dissolves upon dilution with water.
ii) Dispersible concentrates (DC)
   5-25 wt% of a compound I according to the invention and 1-10 wt% dispersant (e. g. polyvinylpyrrolidone) are dissolved in up to 100 wt% organic solvent (e.g. cyclohexanone). Dilution with water gives a dispersion.
iii) Emulsifiable concentrates (EC)
   15-70 wt% of a compound I according to the invention and 5-10 wt% emulsifiers (e.g. calcium dodecylbenzenesulfonate and castor oil ethoxylate) are dissolved in up to 100 wt% water-insoluble organic solvent (e.g. aromatic hydrocarbon). Dilution with water gives an emulsion.
iv) Emulsions (EW, EO, ES)
   5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into up to 100 wt% water by means of an emulsifying machine and made into a homogeneous emulsion. Dilution with water gives an emulsion.
v) Suspensions (SC, OD, FS)
   In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and up to 100 wt% water to give a fine active substance suspension. Dilution with water gives a stable suspension of the active sub-stance. For FS type composition up to 40 wt% binder (e.g. polyvinylalcohol) is added.
vi) Water-dispersible granules and water-soluble granules (WG, SG)
   50-80 wt% of a compound I according to the invention are ground finely with addition of up to 100 wt% dispersants and wetting agents (e.g. sodium lignosulfonate and alcohol ethoxylate) and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound I according to the invention are ground in a rotor-stator mill with ad-dition of 1-5 wt% dispersants (e.g. sodium lignosulfonate), 1-3 wt% wetting agents (e.g. alcohol ethoxylate) and up to 100 wt% solid carrier, e.g. silica gel. Dilution with water gives a stable dis-persion or solution of the active substance.
viii) Gel (GW, GF)
   In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and up to 100 wt% water to give a fine suspension of the active sub-stance. Dilution with water gives a stable suspension of the active substance.
ix) Microemulsion (ME)
   5-20 wt% of a compound I according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water up to 100 %. This mixture is stirred for 1 h to produce spontaneously a thermodynamically stable microemulsion.
x) Microcapsules (CS)
   An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. diphenylme-thene-4,4'-diisocyanatae) are dispersed into an aqueous solution of a protective colloid (e.g. polyvinyl alcohol). The addition of a polyamine (e.g. hexamethylenediamine) results in the for-mation of a polyurea microcapsule. The monomers amount to 1-10 wt%. The wt% relate to the total CS composition.
xi) Dustable powders (DP, DS)
   1-10 wt% of a compound I according to the invention are ground finely and mixed intimately with up to 100 wt% solid carrier, e.g. finely divided kaolin.
xii) Granules (GR, FG)
   0.5-30 wt% of a compound I according to the invention is ground finely and associated with up to 100 wt% solid carrier (e.g. silicate). Granulation is achieved by extrusion, spray-drying or the fluidized bed.
xiii) Ultra-low volume liquids (UL)
   1-50 wt% of a compound I according to the invention are dissolved in up to 100 wt% organic solvent, e.g. aromatic hydrocarbon.

The compositions types i) to xi) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably be-tween 0.1 and 90%, and most preferably between 0.5 and 75%, by weight of active sub-stance. The active substances are employed in a purity of from 90% to 100%, preferably from 95% to 100% (according to NMR spectrum).

Various types of oils, wetters, adjuvants, fertilizer, or micronutrients, and other pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage de-vice, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the in-vention such as parts of a kit or parts of a binary or ternary mixture may be mixed by the user himself in a spray tank and further auxiliaries may be added, if appropriate.

In a further embodiment, either individual components of the composition according to the in-vention or partially premixed components, e. g. components comprising compounds of the present invention and/or mixing partners as defined above, may be mixed by the user in a spray tank and further auxiliaries and additives may be added, if appropriate.

In a further embodiment, either individual components of the composition according to the in-vention or partially premixed components, e. g. components comprising compounds of the present invention and/or mixing partners as defined above, can be applied jointly (e.g. after tank mix) or consecutively.

The compounds of the present invention are suitable for use in protecting crops, plants, plant propagation materials, such as seeds, or soil or water, in which the plants are growing, from attack or infestation by animal pests. Therefore, the present invention also relates to a plant protection method, which comprises contacting crops, plants, plant propagation materials, such as seeds, or soil or water, in which the plants are growing, to be protected from attack or infestation by animal pests, with a pesticidally effective amount of a compound of the present invention.

The compounds of the present invention are also suitable for use in combating or controlling animal pests. Therefore, the present invention also relates to a method of combating or controlling animal pests, which comprises contacting the animal pests, their habitat, breeding ground, or food supply, or the crops, plants, plant propagation materials, such as seeds, or soil, or the area, material or environment in which the animal pests are growing or may grow, with a pesticidally effective amount of a compound of the present invention.

The compounds of the present invention are effective through both contact and ingestion. Furthermore, the compounds of the present invention can be applied to any and all developmental stages, such as egg, larva, pupa, and adult.

The compounds of the present invention can be applied as such or in form of compositions comprising them as defined above. Furthermore, the compounds of the present invention can be applied together with a mixing partner as defined above or in form of compositions comprising said mixtures as defined above. The components of said mixture can be applied simultaneously, jointly or separately, or in succession, that is immediately one after another and thereby creating the mixture "in situ" on the desired location, e.g. the plant, the sequence, in the case of separate application, generally not having any effect on the result of the control measures.

The application can be carried out both before and after the infestation of the crops, plants, plant propagation materials, such as seeds, soil, or the area, material or environment by the pests. Suitable application methods include inter alia soil treatment, seed treatment, in furrow application, and foliar application. Soil treatment methods include drenching the soil, drip irrigation (drip application onto the soil), dipping roots, tubers or bulbs, or soil injection. Seed treatment techniques include seed dressing, seed coating, seed dusting, seed soaking, and seed pelleting. In furrow applications typically include the steps of making a furrow in cultivated land, seeding the furrow with seeds, applying the pesticidally active compound to the furrow, and closing the furrow. Foliar application refers to the application of the pesticidally active compound to plant foliage, e.g. through spray equipment. For foliar applications, it can be advantageous to modify the behavior of the pests by use of pheromones in combination with the compounds of the present invention. Suitable pheromones for specific crops and pests are known to a skilled person and publicly available from databases of pheromones and semiochemicals, such as http://www.pherobase.com.

As used herein, the term "contacting" includes both direct contact (applying the compounds/compositions directly on the animal pest or plant - typically to the foliage, stem or roots of the plant) and indirect contact (applying the compounds/compositions to the locus, i.e. habitat, breeding ground, plant, seed, soil, area, material or environment in which a pest is growing or may grow, of the animal pest or plant).

The term "animal pest" includes arthropods, gastropods, and nematodes. Preferred animal pests according to the invention are arthropods, preferably insects and arachnids, in particular insects. Insects, which are of particular relevance for crops, are typically referred to as crop insect pests.

The term "crop" refers to both, growing and harvested crops.

The term "plant" includes cereals, e.g. durum and other wheat, rye, barley, triticale, oats, rice, or maize (fodder maize and sugar maize / sweet and field corn); beet, e.g. sugar beet or fodder beet; fruits, such as pomes, stone fruits or soft fruits, e.g. apples, pears, plums, peaches, nectarines, almonds, cherries, papayas, strawberries, raspberries, blackberries or gooseberries; leguminous plants, such as beans, lentils, peas, alfalfa or soybeans; oil plants, such as rapeseed (oilseed rape), turnip rape, mustard, olives, sunflowers, coconut, cocoa beans, castor oil plants, oil palms, ground nuts or soybeans; cucurbits, such as squashes, pumpkins, cucumber or melons; fiber plants, such as cotton, flax, hemp or jute; citrus fruit, such as oranges, lemons, grapefruits or mandarins; vegetables, such as eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, such as avocados, cinnamon or camphor; energy and raw material plants, such as corn, soybean, rapeseed, sugar cane or oil palm; tobacco; nuts, e.g. walnuts; pistachios; coffee; tea; bananas; vines (table grapes and grape juice grape vines); hop; sweet leaf (also called Stevia); natural rubber plants or ornamental and forestry plants, such as flowers (e.g. carnation, petunias, geranium/pelargoniums, pansies and impatiens), shrubs, broad-leaved trees (e.g. poplar) or evergreens, e.g. conifers; eucalyptus; turf; lawn; grass such as grass for animal feed or ornamental uses. Preferred plants include potatoes sugar beets, tobacco, wheat, rye, barley, oats, rice, corn, cotton, soybeans, rapeseed, legumes, sunflowers, coffee or sugar cane; fruits; vines; ornamentals; or vegetables, such as cucumbers, tomatoes, beans or squashes.

The term "plant" is to be understood as including wild type plants and plants, which have been modified by either conventional breeding, or mutagenesis or genetic engineering, or by a combination thereof. Plants, which have been modified by mutagenesis or genetic engineering, and are of particular commercial importance, include alfalfa, rapeseed (e.g. oilseed rape), bean, carnation, chicory, cotton, eggplant, eucalyptus, flax, lentil, maize, melon, papaya, petunia, plum, poplar, potato, rice, soybean, squash, sugar beet, sugarcane, sunflower, sweet pepper, tobacco, tomato, and cereals (e.g. wheat), in particular maize, soybean, cotton, wheat, and rice. In plants, which have been modified by mutagenesis or genetic engineering, one or more genes have been mutagenized or integrated into the genetic material of the plant. The one or more mutagenized or integrated genes are preferably selected from pat, epsps, cry1Ab, bar, cry1Fa2, cry1Ac, cry34Ab1, cry35AB1, cry3A, cryF, cry1F, mcry3a, cry2Ab2, cry3Bb1, cry1A.105, dfr, barnase, vip3Aa20, barstar, als, bxn, bp40, asn1, and ppo5. The mutagenesis or integration of the one or more genes is performed in order to improve certain properties of the plant. Such properties, also known as traits, include abiotic stress tolerance, altered growth/yield, disease resistance, herbicide tolerance, insect resistance, modified product quality, and pollination control. Of these properties, herbicide tolerance, e.g. imidazolinone tolerance, glyphosate tolerance, or glufosinate tolerance, is of particular importance. Several plants have been rendered tolerant to herbicides by mutagenesis, for example Clearfield® oilseed rape being tolerant to imidazolinones, e.g. imazamox. Alternatively, genetic engineering methods have been used to render plants, such as soybean, cotton, corn, beets and oil seed rape, tolerant to herbicides, such as glyphosate and glufosinate, some of which are commercially available under the trade names RoundupReady® (glyphosate) and LibertyLink® (glufosinate). Furthermore, insect resistance is of importance, in particular lepidopteran insect resistance and coleopteran insect resistance. Insect resistance is typically achieved by modifying plants by integrating cry and/or vip genes, which were isolated from *Bacillus thuringiensis* (Bt)*,* and code for the respective Bt toxins. Genetically modified plants with insect resistance are commercially available under trade names including WideStrike®, Bollgard®, Agrisure®, Herculex®, Yield-Gard®, Genuity®, and Intacta®. Plants may be modified by mutagenesis or genetic engineering either in terms of one property (singular traits) or in terms of a combination of properties (stacked traits). Stacked traits, e.g. the combination of herbicide tolerance and insect resistance, are of increasing importance. In general, all relevant modified plants in connection with singular or stacked traits as well as detailed information as to the mutagenized or integrated genes and the respective events are available from websites of the organizations "International Service for the Acquisition of Agri-biotech Applications (ISAAA)" (http://www.isaaa.org/gmapprovaldatabase) and "Center for Environmental Risk Assessment (CERA)" (http://cera-gmc.org/GMCropDatabase).

It has surprisingly been found that the pesticidal activity of the compounds of the present invention may be enhanced by the insecticidal trait of a modified plant. Furthermore, it has been found that the compounds of the present invention are suitable for preventing insects to become resistant to the insecticidal trait or for combating pests, which already have become resistant to the insecticidal trait of a modified plant. Moreover, the compounds of the present invention are suitable for combating pests, against which the insecticidal trait is not effective, so that a complementary insecticidal activity can advantageously be used.

The term "plant propagation material" refers to all the generative parts of the plant such as seeds and vegetative plant material such as cuttings and tubers (e.g. potatoes), which can be used for the multiplication of the plant. This includes seeds, roots, fruits, tubers, bulbs, rhizomes, shoots, sprouts and other parts of plants. Seedlings and young plants, which are to be transplanted after germination or after emergence from soil, may also be included. These plant propagation materials may be treated prophylactically with a plant protection compound either at or before planting or transplanting.

The term "seed" embraces seeds and plant propagules of all kinds including but not limited to true seeds, seed pieces, suckers, corms, bulbs, fruit, tubers, grains, cuttings, cut shoots and the like, and means in a preferred embodiment true seeds.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application, and the like.

In the case of soil treatment, in furrow application or of application to the pests dwelling place or nest, the quantity of active ingredient ranges from 0.0001 to 500 g per 100 m², preferably from 0.001 to 20 g per 100 m².

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare, more desirably from 10 g to 50 g per hectare, e.g., 10 to 20 g per hectare, 20 to 30 g per hectare, 30 to 40 g per hectare, or 40 to 50 g per hectare.

The compounds of the invention are particularly suitable for use in the treatment of seeds in order to protect the seeds from insect pests, in particular from soil-living insect pests, and the resulting seedling's roots and shoots against soil pests and foliar insects. The invention therefore also relates to a method for the protection of seeds from insects, in particular from soil insects, and of the seedling's roots and shoots from insects, in particular from soil and foliar insects, said method comprising treating the seeds before sowing and/or after pregermination with a compound of the invention. The protection of the seedling's roots and shoots is preferred. More preferred is the protection of seedling's shoots from piercing and sucking insects, chewing insects and nematodes.

The term "seed treatment" comprises all suitable seed treatment techniques known in the art, such as seed dressing, seed coating, seed dusting, seed soaking, seed pelleting, and in-furrow application methods. Preferably, the seed treatment application of the active compound is carried out by spraying or by dusting the seeds before sowing of the plants and before emergence of the plants.

The invention also comprises seeds coated with or containing the active compound. The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

Suitable seed is for example seed of cereals, root crops, oil crops, vegetables, spices, ornamentals, for example seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugar beet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants such as potatoes, sugar cane, tobacco, grapes, petunias, geranium/pelargoniums, pansies and impatiens.

In addition, the active compound may also be used for the treatment of seeds from plants, which have been modified by mutagenesis or genetic engineering, and which e.g. tolerate the action of herbicides or fungicides or insecticides. Such modified plants have been described in detail above.

Conventional seed treatment formulations include for example flowable concentrates FS, solutions LS, suspoemulsions (SE), powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter. Preferably, the formulations are applied such that germination is not included.

The active substance concentrations in ready-to-use formulations, which may be obtained after two-to-tenfold dilution, are preferably from 0.01 to 60% by weight, more preferably from 0.1 to 40 % by weight.

In a preferred embodiment a FS formulation is used for seed treatment. Typically, a FS formulation may comprise 1-800 g/l of active ingredient, 1-200 g/l Surfactant, 0 to 200 g/l anti- freezing agent, 0 to 400 g/l of binder, 0 to 200 g/l of a pigment and up to 1 litre of a solvent, preferably water.

Especially preferred FS formulations of the compounds of the invention for seed treatment usually comprise from 0.1 to 80% by weight (1 to 800 g/l) of the active ingredient, from 0.1 to 20 % by weight (1 to 200 g/l) of at least one surfactant, e.g. 0.05 to 5 % by weight of a wetter and from 0.5 to 15 % by weight of a dispersing agent, up to 20 % by weight, e.g. from 5 to 20 % of an anti-freeze agent, from 0 to 15 % by weight, e.g. 1 to 15 % by weight of a pigment and/or a dye, from 0 to 40 % by weight, e.g. 1 to 40 % by weight of a binder (sticker /adhesion agent), optionally up to 5 % by weight, e.g. from 0.1 to 5 % by weight of a thickener, optionally from 0.1 to 2 % of an anti-foam agent, and optionally a preservative such as a biocide, antioxidant or the like, e.g. in an amount from 0.01 to 1 % by weight and a filler/vehicle up to 100 % by weight.

In the treatment of seed, the application rates of the compounds of the invention are generally from 0.1 g to 10 kg per 100 kg of seed, preferably from 1 g to 5 kg per 100 kg of seed, more preferably from 1 g to 1000 g per 100 kg of seed and in particular from 1 g to 200 g per 100 kg of seed, e.g. from 1 g to 100 g or from 5 g to 100 g per 100 kg of seed.

The invention therefore also relates to seed comprising a compound of the invention, or an agriculturally useful salt thereof, as defined herein. The amount of the compound of the invention or the agriculturally useful salt thereof will in general vary from 0.1 g to 10 kg per 100 kg of seed, preferably from 1 g to 5 kg per 100 kg of seed, in particular from 1 g to 1000 g per 100 kg of seed. For specific crops such as lettuce the rate can be higher.

The invention also relates to a composition comprising a compound of the present invention, or an agriculturally useful salt thereof, as defined herein, and seed. The amount of the compound of the present invention or the agriculturally useful salt thereof will in general vary from 0.1 g to 10 kg per 100 kg of seed, preferably from 1 g to 5 kg per 100 kg of seed, in particular from 1 g to 1000 g per 100 kg of seed. For specific crops such as lettuce the rate can be higher.

The invention also relates to a composition comprising a compound of the present invention, or an agriculturally useful salt thereof, as defined herein, and plant propagation material. The amount of the compound of the present invention or the agriculturally useful salt thereof will in general vary from 0.1 g to 10 kg per 100 kg of plant propagation material, preferably from 1 g to 5 kg per 100 kg of plant propagation material, in particular from 1 g to 1000 g per 100 kg of plant propagation material. For specific crops such as lettuce the rate can be higher.

The compounds of the invention may also be used for improving the health of a plant. Therefore, the invention also relates to a method for improving plant health by treating a plant, plant propagation material and/or the locus where the plant is growing or is to grow with an effective and non-phytotoxic amount of a compound of the invention.

As used herein "an effective and non-phytotoxic amount" means that the compound is used in a quantity which allows to obtain the desired effect but which does not give rise to any phytotoxic symptom on the treated plant or on the plant grown from the treated propagule or treated soil.

The terms "plant" and "plant propagation material" are defined above.

"Plant health" is defined as a condition of the plant and/or its products which is determined by several aspects alone or in combination with each other such as yield (for example increased biomass and/or increased content of valuable ingredients), quality (for example improved content or composition of certain ingredients or shelf life), plant vigour (for example improved plant growth and/or greener leaves ("greening effect"), tolerance to abiotic (for example drought) and/or biotic stress (for example disease) and production efficiency (for example, harvesting efficiency, processability).

The above identified indicators for the health condition of a plant may be interdependent and may result from each other. Each indicator is defined in the art and can be determined by methods known to a skilled person.

The compounds of the invention are also suitable for use against non-crop insect pests. For use against said non-crop pests, compounds of the invention can be used as bait composition, gel, general insect spray, aerosol, as ultra-low volume application and bed net (impregnated or surface applied). Furthermore, drenching and rodding methods can be used.

As used herein, the term "non-crop insect pest" refers to pests, which are particularly relevant for non-crop targets, such as ants, termites, wasps, flies, ticks, mosquitos, crickets, or cockroaches.

The bait can be a liquid, a solid or a semisolid preparation (e.g. a gel). The bait employed in the composition is a product, which is sufficiently attractive to incite insects such as ants, termites, wasps, flies, mosquitos, crickets etc. or cockroaches to eat it. The attractiveness can be manipulated by using feeding stimulants or sex pheromones. Food stimulants are chosen, for example, but not exclusively, from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are described in the literature (e.g. http://www.pherobase.com), and are known to those skilled in the art.

For use in bait compositions, the typical content of active ingredient is from 0.001 weight % to 15 weight %, desirably from 0.001 weight % to 5% weight % of active compound.

Formulations of the compounds of the invention as aerosols (e.g. in spray cans), oil sprays or pump sprays are highly suitable for the non-professional user for controlling pests such as flies, fleas, ticks, mosquitos or cockroaches. Aerosol recipes are preferably composed of the active compound, solvents, furthermore auxiliaries such as emulsifiers, perfume oils, if appropriate stabilizers, and, if required, propellants.

The oil spray formulations differ from the aerosol recipes in that no propellants are used.

For use in spray compositions, the content of active ingredient is from 0.001 to 80 weights %, preferably from 0.01 to 50 weight % and most preferably from 0.01 to 15 weight %.

The compounds of the invention and its respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniosis) with compounds of the invention and its respective compositions also comprise treating surfaces of huts and houses, air spraying and impregnation of curtains, tents, clothing items, bed nets, tsetse-fly trap or the like. Insecticidal compositions for application to fibres, fabric, knit goods, nonwovens, netting material or foils and tarpaulins preferably comprise a mixture including the insecticide, optionally a repellent and at least one binder.

The compounds of the invention and its compositions can be used for protecting wooden materials such as trees, board fences, sleepers, frames, artistic artifacts, etc. and buildings, but also construction materials, furniture, leathers, fibers, vinyl articles, electric wires and cables etc. from ants and/or termites, and for controlling ants and termites from doing harm to crops or human being (e.g. when the pests invade into houses and public facilities).

Customary application rates in the protection of materials are, for example, from 0.001 g to 2000 g or from 0.01 g to 1000 g of active compound per m² treated material, desirably from 0.1 g to 50 g per m².

Insecticidal compositions for use in the impregnation of materials typically contain from 0.001 to 95 weight %, preferably from 0.1 to 45 weight %, and more preferably from 1 to 25 weight % of at least one repellent and/or insecticide.

The compounds of the invention are especially suitable for efficiently combating animal pests such as arthropods, gastropods and nematodes including but not limited to:
insects from the order of Lepidoptera, for example *Achroia grisella, Acleris* spp. such as *A. fimbriana, A. gloverana, A. variana; Acrolepiopsis assectella, Acronicta major, Adoxophyes* spp. such as *A. cyrtosema, A. orana; Aedia leucomelas, Agrotis* spp. such as *A. exclamationis, A. fucosa, A. ipsilon, A. orthogoma, A. segetum, A. subterranea; Alabama argillacea, Aleurodicus dispersus, Al sophila pometaria, Ampelophaga rubiginosa, Amyelois transitella, Anacampsis sarcitella, Anagasta kuehniella, Anarsia lineatella, Anisota senatoria, Antheraea pernyi, Anticarsia (=Thermesia)* spp. such as *A. gemmatalis; Apamea* spp., *Aproaerema modicella, Archips* spp. such as *A. argyrospila, A. fuscocupreanus, A. rosana, A. xyloseanus; Argyresthia conjugella, Argyroploce* spp., *Argyrotaenia* spp. such as *A. velutinana; Athetis mindara, Austroasca viridigrisea, Autographa gamma, Autographa nigrisigna, Barathra brassicae, Bedellia* spp., *Bonagota salubricola, Borbo cinnara, Bucculatrix thurberiella, Bupalus piniarius, Busseola spp., Cacoecia* spp. such as *C. murinana, C. podana; Cactoblastis cactorum, Cadra cautella, Calingo braziliensis, Caloptilis theivora, Capua reticulana, Carposina* spp. such as *C. niponensis, C. sasakii; Cephus* spp., *Chaetocnema aridula, Cheimatobia brumata, Chilo* spp. such as *C. Indicus, C. suppressalis, C. partellus; Choreutis pariana, Choristoneura* spp. such as *C. conflictana, C. fumiferana, C. longicellana, C. murinana, C. occidentalis, C. rosaceana; Chrysodeixis (=Pseudoplusia)* spp. *such as C. eriosoma, C. includes; Cirphis unipuncta, Clysia ambiguella, Cnaphalocerus* spp., *Cnaphalocrocis medinalis, Cnephasia* spp., *Cochylis hospes, Coleophora* spp., *Colias eurytheme, Conopomorpha* spp., *Conotrachelus* spp., *Copitarsia* spp., *Corcyra cephalonica, Crambus caliginosellus, Crambus teterrellus, Crocidosema (=Epinotia) aporema, Cydalima (=Diaphania) perspectalis, Cydia (=Carpocapsa)* spp. such as *C. pomonella, C. latiferreana; Dalaca noctuides, Datana integerrima, Dasychira pinicola, Dendrolimus* spp. such as *D. pini, D. spectabilis, D. sibiricus; Desmia funeralis, Diaphania* spp. such as *D. nitidalis, D. hyalinata; Diatraea grandiosella, Diatraea saccharalis, Diphthera festiva, Earias* spp. such as *E. insulana, E. vittella; Ecdytolopha aurantianu Egira (=Xylomyges) curialis, Elasmopalpus lignosellus, Eldana saccharina, Endopiza viteana, Ennomos subsignaria, Eoreuma loftini, Ephestia* spp. such as *E. cautella, E. elutella, E kuehniella; Epinotia aporema, Epiphyas postvittana, Erannis tiliaria, Erionota thrax, Etiella spp., Eulia* spp., *Eupoecilia ambiguella, Euproctis chrysorrhoea, Euxoa spp., Evetria bouliana, Faronta albilinea, Feltia* spp. such as *F subterranean; Galleria mellonella, Gracillaria* spp., *Grapholita* spp. such as *G. funebrana, G. molest, G. inopinata; Halysidota* spp., *Harrisina americana, Hedylepta* spp., *Helicoverpa* spp. such as *H. armigera (=Heliothis armigera), H. zea (=Heliothis zea); Heliothis* spp. such as *H. assulta, H. subflexa, H. virescens; Hellula* spp. such as *H. undalis, H. rogatalis; Heloco verpa gelotopoeon, Hemileuca oliviae, Herpetogramma licarsisalis, Hibernia defoliaria, Hofmannophila pseudospretella, Homoeosoma electellum, Homona magnanima, Hypena scabra, Hyphantria cunea, Hyponomeuta padella, Hyponomeuta malinellus, Kakivoria flavofasciata, Keiferia lycopersicella, Lambdina fiscellaria fiscellaria, Lambdina fiscellaria lugubrosa, Lamprosema indicata, Laspeyresia molesta, Leguminivora glycinivorella, Lerodea eufala, Leucinodes orbonalis, Leucoma salicis, Leucoptera* spp. such as *L. coffeella, L. scitella; Leuminivora lycinivorella, Lithocolletis blancardella, Lithophane antennata, Llattia octo (=Amyna axis), Lobesia botrana, Lophocampa* spp., *Loxagrotis albicosta, Loxostege* spp. such as *L. sticticalis, L. cereralis; Lymantria* spp. such as *L. dispar, L. monacha; Lyonetia clerkella, Lyonetia prunifoliella, Malacosoma spp.* such as *M. americanum, M. californicum, M. constrictum, M. neustria; Mamestra* spp. such as *M. brassicae, M. configurata; Mamstra brassicae, Manduca* spp. such as *M. quinquemaculata, M. sexta; Marasmia spp, Marmara* spp., *Maruca testulalis, Megalopyge lanata, Melanchra picta, Melanitis leda, Mocis* spp. such as *M. lapites, M. repanda; Mocis latipes, Monochroa fragariae, Mythimna separata, Nemapogon cloacella, Neoleucinodes elegantalis, Nepytia spp., Nymphula* spp.*, Oiketicus spp., Omiodes indicata, Omphisa anastomosalis, Operophtera brumata, Orgyia pseudotsugata, Oria* spp., *Orthaga thyrisalis, Ostrinia* spp. such as *O. nubila*/*is; Oulema oryzae, Paleacrita vernata, Panolis flammea, Parnara* spp., *Papaipema nebris, Papilio cresphontes, Paramyelois transitella, Paranthrene regalis, Paysandisia archon, Pectinophora* spp. such as *P. gossypiella; Peridroma saucia, Perileucoptera* spp., such as *P. coffeella; Phalera bucephala, Phryganidia californica, Phthorimaea* spp. such as *P. operculella; Phyllocnistis citrella, Phyllonorycter* spp. such as *P. blancardella, P. crataegella, P. issikii, P. ringoniella; Pieris* spp. such as *P. brassicae, P. rapae, P. napi; Pilocrocis tripunctata, Plathypena scabra, Platynota* spp. such as *P. flavedana, P. idaeusalis, P. stultana; Platyptilia carduidactyla, Plebejus argus, Plodia inter-punctella, Plusia spp, Plutella maculipennis, Plutella xylostella, Pontia protodica, Prays* spp., *Prodenia* spp., *Proxenus lepigone, Pseudaletia* spp. such as *P. sequax, P. unipuncta; Pyrausta nu-bilalis, Rachiplusia nu, Richia albicosta, Rhizobius ventralis, Rhyacionia frustrana, Sabulodes ae-grotata, Schizura concinna, Schoenobius* spp., *Schreckensteinia festaliella, Scirpophaga* spp. such as *S. incertulas, S. innotata; Scotia segetum, Sesamia* spp. such as *S. inferens, Seudyra subflava, Sitotroga cerealella, Sparganothis pilleriana, Spilonota lechriaspis, S. ocellana, Spodoptera (=Lam-phygma)* spp. such as *S. cosmoides, S. eridania, S. exigua, S. frugiperda, S. latisfascia, S. littoralis, S. litura, S. omithogalli; Stigmella* spp., *Stomopteryx subsecivella, Strymon bazochii, Sylepta dero-gata, Synanthedon* spp. such as S. exitiosa, *Tecia solanivora, Telehin licus, Thaumatopoea pityocampa, Thaumatotibia (=Cryptophlebia) leucotreta, Thaumetopoea pityocampa, Thecla* spp., *Theresimima ampelophaga, Thyrinteina* spp, *Tildenia inconspicuella, Tinea* spp. such as *T. cloa-cella, T. pellionella; Tineola bisselliella, Tortrix* spp. such as *T. viridana; Trichophaga tapetzella, Tri-choplusia* spp. such as *T. ni; Tuta (=Scrobipalpula) absoluta, Udea* spp. such as *U. rubigalis, U. ru-bigalis; Virachola* spp., *Yponomeuta padella, and Zeiraphera canadensis;*
insects from the order of Coleoptera, for example *Acalymma vittatum, Acanthoscehdes obtectus, Adoretus* spp., *Agelastica alni, Agrilus* spp. such as *A. anxius, A. planipennis, A. sinuatus; Agriotes* spp. such as *A. fuscicollis, A. lineatus, A. obscurus; Alphitobius diaperinus, Amphimallus solstitialis, Anisandrus dispar, Anisoplia austriaca, Anobium punctatum, Anomala corpulenta, Anomala rufocuprea, Anoplophora* spp. such as *A. glabripennis; Anthonomus* spp. such as *A. eugenii, A. grandis, A. pomorum; Anthrenus* spp., *Aphthona euphoridae, Apion* spp., *Apogonia* spp., *Athous haemorrhoidalis, Atomaria* spp. such as *A. linearis; Attagenus* spp., *Aulacophora femoralis, Blastophagus piniperda, Blitophaga undata, Bruchidius obtectus, Bruchus* spp. such as *B. lentis, B. pisorum, B. rufimanus; Byctiscus betulae, Callidiellum rufipenne, Callopistria floridensis, Callosobruchus chinensis, Cameraria ohridella, Cassida nebulosa, Cerotoma trifurcata, Cetonia aurata, Ceuthorhynchus* spp. such as *C. assimilis, C. napi; Chaetocnema tibialis, Cleonus mendicus, Conoderus* spp. such as *C*. *vespertinus; Conotrachelus nenuphar, Cosmopolites* spp., *Costelytra zealandica, Crioceris asparagi, Cryptolestes ferrugineus, Cryptorhynchus lapathi, Ctenicera* spp. such as *C. destructor; Curculio* spp., *Cylindrocopturus* spp., *Cyclocephala* spp., *Dactylispa balyi, Dectes texanus, Dermestes* spp., *Diabrotica* spp. such as *D. undecimpunctata, D. speciosa, D. longicornis, D. semi-punctata, D. virgifera; Diaprepes abbreviates, Dichocrocis* spp., *Dicladispa armigera, Diloboderus abderus, Diocalandra frumenti (Diocalandra stigmaticollis), Enaphalodes rufulus, Epilachna* spp. such as *E. varivestis, E. vigintioctomaculata; Epitrixspp.* such as *E. hirtipennis, E. similaris; Eutheola humilis, Eutinobothrus brasiliensis, Faustinus cubae, Gibbium psylloides, Gnathocerus cornutus, Hellula undalis, Heteronychus arator, Hylamorpha elegans, Hylobius abietis, Hylotrupes bajulus, Hypera* spp*.* such as *H. brunneipennis, H. postica; Hypomeces squamosus, Hypothenemus* spp., *lps typographus, Lachnosterna consanguinea, Lasioderma serricorne, Latheticus oryzae, Lathridius* spp., *Lema* spp. such as *L. bilineata, L. melanopus; Leptinotarsa* spp. such as *L. decemlineata; Leptispa pygmaea, Limonius californicus, Lissorhoptrus oryzophilus, Lixus* spp., *Luperodes* spp., *Lyctus* spp. such as *L. bruneus; Liogenys fuscus*, *Macrodactylus* spp. such as *M. subspi-nous; Maladera matrida, Megaplatypus mutates, Megascelis* spp., *Melanotus communis, Meli-gethes* spp. such as *M. aeneus; Melolontha* spp. such as *M. hippocastani, M. melolontha; Meta-masius hemipterus, Microtheca* spp., *Migdolus* spp. such as *M. fryanus, Monochamus* spp. such as *M. alternatus; Naupactus xanthographus, Niptus hololeucus, Oberia brevis, Oemona hirta, Oryctes rhinoceros, Oryzaephilus surinamensis, Oryzaphagus oryzae, Otiorrhynchus sulcatus, Otiorrhynchus ovatus, Otiorrhynchus sulcatus, Oulema melanopus, Oulema oryzae, Oxycetonia jucunda, Phaedon* spp. such as *P. brassicae, P. cochleariae; Phoracantha recurva, Phyllobius pyri, Phyllopertha horticola, Phyllophaga* spp. such as *P. helleri; Phyllotreta* spp. such as *P. chrysocephala, P. nemorum, P. striolata, P. vittula; Phyllopertha horticola, Popillia japonica, Premnotrypes* spp., *Psacothea hilaris, Psylliodes chrysocephala, Prostephanus truncates, Psylliodes* spp., *Ptinus* spp., *Pulga saltona, Rhizopertha dominica, Rhynchophorus* spp. such as *R. billineatus, R. ferrugineus, R. palmarum, R. phoenicis, R. vulneratus; Saperda candida, Scolytus schevyrewi, Scyphophorus acupunctatus, Sitonalineatus, Sitophilus* spp. such as *S. granaria, S. oryzae, S. zeamais; Sphenophorus* spp. such as *S. levis; Stegobium paniceum, Sternechus* spp. such as *S. subsignatus; Strophomorphus ctenotus, Symphyletes* spp., *Tanymecus* spp., *Tenebrio molitor, Tenebrioides mauretanicus, Tribolium* spp. such as *T. castaneum; Trogoderma* spp., *Tychius* spp., *Xylotrechus* spp. such as *X. pyrrhoderus; and, Zabrus spp. such as Z. tenebrioides;*
insects from the order of Diptera e.g. *Aedes* spp. such as *A. aegypti, A. albopictus, A. vexans; Anastrepha ludens, Anopheles* spp. such as *A. albimanus, A. crucians, A. freeborni, A. gambiae, A. leucosphyrus, A. maculipennis, A. minimus, A. quadrimaculatus, A. sinensis; Bactrocera invadens, Bibio hortulanus, Calliphora erythrocephala, Calliphora vicina, Ceratitis capitata, Chrysomyia* spp. such as *C. bezziana, C. hominivorax, C. macellaria; Chrysops atlanticus, Chrysops discalis, Chrysops silacea, Cochliomyia* spp. such as *C. hominivorax; Contarinia* spp. such as *C. sorghicola; Cordylobia anthropophaga, Culexspp.* such as *C. nigripalpus, C. pipiens, C. quinquefasciatus, C. tarsalis, C. tritaeniorhynchus; Culicoides furens, Culiseta inornata, Culiseta melanura, Cuterebra* spp., *Dacus cucurbitae, Dacus oleae, Dasineura brassicae, Dasineura oxycoccana, Delia* spp. such as *D. antique, D. coarctata, D. platura, D. radicum; Dermatobia hominis, Drosophila* spp. such as *D. suzukii, Fannia* spp. such as *F. canicularis; Gastraphilus* spp. such as *G. intestinalis; Geomyza tipunctata, Glossina* spp. such as *G. fuscipes, G. morsitans, G. palpalis, G. tachinoides; Haematobia irritans, Haplodiplosis equestris, Hippelates* spp., Hylemyia spp. such as *H. platura; Hypoderma* spp. such as *H. lineata; Hyppobosca* spp., *Hydrellia philippina, Leptoconops torrens, Liriomyza* spp. such as *L. sativae, L. trifolii; Lucilia* spp. such as *L. caprina, L. cuprina, L. sericata; Lycoria pectoralis, Mansonia titillanus, Mayetiola* spp. such as *M. destructor; Musca* spp. such as *M. autumnalis, M. domestica; Muscina stabulans, Oestrus* spp. such as *O. ovis; Opomyza florum, Oscinella* spp. such as *O. frit; Orseolia oryzae, Pegomya hysocyami, Phlebotomus argentipes, Phorbia* spp. such as *P. antiqua, P. brassicae, P. coarctata; Phytomyza gymnostoma, Prosimulium mixtum, Psila rosae, Psorophora columbiae, Psorophora discolor, Rhagoletis* spp. such as *R. cerasi, R. cingulate, R. indifferens, R. mendax, R. pomonella; Rivellia quadrifasciata, Sarcophaga* spp. such as *S. haemorrhoidalis; Simulium vittatum, Sitodiplosis mosellana, Stomoxys* spp. such as *S. calcitrans; Tabanus* spp. such as *T. atratus, T. bovinus, T. lineola, T. similis; Tannia spp., Thecodiplosis japonensis, Tipula oleracea, Tipula paludosa,* and *Wohlfahrtia* spp;
insects from the order of Thysanoptera for example, *Baliothrips biformis, Dichromothrips corbetti, Dichromothrips* ssp., *Echinothrips americanus, Enneothrips flavens, Frankliniella* spp. such as *F. fusca, F. occidentalis, F. tritici; Heliothrips* spp., *Hercinothrips femoralis, Kakothrips* spp., *Micro-cephalothrips abdominalis, Neohydatothrips samayunkur, Pezothrips kellyanus, Rhipiphorothrips cruentatus, Scirtothrips* spp. such as *S. citri, S. dorsalis, S. perseae; Stenchaetothrips spp, Taeniothrips cardamoni, Taeniothrips inconsequens, Thrips* spp. such as *T. imagines, T. hawaiiensis, T. oryzae, T. palmi, T. parvispinus, T. tabaci;*
insects from the order of Hemiptera for example, *Acizziajamatonica, Acrosternum* spp. such as *A. hilare;* Acyrthosipon spp. such as *A. onobrychis, A. pisum; Adelges laricis, Adelges tsugae, Adelphocoris* spp., such as *A. rapidus, A. superbus; Aeneolamia* spp., *Agonoscena spp., Aulacorthum solani, Aleurocanthus woglumi, Aleurodes* spp., *Aleurodicus disperses, Aleurolobus barodensis, Aleurothrixus* spp., *Amrasca* spp., *Anasa tristis, Antestiopsis* spp., *Anuraphis cardui, Aonidiella* spp., *Aphanostigma piri, Aphidula nasturtii, Aphis* spp. such as *A. craccivora, A. fabae, A. forbesi, A. gossypii, A. grossulariae, A. maidiradicis, A. pomi, A. sambuci, A. schneideri, A. spiraecola; Arboridia apicalis, Arilus critatus, Aspidiella* spp., *Aspidiotus* spp., *Atanus* spp., *Aulacaspis yasumatsui, Aulacorthum solani, Bactericera cockerelli (Paratrioza cockerelli), Bemisia* spp. such as *B. argentifolii, B. tabaci (Aleurodes tabaci); Blissus* spp. such as *B. leucopterus; Brachycaudus* spp. such as *B. cardui, B. helichrysi, B. persicae, B. prunicola; Brachycolus* spp., *Brachycorynella asparagi, Brevicoryne brassicae, Cacopsylla* spp. such as *C. fulguralis, C. pyricola (Psylla piri); Calligypona marginata, Calocoris* spp., *Campylomma livida, Capitophorus horni, Carneocephala fulgida, Cavelerius* spp., *Ceraplastes* spp., *Ceratovacuna lanigera, Ceroplastes ceriferus, Cerosipha gossypii, Chaetosiphon fragaefolii, Chionaspis tegalensis, Chlorita onukii, Chromaphis juglandicola, Chrysomphalus ficus, Cicadulina mbila, Cimexspp.* such as *C. hemipterus, C. lectularius; Coccomytilus halli, Coccus* spp. such as *C. hesperidum, C. pseudomagnoliarum; Corythucha arcuata, Creontiades dilutus, Cryptomyzus ribis, Chrysomphalus aonidum, Cryptomyzus ribis, Ctenarytaina spatulata, Cyrtopeltis notatus, Dalbulus* spp., *Dasynus piperis, Dialeurodes spp.* such as *D. citrifolii; Dalbulus maidis, Diaphorina* spp. such as *D. citri; Diaspis* spp. such as *D. bromeliae; Dichelops furcatus, Diconocoris hewetti, Doralis* spp., *Dreyfusia nordmannianae, Dreyfusia piceae, Drosicha* spp., *Dysaphis* spp*.* such as *D. plantaginea, D. pyri, D. radicola; Dysaulacorthum pseudosolani, Dysdercus* spp*.* such as *D. cingulatus, D. intermedius; Dysmicoccus* spp., *Edessa* spp., *Geocoris* spp., *Empoasca* spp. such as *E. fabae, E. solana; Epidiaspis leperii, Eriosoma* spp. such as *E. lanigerum, E. pyricola; Erythroneura* spp., *Eurygasterspp.* such as *E. integriceps; Euscelis bilobatus, Euschistus* spp. such as *E. heros, E. impictiventris, E. servus; Fiorinia theae, Geococcus coffeae, Glycaspis brimblecombei, Halyomorpha* spp. such as *H. halys; Heliopeltis* spp., *Homalodisca vitripennis (=H. coagulata), Horcias nobilellus, Hyalopterus pruni, Hyperomyzus lactucae, Icerya* spp. such as *I*. *purchase; Idiocerus* spp., *Idioscopus* spp., *Laodelphax striatellus, Lecanium* spp., *Lecanoideus floccissimus, Lepidosaphes* spp. such as *L. ulmi; Leptocorisa* spp., *Leptoglossus phyllopus, Lipaphis erysimi, Lygus* spp. such as *L. hesperus, L. lineolaris, L. pratensis; Maconellicoccus hirsutus, Marchalina hellenica*, *Macropes excavatus, Macrosiphum* spp. such as *M. rosae, M. avenae, M. euphorbiae; Macrosteles quadrilineatus, Mahanarva fimbriolata, Megacopta cribraria, Megoura viciae, Melanaphis pyrarius, Melanaphis sacchari, Melanocallis (=Tinocallis) caryaefoliae, Metcafiella* spp., *Metopolophium dirhodum, Monellia costalis, Monelliopsis pecanis, Myzocallis coryli, Murgantia* spp., *Myzus* spp. such as *M. ascalonicus, M. cerasi, M. nicotianae, M. persicae, M. varians; Nasonovia ribis-nigri, Neotoxoptera formosana, Neomegalotomus spp, Nephotettix* spp. such as *N. malayanus, N. nigropictus, N. parvus, N. virescens; Nezara* spp. such as *N. viridula; Nilaparvata lugens, Nysius huttoni, Oebalus* spp. such as *O. pugnax; Oncometopia* spp., *Orthezia praelonga*, *Oxycaraenus hyalinipennis, Parabemisia myricae, Parlatoria* spp., *Parthenolecanium* spp. such as *P. corni, P. persicae; Pemphigus* spp. such as *P. bursarius, P. populivenae; Peregrinus maidis, Perkinsiella saccharicida, Phenacoccus* spp. such as *P. aceris, P. gossypii; Phloeomyzus passerinii, Phorodon humuli, Phylloxera* spp. such as *P. devastatrix, Piesma quadrata, Piezodorus* spp. such as *P. guildinii; Pinnaspis aspidistrae, Planococcus* spp. such as *P. citri, P. focus; Prosapia bicincta, Protopulvinaria pyriformis, Psallus seriatus, Pseudacysta persea, Pseudaulacaspis pentagona, Pseudococcus* spp. such as *P. comstocki; Psylla* spp. such as *P. mali; Pteromalus* spp., *Pulvinaria amygdali, Pyrilla* spp., *Quadraspidiotus* spp., such as *Q. perniciosus; Quesada gigas, Rastrococcus* spp., *Reduvius senilis, Rhizoecus americanus, Rhodnius spp., Rhopalomyzus ascalonicus, Rhopalosiphum* spp. such as *R. pseudobrassicas, R. insertum, R. maidis, R. padi; Sagatodes* spp., *Sahlbergella singularis, Saissetia* spp., *Sappaphis mala, Sappaphis mali, Scaptocoris* spp., *Scaphoides titanus, Schizaphis graminum, Schizoneura lanuginosa, Scotinophora* spp., *Selenaspidus articulatus, Sitobion avenae, Sogata* spp., *Sogatella furcifera, Solubea insularis, Spissistilus festinus (=Stictocephala festina), Stephanitis nashi, Stephanitis pyrioides, Stephanitis takeyai, Tenalaphara malayensis, Tetraleurodes perseae, Therioaphis maculate, Thyanta* spp. such as *T. accerra, T. perditor; Tibraca* spp., *Tomaspis* spp., *Toxoptera* spp*.* such as *T. aurantii; Trialeurodes* spp. such as *T. abutilonea, T. ricini, T. vaporariorum; Triatoma* spp., *Trioza* spp., *Typhlocyba* spp., *Unaspis* spp. such as *U. citri, U. yanonensis; and Viteus vitifolii,*
Insects from the order Hymenoptera for example *Acanthomyops interjectus, Athalia rosae, Atta* spp. *such asA. capiguara, A. cephalotes, A. cephalotes, A. laevigata, A. robusta, A. sexdens, A. texana, Bombus* spp., *Brachymyrmexspp., Camponotus* spp*.* such as *C. floridanus, C. pennsylvanicus, C. modoc; Cardiocondyla nuda, Chalibion sp, Crematogasterspp., Dasymutilla occidentalis, Diprion* spp., *Dolichovespula maculata, Dorymyrmex* spp*., Dryocosmus kuriphilus, Formica* spp., *Hoplocampa* spp. such as *H. minuta, H. testudinea; Iridomyrmex humilis,* Lasius spp. such as *L. niger, Linepithema humile, Liometopum* spp*.*, *Leptocybe invasa, Monomorium s*pp*.* such as *M. pharaonis, Monomorium, Nylandria fulva, Pachycondyla chinensis, Paratrechina longicornis, Paravespula* spp., such as *P. germanica, P. pennsylvanica, P. vulgaris; Pheidole* spp. such as *P. megacephala; Pogonomyrmex* spp. such as *P. barbatus, P. californicus, Polistes rubiginosa, Prenolepis impairs, Pseudomyrmexgracilis, Schelipron* spp., *Sirex cyaneus, Solenopsis* spp. such as *S. geminata, S.invicta, S. molesta, S. richteri, S. xyloni, Sphecius speciosus, Sphex* spp., *Tapinoma* spp. such as *T. melanocephalum, T. sessile; Tetramorium* spp. such as *T. caespitum, T. bicarinatum, Vespa* spp. such as *V. crabro; Vespula* spp. such as *V. squamosal; Wasmannia auropunctata, Xylocopa* sp;
Insects from the order Orthoptera for example *Acheta domesticus, Calliptamus italicus, Chortoicetes terminifera, Ceuthophilus* spp., *Diastrammena asynamora, Dociostaurus maroccanus, Gryllotalpa* spp. such as *G. africana, G. gryllotalpa; Gryllus* spp.*, Hieroglyphus daganensis, Kraussaria angulifera, Locusta* spp. such as *L. migratoria, L. pardalina; Melanoplus* spp. such as *M. bivittatus, M. femurrubrum, M. mexicanus, M. sanguinipes, M. spretus; Nomadacris septemfasciata, Oedaleus senegalensis, Scapteriscus* spp., *Schistocerca* spp. such as *S. americana, S. gregaria, Stemopelmatus* spp., *Tachycines asynamorus,* and *Zonozerus variegatus;*
Pests from the Class Arachnida for example Acari,e.g. of the families Argasidae, Ixodidae and Sarcoptidae, such as *Amblyomma* spp. (e.g. *A. americanum, A. variegatum, A. maculatum*), Argas spp. such as *A. persicu*)*, Boophilus* spp. such as *B. annulatus, B. decoloratus, B. microplus, Dermacentor* spp*.* such as *D.silvarum, D. andersoni, D. variabilis, Hyalomma* spp. such as *H. truncatum, Ixodes* spp. such as *I*. *ricinus, I*. *rubicundus, I*. *scapularis, I*. *holocyclus, I*. *pacificus, Rhipicephalus sanguineus, Ornithodorus* spp. such as *O. moubata, O. hermsi, O. turicata, Ornithonyssus bacoti, Otobius megnini, Dermanyssus gallinae, Psoroptes* spp. such as *P. ovis, Rhipicephalus* spp. such as *R. sanguineus, R. appendiculatus, Rhipicephalus evertsi, Rhizoglyphus* spp., *Sarcoptes* spp. such as *S*. *Scabiei*; and Family Eriophyidae including *Aceria* spp. such as *A. sheldoni, A. anthocoptes, Acallitus* spp., *Aculops* spp. such as *A. lycopersici, A. pelekassi*; *Aculus* spp. such as *A. schlechtendali; Colomerus vitis, Epitrimerus pyri, Phyllocoptruta oleivora; Eriophytes ribis* and *Eriophyes* spp. such as *Eriophyes sheldoni*; Family Tarsonemidae including *Hemitarsonemus* spp., *Phytonemus pallidus and Polyphagotarsonemus latus, Stenotarsonemus* spp. *Steneotarsonemus spinki*; Family Tenuipalpidae including Brevipalpus spp. such as *B. phoenicis,* Family Tetranychidae including *Eotetranychus* spp., *Eutetranychus* spp., *Oligonychus* spp., *Petrobia latens, Tetranychus* spp. such as *T. cinnabarinus, T. evansi, T. kanzawai, T, pacificus, T. phaseulus, T. telarius* and *T. urticae*; *Bryobia praetiosa*; *Panonychus* spp. such as *P. ulmi, P. citri*; *Metatetranychus* spp. and *Oligonychus* spp. such as *O. pratensis, O. perseae, Vasates lycopersici*; *Raoiella indica, Family* Carpoglyphidae including *Carpoglyphus* spp.; *Penthaleidae* spp. such as *Halotydeus destructor,* Family Demodicidae with species such as *Demodex* spp.; Family Trombicidea including *Trombicula* spp.; Family Macronyssidae including *Ornothonyssus* spp.; Family Pyemotidae including *Pyemotes tritici*; *Tyrophagus putrescentiae;* Family Acaridae including *Acarus siro*; Family Araneida including *Latrodectus mactans, Tegenaria agrestis, Chiracanthium sp, Lycosa sp Achaearanea tepidariorum* and *Loxosceles reclusa*;
Pests from the Phylum Nematoda, for example, plant parasitic nematodes such as root-knot nematodes, *Meloidogyne* spp. such as *M. hapla, M. incognita, M. javanica;* cyst-forming nematodes, *Globodera* spp. such as *G. rostochiensis*; *Heterodera* spp. such as *H. avenae, H. glycines, H. schachtii, H. trifolii;* Seed gall nematodes, *Anguina* spp.; Stem and foliar nematodes, *Aphelenchoides* spp. such as *A. besseyi;* Sting nematodes, *Belonolaimus* spp. such as *B. longicaudatus;* Pine nematodes, *Bursaphe*/*enchus* spp. such as *B. lignicolus, B. xylophilus;* Ring nematodes, *Criconema* spp.*, Criconemella* spp. such as *C. xenoplax and C. ornata;* and, *Criconemoides spp.* such as *Criconemoides informis; Mesocriconema* spp.; Stem and bulb nematodes, *Ditylenchus* spp. such as *D. destructor, D. dipsaci;* Awl nematodes, *Dolichodorus* spp.*;* Spiral nematodes, *Heliocotylenchus multicinctus;* Sheath and sheathoid nematodes, *Hemicycliophora* spp. and *Hemicriconemoides* spp.*; Hirshmanniella* spp.; Lance nematodes, *Hoploaimus* spp.*;* False rootknot nematodes, *Nacobbus* spp*.;* Needle nematodes, *Longidorus* spp. such as *L. elongatus;* Lesion nematodes, *Pratylenchus* spp. such as *P. brachyurus, P. neglectus, P. penetrans, P. curvitatus, P. goodeyi;* Burrowing nematodes, *Radopholus* spp*.* such as *R. similis; Rhadopholus* spp.*; Rhodopholus* spp.*;* Reniform nematodes, *Rotylenchus* spp. such as *R. robustus, R. reniformis; Scutellonema* spp.; Stubby-root nematode, *Trichodorus* spp. such as *T. obtusus, T. primitivus; Paratrichodorus* spp. such as *P. minor;* Stunt nematodes, *Tylenchorhynchus* spp*.* such as *T. claytoni, T. dubius;* Citrus nematodes, *Tylenchulus* spp. such as *T. semipenetrans;* Dagger nematodes, *Xiphinema* spp.*;* and other plant parasitic nematode species;
Insects from the order Isoptera for example *Calotermes flavicollis, Coptotermes* spp. such as *C*. *formosanus, C. gestroi, C. acinaciformis; Cornitermes cumulans, Cryptotermes* spp. such as *C*. *brevis, C. cavifrons; Globitermes sulfureus, Heterotermes* spp. such as *H. aureus, H. longiceps, H. tenuis; Leucotermes flavipes, Odontotermes spp., Incisitermes* spp. such as *I*. *minor, I*. *Snyder, Marginitermes hubbardi, Mastotermes* spp. such as *M. darwiniensis Neocapritermes* spp. such as *N. opacus, N. parvus; Neotermes* spp., *Procornitermes spp., Zootermopsis* spp. *such as Z. angusticollis, Z. nevadensis, Reticulitermes* spp*.* such as *R. hesperus, R. tibialis, R. speratus, R. flavipes, R. grassei, R. lucifugus, R. santonensis, R. virginicus; Termes natalensis,*
Insects from the order Blattaria for example *Blatta* spp. such as *B. orientalis, B. lateralis; Blattella* spp. such as *B. asahinae, B. germanica; Leucophaea maderae, Panchlora nivea, Periplaneta* spp. such as *P. americana, P. australasiae, P. brunnea, P. fuligginosa, P. japonica; Supella longipalpa, Parcoblatta pennsylvanica, Eurycotis floridana, Pycnoscelus surinamensis,*
Insects from the order Siphonoptera for example *Cediopsylla simples, Ceratophyllus* spp., *Ctenocephalides* spp. such as *C. felis, C. canis, Xenopsylla cheopis, Pulex irritans*, *Trichodectes canis, Tunga penetrans,* and *Nosopsyllus fasciatus,*
Insects from the order Thysanura for example *Lepisma saccharina*, *Ctenolepisma urbana,* and *Thermobia domestica,*
Pests from the class Chilopoda for example *Geophilus* spp., Scutigera spp. such as *Scutigera coleoptrata*;
Pests from the class Diplopoda for example *Blaniulus guttulatus, Julus* spp., *Narceus* spp.,
Pests from the class Symphyla for example *Scutigerella immaculata,*
Insects from the order Dermaptera, for example *Forficula auricularia,*
Insects from the order Collembola, for example *Onychiurus* spp., such as *Onychiurus armatus,*
Pests from the order Isopoda for example, *Armadillidium vulgare, Oniscus asellus, Porcellio scaber,*
Insects from the order Phthiraptera, for example *Damalinia* spp*.,* Pediculus spp. such as *Pediculus humanus capitis, Pediculus humanus corporis, Pediculus humanus humanus; Pthirus pubis,* Haematopinus spp. such as *Haematopinus eurysternus, Haematopinus suis;* Linognathus spp. such as *Linognathus vituli; Bovicola bovis, Menopon gallinae, Menacanthus stramineus* and *Solenopotes capillatus, Trichodectes* spp.,
Examples of further pest species which may be controlled by compounds of fomula (I) include: from the Phylum Mollusca, class Bivalvia, for example, *Dreissena* spp.; class Gastropoda, for example, *Arion* spp., *Biomphalaria* spp., *Bulinus* spp., *Deroceras* spp., *Galba spp., Lymnaea* spp., *Oncomelania* spp., *Pomacea canaliclata, Succinea* spp.; from the class of the helminths, for example, *Ancylostoma duodenale, Ancylostoma ceylanicum, Acylostoma braziliensis, Ancylostoma* spp., *Ascaris lubricoides, Ascaris spp., Brugia malayi, Brugia timori, Bunostomum* spp., *Chabertia* spp., *Clonorchis* spp., *Cooperia* spp., *Dicrocoelium* spp., *Dictyocaulus filaria, Diphyllobothrium latum, Dracunculus medinensis, Echinococcus granulosus, Echinococcus multilocularis, Enterobius vermicularis, Faciola* spp., Haemonchus spp. such as *Haemonchus contortus; Heterakis* spp., *Hymenolepis nana, Hyostrongulus* spp., *Loa Loa, Nematodirus* spp., *Oesophagostomum* spp., *Opisthorchis* spp., *Onchocerca volvulus, Ostertagia* spp., *Paragonimus* spp., *Schistosomen* spp., *Strongyloides fuelleborni*, *Strongyloides stercora lis, Stronyloides* spp., *Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.*

The compounds of the invention are suitable for use in treating or protecting animals against infestation or infection by parasites. Therefore, the invention also relates to the use of a compound of the invention for the manufacture of a medicament for the treatment or protection of animals against infestation or infection by parasites. Furthermore, the invention relates to a method of treating or protecting animals against infestation and infection by parasites, which comprises orally, topically or parenterally administering or applying to the animals a parasiticidally effective amount of a compound of the invention.

The present invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound of the invention.

The compounds of the invention are further suitable for use in combating or controlling parasites in and on animals. Furthermore, the invention relates to a method of combating or controlling parasites in and on animals, which comprises contacting the parasites with a parasitically effective amount of a compound of the invention.

The invention also relates to the non-therapeutic use of compounds of the invention for controlling or combating parasites. Moreover, the invention relates to a non-therapeutic method of combating or controlling parasites, which comprises applying to a locus a parasiticidally effective amount of a compound of the invention.

The compounds of the invention can be effective through both contact (via soil, glass, wall, bed net, carpet, blankets or animal parts) and ingestion (e.g. baits). Furthermore, the compounds of the invention can be applied to any and all developmental stages.

The compounds of the invention can be applied as such or in form of compositions comprising the compounds of the invention.

The compounds of the invention can also be applied together with a mixing partner, which acts against pathogenic parasites, e.g. with synthetic coccidiosis compounds, polyether antibiotics such as Amprolium, Robenidin, Toltrazuril, Monensin, Salinomycin, Maduramicin, Lasalocid, Narasin or Semduramicin, or with other mixing partners as defined above, or in form of compositions comprising said mixtures.

The compounds of the invention and compositions comprising them can be applied orally, parenterally or topically, e.g. dermally. The compounds of the invention can be systemically or non-systemically effective.

The application can be carried out prophylactically, therapeutically or non-therapeutically. Furthermore, the application can be carried out preventively to places at which occurrence of the parasites is expected.

As used herein, the term "contacting" includes both direct contact (applying the compounds/compositions directly on the parasite, including the application directly on the animal or excluding the application directly on the animal, e.g. at its locus for the latter) and indirect contact (applying the compounds/compositions to the locus of the parasite). The contact of the parasite through application to its locus is an example of a non-therapeutic use of the compounds of the invention.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include, but are not limited to, lice, biting lice, ticks, nasal bots, keds, biting flies, muscoid flies, flies, myiasitic fly larvae, chiggers, gnats, mosquitoes and fleas.

The compounds of the invention are especially useful for combating parasites of the following orders and species, respectively:
fleas (Siphonaptera), e.g. *Ctenocephalides felis, Ctenocephalides canis, Xenopsylla cheopis, Pulex irritans, Tunga penetrans,* and *Nosopsyllus fasciatus;* cockroaches (Blattaria - Blattodea), e.g. *Blattella germanica, Blattella asahinae, Periplaneta americana, Periplaneta japonica, Periplaneta brunnea, Periplaneta fuligginosa, Periplaneta australasiae,* and *Blatta orientalis;* flies, mosquitoes (Diptera), e.g. *Aedes aegypti, Aedes albopictus, Aedes vexans, Anastrepha ludens, Anopheles maculipennis, Anopheles crucians, Anopheles albimanus, Anopheles gambiae, Anopheles freeborni, Anopheles leucosphyrus, Anopheles minimus, Anopheles quadrimaculatus, Calliphora vicina, Chrysomya bezziana, Chrysomya hominivorax, Chrysomya macellaria, Chrysops discalis, Chrysops silacea, Chrysops atlanticus, Cochliomyia hominivorax, Cordylobia anthropophaga, Culicoides furens, Culex pipiens, Culex nigripalpus, Culex quinquefasciatus, Culex tarsalis, Culiseta inornata, Culiseta melanura, Dermatobia hominis, Fannia canicularis, Gasterophilus intestinalis, Glossina morsitans, Glossina palpalis, Glossina fuscipes, Glossina tachinoides, Haematobia irritans, Haplodiplosis equestris, Hippelates spp., Hypoderma lineata, L eptoconops torrens, Lucilia caprina, Lucilia cuprina, Lucilia sericata, Lycoria pectoralis, Mansonia spp., Musca domestica, Muscina stabulans, Oestrus ovis, Phlebotomus argentipes, Psorophora columbiae, Psorophora discolor, Prosimulium mixtum, Sarcophaga haemorrhoidalis, Sarcophaga sp., Simulium vittatum, Stomoxys calcitrans, Tabanus bovinus, Tabanus atratus, Tabanus lineola,* and *Tabanus similis;* lice (Phthiraptera), e.g. *Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus* and *Solenopotes capillatus;* ticks and parasitic mites (Parasitiformes): ticks (Ixodida), e.g. *Ixodes scapularis, Ixodes holocyclus, Ixodes pacificus, Rhiphicephalus sanguineus, Dermacentor andersoni, Dermacentor variabilis, Amblyomma americanum, Ambryomma maculatum, Ornithodorus hermsi, Ornithodorus turicata* and parasitic mites (Mesostigmata), e.g. *Ornithonyssus bacoti* and *Dermanyssus gallinae*; Actinedida (Prostigmata) und Acaridida (Astigmata), e.g. *Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp.,Knemidocoptes spp., Cytodites spp.,* and *Laminosioptes spp;* Bugs (Heteropterida): *Cimexlectularius, Cimexhemipterus, Reduvius senilis, Triatoma spp., Rhodnius ssp., Panstrongylus ssp.,* and *Arilus critatus;* Anoplurida, e.g. *Haematopinus spp., Linognathus spp., Pediculus spp., Phtirus spp.,* and *Solenopotes spp.;* Mallophagida (suborders Arnblycerina and Ischnocerina), e.g. *Trimenopon spp., Menopon spp., Trinoton spp., Bovicola spp., Werneckiella spp., Lepikentron spp., Trichodectes spp.,* and *Felicola spp.;* Roundworms Nematoda: Wipeworms and Trichinosis (Trichosyringida), e.g. Trichinellidae *(Trichinella spp.), (*Trichuridae*) Trichuris spp., Capillaria spp.;* Rhabditida, e.g. *Rhabditis spp., Strongyloides spp., Helicephalobus spp.;* Strongylida, e.g. *Strongylus spp., Ancylostoma spp., Necatoramericanus, Bunostomum spp. (Hookworm), Trichostrongylus spp., Haemonchus contortus, Ostertagia spp., Cooperia spp., Nematodirus spp., Dictyocaulus spp., Cyathostoma spp., Oesophagostomum spp., Stephanurus dentatus, Ollulanus spp., Chabertia spp., Stephanurus dentatus, Syngamus trachea, Ancylostoma spp., Uncinaria spp., Globocephalus spp., Necatorspp., Metastrongylus spp., Muellerius capillaris, Protostrongylus spp., Angiostrongylus spp., Parelaphostrongylus spp., Aleurostrongylus abstrusus,* and *Dioctophyma renale;* Intestinal roundworms (Ascaridida), e.g. *Ascaris lumbricoides, Ascaris suum, Ascaridia galli, Parascaris equorum, Enterobius vermicularis (Threadworm), Toxocara canis, Toxascaris leonine, Skrjabinema spp.,* and *Oxyuris equi;* Camallanida, e.g. *Dracunculus medinensis* (guinea worm); Spirurida, e.g. *Thelazia spp., Wuchereria spp., Brugia spp., Onchocerca spp., Dirofilari spp.a, Dipetalonema spp., Setaria spp., Elaeophora spp., Spirocerca lupi,* and *Habronema spp.;* Thorny headed worms (Acanthocephala), e.g. *Acanthocephalus spp., Macracanthorhynchus hirudinaceus* and *Oncicola spp.;* Planarians (Plathelminthes): Flukes (Trematoda), e.g. *Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp.,* and *Nanocyetes spp*.; Cercomeromorpha, in particular Cestoda (Tapeworms), e.g. *Diphyllobothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp.,* and *Hymenolepis spp..*

As used herein, the term "animal" includes warm-blooded animals (including humans) and fish. Preferred are mammals, such as cattle, sheep, swine, camels, deer, horses, pigs, poultry, rabbits, goats, dogs and cats, water buffalo, donkeys, fallow deer and reindeer, and also in fur-bearing animals such as mink, chinchilla and raccoon, birds such as hens, geese, turkeys and ducks and fish such as fresh- and salt-water fish such as trout, carp and eels. Particularly preferred are domestic animals, such as dogs or cats.

In general, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions such as desired parasiticidal effect and duration, target species, mode of application, and the like.

Generally, it is favorable to apply the compounds of the invention in total amounts of 0.5 mg/kg to 100 mg/kg per day, preferably 1 mg/kg to 50 mg/kg per day.

For oral administration to warm-blooded animals, the formula I compounds may be formulated as animal feeds, animal feed premixes, animal feed concentrates, pills, solutions, pastes, suspensions, drenches, gels, tablets, boluses and capsules. In addition, the formula I compounds may be administered to the animals in their drinking water. For oral administration, the dosage form chosen should provide the animal with 0.01 mg/kg to 100 mg/kg of animal body weight per day of the formula I compound, preferably with 0.5 mg/kg to 100 mg/kg of animal body weight per day.

Alternatively, the formula I compounds may be administered to animals parenterally, for example, by intraruminal, intramuscular, intravenous or subcutaneous injection. The formula I compounds may be dispersed or dissolved in a physiologically acceptable carrier for subcutaneous injection. Alternatively, the formula I compounds may be formulated into an implant for subcutaneous administration. In addition the formula I compound may be transdermally administered to animals. For parenteral administration, the dosage form chosen should provide the animal with 0.01 mg/kg to 100 mg/kg of animal body weight per day of the formula I compound.

The formula I compounds may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the formula I compound. In addition, the formula I compounds may be formulated as ear tags for animals, particularly quadrupeds such as cattle and sheep.

Suitable preparations are:
- Solutions such as oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations such as powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further auxiliaries such as acids, bases, buffer salts, preservatives, and solubilizers. Suitable auxiliaries for injection solutions are known in the art. The solutions are filtered and filled sterile.

Oral solutions are administered directly. Concentrates are administered orally after prior dilution to the use concentration. Oral solutions and concentrates are prepared according to the state of the art and as described above for injection solutions, sterile procedures not being necessary. Solutions for use on the skin are trickled on, spread on, rubbed in, sprinkled on or sprayed on. Solutions for use on the skin are prepared according to the state of the art and according to what is described above for injection solutions, sterile procedures not being necessary.

Gels are applied to or spread on the skin or introduced into body cavities. Gels are prepared by treating solutions which have been prepared as described in the case of the injection solutions with sufficient thickener that a clear material having an ointment-like consistency results. Suitable thickeners are known in the art.

Pour-on formulations are poured or sprayed onto limited areas of the skin, the active compound penetrating the skin and acting systemically. Pour-on formulations are prepared by dissolving, suspending or emulsifying the active compound in suitable skin-compatible solvents or solvent mixtures. If appropriate, other auxiliaries such as colorants, bioabsorption-promoting substances, antioxidants, light stabilizers, adhesives are added. Suitable such auxiliaries are known in the art.

Emulsions can be administered orally, dermally or as injections. Emulsions are either of the water-in-oil type or of the oil-in-water type. They are prepared by dissolving the active compound either in the hydrophobic or in the hydrophilic phase and homogenizing this with the solvent of the other phase with the aid of suitable emulsifiers and, if appropriate, other auxiliaries such as colorants, absorption-promoting substances, preservatives, antioxidants, light stabilizers, viscosity-enhancing substances. Suitable hydrophobic phases (oils), suitable hydrophilic phases, suitable emulsifiers, and suitable further auxiliaries for emulsions are known in the art.

Suspensions can be administered orally or topically/dermally. They are prepared by suspending the active compound in a suspending agent, if appropriate with addition of other auxiliaries such as wetting agents, colorants, bioabsorption-promoting substances, preservatives, antioxidants, light stabilizers. Suitable suspending agents, and suitable other auxiliaries for suspensions including wetting agents are known in the art.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form. Suitable auxiliaries for this purpose are known in the art.

The compositions which can be used in the invention can comprise generally from about 0.001 to 95% of the compound of the invention.

Ready-to-use preparations contain the compounds acting against parasites, preferably ectoparasites, in concentrations of 10 ppm to 80 per cent by weight, preferably from 0.1 to 65 per cent by weight, more preferably from 1 to 50 per cent by weight, most preferably from 5 to 40 per cent by weight.

Preparations which are diluted before use contain the compounds acting against ectoparasites in concentrations of 0.5 to 90 per cent by weight, preferably of 1 to 50 per cent by weight.

Furthermore, the preparations comprise the compounds of formula I against endoparasites in concentrations of 10 ppm to 2 per cent by weight, preferably of 0.05 to 0.9 per cent by weight, very particularly preferably of 0.005 to 0.25 per cent by weight.

Topical application may be conducted with compound-containing shaped articles such as collars, medallions, ear tags, bands for fixing at body parts, and adhesive strips and foils.

Generally it is favorable to apply solid formulations which release compounds of the invention in total amounts of 10 mg/kg to 300 mg/kg, preferably 20 mg/kg to 200 mg/kg, most preferably 25 mg/kg to 160 mg/kg body weight of the treated animal in the course of three weeks.

### Examples

The present invention is now illustrated in further details by the following example, without imposing any limitation thereto.

With appropriate modification of the starting materials or intermediates thereof, the procedures as described in the examples below can used to obtain further compounds of formula I.

¹H-NMR: The signals are characterized by chemical shift (ppm, δ [delta]) vs. tetramethylsilane, respectively CDCl₃ for ¹³C-NMR, by their multiplicity and by their integral (relative number of hydrogen atoms given). The following abbreviations are used to characterize the multiplicity of the signals: m = multiplet, q = quartet, t = triplet, d = doublet and s = singlet.

Abbreviations used are: d for day(s), h for hour(s), min for minute(s), RT/room temperature for 20-25°C, Rt for retention time; DCM for dichloromethane, Mel for methyl iodide, TEA for triethylamine, DMF for dimethylformamide, THF for tetrahydrofuran, m-CPBA for meta-chloroperbenzoic acid.

### Synthesis of ethyl 2-(3-(ethylsulfonyl)pyridin-2-yl)-1-methyl-5-(3,3,3-trifluoropropyl)-1H-imidazole-4-carboxylate (6):

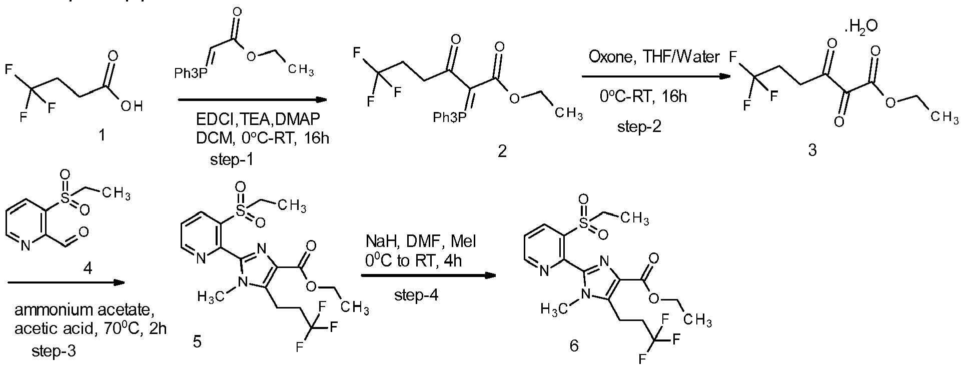

### Synthesis of ethyl 6,6,6-trifluoro-3-oxo-2-(triphenyl-I5-phosphaneylidene)hexanoate (2):

A solution of 4,4,4-trifluorobutanoic acid (20 g) in dichloromethane (500 mL) was cooled to 0 °C and treated with EDC•HCl (29.7 g), TEA (39.0 mL) and DMAP (10 mg). The reaction mixture was stirred at 0 °C for 15 min and ethyl 2-(triphenyl-l5-phosphaneylidene)acetate (49.0 g) was added in portions. The reaction was then stirred at room temperature for 16 h. The mixture was diluted with water (300 mL) and extracted with dichloromethane (3 × 200 mL). The organic layer was washed with NaHCO3 solution and dried over anhydrous Na₂SO₄. The solvent was removed under reduced pressure to obtain a residue which was purified by silica gel (100-200 mesh) column chromatography using 15-20% ethyl acetate in hexanes to afford ethyl 6,6,6-trifluoro-3-oxo-2-(triphenyl-I5-phosphaneylidene)hexanoate (2) as yellow solid (23.6 g, 35%). ¹H NMR (400 MHz, DMSO-d6) δ ppm 7.58-7.66 (m, 10H), 7.51-7.57 (m, 5H), 3.55 (q, *J* = 7.2 Hz, 2H), 3.05 (t, *J* = 7.6 Hz, 2H), 2.30-2.40 (m, 2H), 0.49 (t, *J* = 7.2 Hz, 3H).

### Synthesis of ethyl 2-(3-(ethylsulfonyl)pyridin-2-yl)-5-(3,3,3-trifluoropropyl)-1H-imidazole-4-carboxylate (5):

To a solution of ethyl 6,6,6-trifluoro-3-oxo-2-(triphenyl-l5-phosphaneylidene)hexanoate (21.0 g) in THF (230 mL) and water (120 mL) was cooled to 0 °C and treated with Oxone (41.0 g). The reaction mixture was then stirred at room temperature for 16 h. The mixture was diluted with water (200 mL) and extracted with ethyl acetate (3 × 200 mL). The organic layer was dried over anhydrous sodium sulphate and the solvent was removed under reduced pressure to obtain a crude residue. The residue was purified by silica gel (100-200 mesh) column chromatography using 30% ethyl acetate in hexanes to afford ethyl 6,6,6-trifluoro-2,3-dioxohexanoate hydrate (3)as brown liquid (4.68 g, 43%) which was carried on without further characterization.

A solution of 6,6,6-trifluoro-2,3-dioxohexanoate hydrate (5.9 g) and 3-(ethylsulfonyl)picolinaldehyde (9.6 g)in acetic acid (40 mL) was treated with ammonium acetate (18.6 g) and heated the mixture at 70 °C for 2 h and monitored by TLC which showed completion. The mixture was cooled and basified with NaHCO₃ and extracted with ethyl acetate (3 × 200 mL). The organic layer was dried over anhydrous sodium sulphate and the solvent was removed under reduced pressure to obtain a residue. The residue was purified by silica gel (100-200 mesh) column chromatography using 50% ethyl acetate in hexanes to afford ethyl 2-(3-(ethylsulfonyl)pyridin-2-yl)-5-(3,3,3-trifluoropropyl)-1H-imidazole-4-carboxylate (5) as yellow solid (6.0 g, 61%).¹H NMR (400 MHz, DMSO-d6) δ ppm 13.48 (s, 1H), 8.94(d, *J* = 4.8 Hz, 1H), 8.48 (d, *J* = 8.2 Hz, 1H), 7.74 (dd, *J* = 8.2, 4.8 Hz, 1H), 4.33 (q, *J* = 7.6 Hz, 2H), 4.26 (q, *J* = 7.2 Hz, 2H), 3.20-3.26 (m, 2H), 2.60-2.72 (m, 2H), 1.20-1.36 (m, 6H).

### Synthesis of ethyl 2-(3-(ethylsulfonyl)pyridin-2-yl)-1-methyl-5-(3,3,3-trifluoropropyl)-1H-imidazole-4-carboxylate (6):

A solution of ethyl 2-(3-(ethylsulfonyl)pyridin-2-yl)-5-(3,3,3-trifluoropropyl)-1H-imidazole-4-carboxylate (6.0 g) in dimethyl formamide (30 mL) was cooled to 0 °C and treated with caesium carbonate (12.0 g) and dimethyl sulphate (3.7 g). The reaction mixture was then stirred at room temperature for 16 h and monitored by TLC which showed completion. The mixture was diluted with ice cold water (100 mL) and extracted with ethyl acetate (2 × 200 mL). The organic layer was washed with cold water and brine and the organic layer was dried over anhydrous sodium sulphate and the solvent was removed under reduced pressure to obtain a residue. The residue was purified by silica gel (100-200 mesh) column chromatography using 85% ethyl acetate in hexanes to afford ethyl 2-(3-(ethylsulfonyl)pyridin-2-yl)-1-methyl-5-(3,3,3-trifluoropropyl)-1H-imidazole-4-carboxylate as yellow solid (0.78 g, 12%).¹H NMR (400 MHz, DMSO-d6) δ ppm 9.00(d, *J* = 3.6 Hz, 1H), 8.46(d, *J* = 7.2 Hz, 1H), 7.84 (dd, *J* = 8.0, 4.8 Hz, 1H), 4.24 (q, *J* = 7.6 Hz, 2H), 3.90 (q, *J* = 7.2 Hz, 2H), 3.58 (s, 3H), 3.22-3.32 (m, 2H), 2.56-2.70 (m, 2H), 1.28 (t, *J* = 7.2 Hz, 3H), 1.18 (t, *J* = 7.6 Hz, 2H).

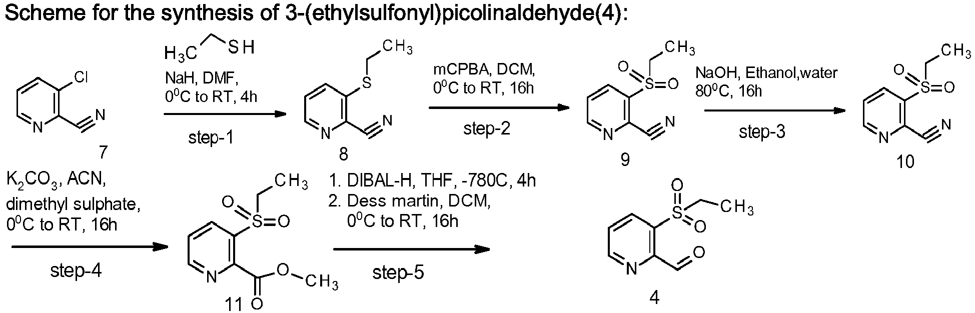

### Synthesis of 3-(ethylthio)picolinonitrile (8):

A solution of 3-chloropicolinonitrile(24.0 g) and ethanethiol (12.9 g) in dimethyl formamide (150 mL) was cooled to 0 °C and treated with sodium hydride (60% in oil dispersion) (9.0 g, 0.22 mol). The reaction mixture was then stirred at room temperature for 4 h and monitored by TLC which showed completion. The mixture was diluted with ice cold water (150 mL) and extracted with ethyl acetate (2 × 200 mL).The organic layer was washed with cold water and brine solution then with NaHCO₃. The organic layer was dried over anhydrous sodium sulphate and the solvent was removed under reduced pressure to afford 3-(ethylthio)picolinonitrile (8) as sticky liquid (38 g). ¹H NMR (400 MHz, DMSO-d6) δ ppm 8.52 (d, *J* = 3.6 Hz, 1H), 8.07(d, *J* = 8.4 Hz, 1H), 7.68(dd, *J* = 8.4, 4.8 Hz, 1H), 3.17 (q, *J* = 7.2 Hz, 2H), 1.27 (t, *J* = 7.2 Hz, 3H).

### Synthesis of 3-(ethylsulfonyl)picolinonitrile (9):

A solution of 3-(ethylthio)picolinonitrile (38.0 g, 0.23 mol) in dichloromethane (600 mL) was cooled to 0 °C and treated with m-CPBA (99.6 g, 0.57 mol). The reaction mixture was then stirred at room temperature for 16 h. The mixture was then diluted with water (200 mL) and extracted with dichloromethane (3 × 200 mL). The organic layer was washed with 1N NaOH solution and layers were separated. The organic layer was dried over anhydrous sodium sulphate and the solvent was removed under reduced pressure to afford 3-(ethylsulfonyl)picolinonitrile (9) as white solid (25.0 g, 55%). ¹H NMR (400 MHz, DMSO-d6) δ ppm 9.07 (d, *J* = 4.8 Hz, 1H), 8.50 (d, *J* = 8.4 Hz, 1H), 8.03 (dd, *J* = 8.4, 4.8 Hz, 1H), 3.57 (q, *J* = 7.2 Hz, 2H), 1.29 (t, *J* = 7.2 Hz, 3H).

### Synthesis of 3-(ethylsulfonyl)picolinic acid (10):

A solution of 3-(ethylsulfonyl)picolinonitrile(25.0 g) in ethanol (150 mL) and water (150 mL) was treated with NaOH (25.5 g) and the reaction mixture was then heated at 80 °C for 16 h. The ethanol was removed under reduced pressure and residue was diluted with water (100 mL) and extracted with ethyl acetate (2 × 100 mL).The organic layer was discarded and aqueous layer was acidified with 1N HCl and then extracted with 10% methanol in dichloromethane. The organic layer was dried over anhydrous sodium sulphate and the solvent was removed under reduced pressure to afford 3-(ethylsulfonyl)picolinic acid (10) as brown sticky liquid (22.0 g, 80%). ¹H NMR (400 MHz, DMSO-d6) δ ppm 14.09 (brs, 1H), 8.89 (d, *J* = 4.8 Hz, 1H), 8.34 (d, *J* = 8.4 Hz, 1H), 7.78 (dd, *J* = 8.4, 4.8 Hz, 1H), 3.52 (q, *J* = 7.2 Hz, 2H), 1.16 (t, *J* = 7.2 Hz, 3H).

### Synthesis of methyl 3-(ethylsulfonyl)picolinate(11):

To a solution of 3-(ethylsulfonyl)picolinic acid (22.0 g) in acetonitrile(200 mL), K₂CO₃ (42.3 g) was added at room temperature. The reaction mixture was cooled to 0 °C and treated with dimethyl sulphate (19.3 g). The reaction mixture was then stirred at room temperature for 16 h. The mixture was diluted with water (200 mL) and extracted with ethyl acetate (2 × 200 mL). The organic layer was dried over anhydrous sodium sulphate and the solvent was removed under reduced pressure to obtain a residue. The residue was purified by silica gel (100-200 mesh) column chromatography using 40% ethyl acetate in hexanes to afford methyl 3-(ethylsulfonyl)picolinate (11) as brown liquid (13.0 g, 55%). ¹H NMR (400 MHz, DMSO-d6) δ ppm 8.92 (d, *J* = 4.8 Hz, 1H), 8.40 (d, *J* = 8.4 Hz, 1H), 7.86 (dd, *J* = 8.4, 4.8 Hz, 1H), 3.90 (s, 3H), 3.49 (q, *J* = 7.2 Hz, 2H), 1.19 (t, *J* = 7.2 Hz, 3H).

### Synthesis of 3-(ethylsulfonyl)picolinaldehyde(4):

A solution of methyl 3-(ethylsulfonyl)picolinate(13.0 g) in tetrahydrofuran (300 mL) was cooled to - 78 °C and treated with DIBAL-H (85.1 mL). The reaction mixture was then stirred at -78 °C for 4 h. The mixture was quenched with ammonium chloride solution (100 mL) and extracted with ethyl acetate (2 × 200 mL). The organic layer was dried over anhydrous sodium sulphate and the solvent was removed under reduced pressure to obtain a residue. The residue was dissolved in dichloromethane (300 mL), cooled to 0 °C and treated with the Dess-Martin reagent (14.0 g). The reaction mixture was then stirred at room temperature for 16 h. The mixture was filtered and filtrate was washed with water (3 × 100 mL).The organic layer was dried over anhydrous sodium sulphate and the solvent was removed under reduced pressure to afford 3-(ethylsulfonyl)picolinaldehyde (4) as brown sticky liquid (10.5 g, 93%). ¹H NMR (400 MHz, DMSO-d6) δ ppm 10.31 (s, 1H), 9.08 (d, *J* = 4.8 Hz, 1H), 8.88 (d, *J* = 8.4 Hz, 1H), 8.31 (dd, *J* = 8.4, 4.8 Hz, 1H), 3.67 (q, *J* = 7.2 Hz, 2H), 1.21 (t, *J* = 7.2 Hz, 3H).

## Claims

1. A compound of formula I 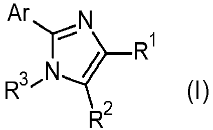 wherein
R¹ is CN, NO₂, C(O)-OR^{b}, C(O)NR^{b}R^{c}, C₁-C₆-alkyl, C₂-C₆-alkenyl-C₁-C₆-alkyl, C₂-C₆-alkenyl-NR^{b}R^{c}, C₂-C₆-alkenyl-CN, C₂-C₆-alkenyl-C(O)-OR^{b}, C₂-C₆-alkenyl-C(O)-R^{b}, C₂-C₆-alkenyl-S(=O)ₘR^{e}, C(O)-R^{d}, SO₂NR^{b}R^{c}, or S(=O)ₘR^{e}, which are unsubstituted or substituted with R^{a};
R² H, halogen, N₃, CN, NO₂, -SCN, -SF₅, C₁-C₆-alkyl, C₁-C₆-alkoxy, C₂-C₆-alkenyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b}, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxyx-C₁-C₄-alkyl, C(O)-OR^{b}, NR^{b}R^{c}, C(O)-NR^{b}R^{c}, C(O)-R^{d}, SO₂NR^{b}R^{c}, and S(=O)ₘR^{e}, which are unsubstituted or substituted with R^{a};
R³ H, C₁-C₆-alkyl, C₁-C₆-alkoxy, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, which are unsubstituted or substituted with R^{a}, C(O)-OR^{a}, NR^{b}R^{c}, C(O)-NR^{b}R^{c}, C(O)-R^{d}, SO₂NR^{b}R^{c}, and S(=O)ₘR^{e};
Ar is phenyl or 5- or 6-membered heteroaryl, which is unsubstituted or substituted with R^{Ar};
R^{Ar} independently of each other, are selected from the group consisting of halogen, N₃, OH, CN, NO₂, -SCN, -SF₅, C₁-C₆-alkyl, C₁-C₆-alkoxy, C₂-C₆-alkenyl, tri-C₁-C₆-alkylsilyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, C₃-C₆-cycloalkoxy-C₁-C₄-alkyl, which are unsubstituted or substituted with halogen,
C(O)-OR^{a}, NR^{b}R^{c}, C₂-C₆-alkenyl-NR^{b}R^{c}, O-C₂-C₆-alkenyl-NR^{b}R^{c}, C₂-C₆-alkenyl-CN, NH-C₂-C₆-alkenyl-NR^{b}R^{c}, C(O)-NR^{b}R^{c}, C(O)-R^{d}, SO₂NR^{b}R^{c}, and S(=O)ₘR^{e}, one radical may also be phenyl, phenoxy, phenylcarbonyl, phenylthiol, and benzyl, wherein the phenyl ring is unsubstituted or substituted with radicals R^{f};
each Rₐ is selected from halogen, OH, CN, NO₂, NR^{b}R^{c}, C₁-C₆-alkyl, C₁-C₆-alkoxy, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, C₃-C₆-cycloalkoxy-C₁-C₄-alkyl, C(O)-OR^{b}, S(=O)ₘR^{e}, which are unsubstituted or substituted with halogen, phenyl, and benzyl, wherein the phenyl ring is unsubstituted or substituted with radicals R^{f};
each R^{b} is selected from H, C₁-C₆-alkyl, C₂-C₆-alkenyl, C₂-C₆-alkynyl, C₁-C₆-alkoxy-C₁-C₄-alkyl, C₁-C₆-alkylthio-C₁-C₄-alkyl, C₃-C₆-cycloalkyl, C₃-C₆-cycloalkyl-C₁-C₄-alkyl, and C₃-C₆-cycloalkoxy-C₁-C₄-alkyl, which are unsubstituted or substituted with halogen,-(C=O)R^{d}, -C(=O)OR^{d}, -C(=O)NR^{d}, phenyl, benzyl or 5- to 6-membered heterocycle wherein the cyclic groups are unsubstituted or substituted with radicals R^{f};
each R^{c} is selected from H, C₁-C₆-alkyl, C₂-C₆-alkenyl, C₂-C₆-alkynyl, C₁-C₆-alkoxy-C₁-C₄-alkyl, C₃-C₆-cycloalkyl, C₃-C₆-cycloalkyl-C₁-C₄-alkyl, and C₃-C₆-cycloalkoxy-C₁-C₄-alkyl, which are unsubstituted or substituted with halogen;
each moiety NR^{b}R^{c} may also form an N-bound, saturated 3- to 8-membered heterocycle, which in addition to the nitrogen atom may have 1 or 2 further heteroatoms or heteroatom moieties selected from O, S(=O)ₘ and N-R', wherein R' is H, -C(=O)OR, or C₁-C₆-alkyl and wherein the N-bound heterocycle is unsubstituted or substituted with radicals selected from halogen, C₁-C₄-alkyl, C₁-C₄-haloalkyl, C₁-C₄-alkoxy, and C₁-C₄-haloalkoxy;
each R^{d} is selected from H, C₁-C₆-alkyl, C₂-C₆-alkenyl, C₂-C₆-alkynyl, C₁-C₆-alkoxy-C₁-C₄-al-kyl, C₃-C₆-cycloalkyl, C₃-C₆-cycloalkyl-C₁-C₄-alkyl, C₃-C₆-cycloalkoxy-C₁-C₄-alkyl, which are unsubstituted or substituted with halogen, phenyl, and benzyl, wherein the phenyl ring is unsubstituted or substituted with radicals R^{f};
each R^{e} is selected from C₁-C₆-alkyl, C₃-C₆-cycloalkyl, C₃-C₆-cycloalkyl-C₁-C₄-alkyl, which are unsubstituted or substituted with halogen, phenyl, and benzyl, wherein the phenyl ring is unsubstituted or substituted with R^{f};
each R^{f} is selected from halogen, N₃, OH, CN, NO₂, SCN, SF₅, C₁-C₆-alkyl, C₁-C₆-alkoxy, C₂-C₆-alkenyl, C₂-C₆-alkynyl, C₁-C₆-alkoxy-C₁-C₄-alkyl, C₁-C₆-alkoxy-C₁-C₄-alkoxy, C₃-C₆-cycloalkyl, C₃-C₆-cycloalkoxy, C₃-C₆-cycloalkyl-C₁-C₄-alkyl, and C₃-C₆-cycloalkoxy-C₁-C₄-alkyl, which are unsubstituted or substituted with halogen;
m is 0, 1 or 2;
including the N-oxides, stereoisomers, tautomers and agriculturally or veterinarily acceptable salts thereof.

2. The compound according to claim 1, wherein R¹ and R² independently of each other are selected from CN, C(O)-OR^{b}, C(O)NR^{b}R^{c}, C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, C₂-C₆-alkenyl-C(O)-R^{b}, which are unsubstituted or substituted with R^{a},
R^{a} is selected from halogen, OH, CN, NO₂, NR^{b}R^{c}, C(O)-OR^{b}, and S(=O)ₘR^{e};
R^{b} is selected from H, C₁-C₆-alkyl, C₁-C₆-haloalkyl, C₃-C₆-cycloalkyl, C₂-C₆-alkenyl, and C₂-C₆-alkynyl;
R^{c} is selected from H, C₁-C₆-alkyl, C₁-C₆-haloalkyl, and C₃-C₆-cycloalkyl.

3. The compound according to claim 1 or 2, wherein
Ar is a phenyl or 5- or 6-membered heteroaryl which is substituted with R^{Ar};
R^{Ar} is selected from halogen, C₁-C₆-alkyl, C₁-C₆-haloalkyl, C₁-C₆-alkoxy, C₁-C₆-haloalkoxy, S(=O)ₘRₑ, phenyl, phenoxy, phenylcarbonyl, phenylthio and benzyl, wherein the phenyl ring is unsubstituted or substituted with radicals R^{f};
Re is selected from C₁-C₆-alkyl, C₃-C₆- cycloalkyl, C₁-C₆-haloalkyl, and C₃-C₆- halocycloalkyl;
R^{f} is selected from halogen, C₁-C₆-alkyl, C₁-C₆-alkoxy, C₃-C₆-cycloalkyl, C₂-C₆-alkenyl, and C₂-C₆-alkynyl, which are unsubstituted or substituted with halogen.

4. The compound according to claim 3, wherein Ar is selected from formula Ar-1 to Ar-9, 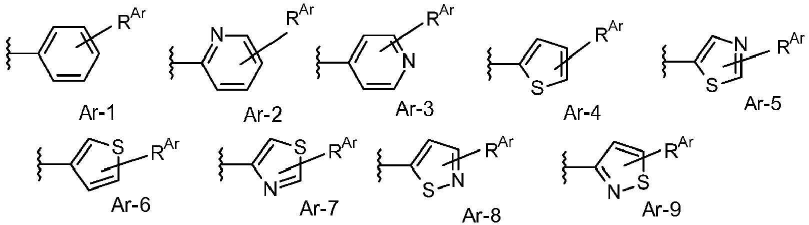
wherein one R^{Ar} substituent which is S(=O)ₘR^{e} is bonded at the ortho position to 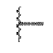 bond; and optionally further substituted with 1 R^{Ar} selected from halogen, C₁-C₆-alkyl, C₁-C₆-haloalkyl, C₁-C₆-alkoxy, C₁-C₆-haloalkoxy, phenyl, and benzyl, wherein the phenyl ring of R^{Ar} is unsubstituted or substituted with radicals R^{f}
Re is selected from C₁-C₆-alkyl, C₃-C₆- cycloalkyl, C₁-C₆-haloalkyl, and C₃-C₆- halocycloalkyl;
R^{f} is selected from halogen, C₁-C₆-alkyl, C₁-C₆-alkoxy, C₃-C₆-cycloalkyl, C₂-C₆-alkenyl, and C₂-C₆-alkynyl, which are unsubstituted or substituted with halogen.

5. The compound according to any of preceding claims, wherein
R³ is C₁-C₆-alkyl, C₁-C₆-alkoxy-C₁-C₄-alkyl, C₃-C₆-cycloalkyl, or C₃-C₆-cycloalkyl-C₁-C₄-alkyl, which are unsubstituted or substituted with R^{a}.

6. The compound according to any of preceding claims, wherein
R¹ and R² independently of each other are selected from CN, C(O)-OR^{b}, C(O)NR^{b}R^{c}, C₁-C₆-alkyl, C₂-C₆-alkenyl-C₁-C₆-alkyl, C₂-C₆-alkenyl-NR^{b}R^{c}, C₂-C₆-alkenyl-C(O)-OR^{b}, and C₂-C₆-alkenyl-C(O)-R^{b}, which are unsubstituted or substituted with R^{a};
R³ is C₁-C₆-alkyl;
Ar is pyridinyl substituted with R^{Ar} as S(=O)ₘRₑ at the ortho position to bond connecting to imidazolyl ring of compound of formula I, and optionally further substituted with 1 R^{Ar} selected from halogen, C₁-C₆-alkyl, C₁-C₆-haloalkyl, C₁-C₆-alkoxy, C₁-C₆-haloalkoxy, phenyl, and benzyl, wherein the phenyl ring of R^{Ar} is unsubstituted or substituted with radicals R^{f};
R^{a} is selected from halogen, OH, CN, NO₂, NR^{b}R^{c}, C(O)-OR^{b}, and S(=O)ₘRₑ;
R^{b} is selected from H, C₁-C₆-alkyl, C₁-C₆-haloalkyl, C₃-C₆-cycloalkyl, C₂-C₆-alkenyl, and C₂-C₆-alkynyl;
R^{c} is selected from H, C₁-C₆-alkyl, and C₃-C₆-cycloalkyl;
R^{e} is selected from C₁-C₆-alkyl, C₃-C₆-cycloalkyl, C₁-C₆-haloalkyl, and C₃-C₆halocycloalkyl;
R^{f} is selected from halogen, C₁-C₆-alkyl, C₁-C₆-alkoxy, C₃-C₆-cycloalkyl, C₂-C₆-alkenyl, and C₂-C₆-alkynyl, which are unsubstituted or substituted with halogen;
m is 0, 1, or 2.

7. A composition comprising the compound of formula I, as defined in any of claims 1 to 6, and solid or liquid carrier.

8. The composition according to claim 7, comprising additionally a further active substance.

9. A method for combating or controlling invertebrate pests, which method comprises contacting said pest or its food supply, habitat or breeding grounds with a pesticidally effective amount of at least one compound of the formula I according to any of claims 1 to 6 or the composition according to claim 7 or 8.

10. A method for protecting growing plants from attack or infestation by invertebrate pests, which method comprises contacting a plant, or soil or water in which the plant is growing, with a pesticidally effective amount of at least one compound of the formula I, according to any of the claims 1 to 6 or the composition according to claim 7 or 8.

11. A seed comprising a compound of the formula I, as defined in any of claims 1 to 6, or the enantiomers, diastereomers or salts thereof, or the composition, as defined in any of the claims 7 or 8, in an amount of from 0.1 g to 10 kg per 100 kg of seed.

12. A use of a compound of the formula I, as defined in any of the claims 1 to 6, and of an agriculturally acceptable salt thereof, or of the compositions as defined in any of the claims 7 or 8, for protecting growing plants from attack or infestation by invertebrate pests.

13. A method for treating or protecting an animal from infestation or infection by invertebrate pests which comprises bringing the animal in contact with a pesticidally effective amount of at least one compound of the formula I as defined in any of claims 1 to 6, a stereoisomer thereof and/or at least one veterinarily acceptable salt thereof.
